(19) Europäisches Patentamt
European Patent Office
Office européen des brevets

(11) EP 4 428 124 A1

(12) EUROPEAN PATENT APPLICATION

(43) Date of publication:
**11.09.2024 Bulletin 2024/37**

(21) Application number: **23160616.1**

(22) Date of filing: **07.03.2023**

(51) International Patent Classification (IPC):
***C07D 239/26*** (2006.01) ***C07C 15/04*** (2006.01)
***A61P 25/18*** (2006.01) ***A61P 25/28*** (2006.01)
***A61P 35/00*** (2006.01)

(52) Cooperative Patent Classification (CPC):
**C07D 239/26; A61P 25/18; A61P 25/28; A61P 35/00; C07C 229/36; C07C 309/04;**
C07B 2200/07

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Vesper Bio Aps**
**1610 København V (DK)**

(72) Inventors:
- **Cases-Thomas, Manuel Javier**
  **2100 Copenhagen (DK)**
- **Kjølby, Mads Fuglsang**
  **2100 Copenhagen (DK)**
- **Nykjær, Anders**
  **2100 Copenhagen (DK)**
- **Little, Brian Paul**
  **2100 Copenhagen (DK)**

(74) Representative: **Gill Jennings & Every LLP**
**The Broadgate Tower**
**20 Primrose Street**
**London EC2A 2ES (GB)**

Remarks:
The complete document including Reference Table(s) and the Sequence Listing(s) can be downloaded from the EPO website

# (54) MODULATORS OF SORTILIN ACTIVITY

(57) The present invention relates to compounds of Formula (I) or a pharmaceutically acceptable salt, solvate, hydrate, tautomer, optical isomer, N oxide, and/or prodrug thereof. The present invention also relates to pharmaceutical compositions comprising the compounds of the invention, and to their use in the treatment or prevention of medical conditions in which modulation of sortilin is beneficial.

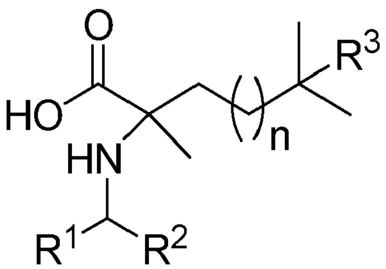

(I)

EP 4 428 124 A1

## Description

### FIELD OF THE INVENTION

The invention relates to compounds of Formula (I), which have surprisingly been found to modulate the activity of sortilin. The invention also relates to pharmaceutical compositions comprising these compounds, and to their use in the treatment or prevention of medical conditions in which modulation of sortilin activity is beneficial. In particular, the compounds of the invention may cross the blood brain barrier and may therefore be particularly useful in the treatment of diseases of the central nervous system. The compounds of the invention may also have an improved half-life in vivo.

### BACKGROUND

Sortilin is a Type I transmembrane protein that acts as a receptor of several ligands (Petersen et al., 1997). Sortilin is abundantly expressed in neurons and microglia of the nervous system, the inner ear, and in some peripheral tissues involved in metabolic control (Tauris et al., 2020; Goettsch et al., 2017; Willnow et al., 2011; Kjolby et al., 2010). Besides acting as a receptor involved in signaling, sortilin mediates sorting of select cargo between the cell surface trans-Golgi network, and endosomal pathway (Nykjaer & Willnow, 2012; Willnow, Petersen, & Nykjaer, 2008). Sortilin harbors a large extracellular domain denoted VPS10 that defines the family of receptors named sortilins or VS10p domain receptors. The VPS10P domain in sortilin is homologous to yeast VPS10P and is made up by a 10-bladed beta-propeller structure and a cysteine-rich 10CC module (Nykjaer & Willnow, 2012; Zheng, Brady, Meng, Mao, & Hu, 2011).

Sortilin binds multiple ligands, including pro-nerve growth factor (pro-NGF), pro-BDNF, pro-neurotrophin-3, neurotensin, and ApoB (Chen et al., 2005; Kjolby et al., 2010; Mazella et al., 1998; Nykjaer et al., 2004; Quistgaard et al., 2009; Yano, Torkin, Martin, Chao, & Teng, 2009). Furthermore, Sortilin binds progranulin (PGRN), a secreted protein involved in many cellular functions including securing lysosomal processes, anti-inflammatory responses, and neurotrophic stimulation (Galimberti, Fenoglio, & Scarpini, 2018). Sortilin targets PGRN for rapid endocytosis and degradation, and it is now well established that sortilin is the most important clearance receptor for PGRN (Hu et al., 2010). Thus, sortilin negatively regulates the extracellular levels of PGRN in the periphery as well as in the brain. Indeed, lack of or blocking the receptor increases plasma PGRN levels both in mice and humans (Carrasquillo et al., 2010; Gass, Prudencio, Stetler, & Petrucelli, 2012; Hu et al., 2010; Lee et al., 2014; Miyakawa et al., 2020; Pottier et al., 2018).

Frontotemporal dementia is a highly heritable dementia and haploinsufficiency of the PGRN gene accounts for up to 25% of all cases (Gijselinck, Van Broeckhoven, & Cruts, 2008). Patients with heterozygous loss-of-function mutations in PGRN have >50% reduced extracellular levels of the protein and will invariably develop FTD, making PGRN a causal gene for the disease (Baker et al., 2006; Carecchio et al., 2011; Cruts & Van Broeckhoven, 2008; Galimberti et al., 2010). In addition, PGRN mutant alleles have been identified in Alzheimer's (AD) patients (Brouwers et al., 2008; Sheng, Su, Xu, & Chen, 2014) and high levels of extracellular PGRN are protective in models of ALS, Parkinson's disease, stroke, arthritis, and atherosclerosis (Egashira et al., 2013; Laird et al., 2010; Martens et al., 2012; Tang et al., 2011; Tao, Ji, Wang, Liu, & Zhu, 2012; Van Kampen, Baranowski, & Kay, 2014).

Sortilin is, however, not required for PGRN to elicit its functions. Hence, neurons devoid in sortilin expression are equally responsive to PGRN-induced neuronal outgrowth (De Muynck et al., 2013; Gass, Lee, et al., 2012). Further, PGRN is successfully delivered to neuronal lysosomes in sortilin-deficient cells, suggesting the existence of alternative trafficking pathways. Indeed, PGRN can bind to the lysosomal protein, prosaposin (PSAP). When PSAP binds to its cognate receptors, the cation-independent mannose-6-phosphate receptor and LRP1, it brings along PGRN to the lysosomes (Zhou et al., 2015). Finally, in a phase II clinical trial with a monoclonal anti-sortilin antibody, markers for lysosomal integrity were normal (NCT03987295).

The functional PGRN receptor remains to be identified. However, studies suggest that PGRN promotes neuronal survival, reduces inflammation and increases A□ endocytosis by microglia (Martens et al., 2012; Pickford et al., 2011; Yin et al., 2010).

Binding of PGRN to sortilin requires the three amino acids in the C-terminal of PGRN (QLL in human, PLL in mouse), and a peptide derived from the last 24 amino acids of PGRN binds with similar affinity as the full-length protein (Zheng et al., 2011). It was proposed that this mode of binding is structurally similar to Neurotensin binding (Zheng et al., 2011), i.e. binding in the NTIS1 binding site of sortilin. There has been a successful small molecule screen that identified a blocker of Neurotensin to sortilin binding done in collaboration with Aarhus University (Andersen et al., 2014; Schroder et al., 2014).

Sortilin exists as a full-length and sorting competent receptor but is also capable of forming multimeric signalling receptor-ligand. Portions of sortilin can also be liberated from the plasma membrane to scavenge ligands (NT in pain) and control the activity of ligands. For example, sortilin is involved in synaptic plasticity by controlling the conversion rate of pro-BDNF into BDNF. This may also apply to other proneurotrophins.

Finally, the propeptide of sortilin, also named spadin, that is a ligand of the receptor has been demonstrated

to control the activity of the membrane transporter TREK-1, which is a target for major depression, among other diseases. Structurally, sortilin has an amino acid sequence according to SEQ ID NO: 1 and comprises a signal peptide, a propeptide, the Vps10p domain, a 10cc domain (10CCa + 10CCb), a transmembrane domain and a cytoplasmic tail. The luminal domain of sortilin has 6 potential N-linked glycosylation sites, whilst the cytoplasmic tail enables for the recruitment of various adapter proteins.

Sortilin binds to a vast number of ligands and membrane receptors and as a result engages in functions known to be important in cellular signalling and sorting. For example, sortilin is involved in signalling by proneurotrophins: the proforms of nerve growth factor (pro-NGF), brain derived neurotrophic factor (pro-BDNF), and neurotrophin-3 (proNT3), respectively. In complex with the protein p75NTR (p75 neurotrophin receptor), sortilin has been reported to form the receptor for proneurotrophin-mediated apoptotic effects leading to degeneration and cell death in cellular and animal models (Jansen et al., 2007; Tenk et al., 2005; Nykjaer et al., 2004).

Previous work has suggested a role for sortilin in cellular sorting and signalling associated with diseases such as diabetes and obesity (Huang et al., 2013). Sortilin facilitates translocation of GLUT4 to the plasma membrane and rescues it from degradation in the lysosomes (Pan et al., 2017). Sortilin levels have been shown to be modulated by the level of inflammation associated with these diseases. The pro-inflammatory cytokine, TNF$\alpha$, reduces both mRNA levels and protein levels of sortilin in cultured mouse and human adipocytes, as well as in vivo when injected into mice (Kaddai et al., 2009). Sortilin can also influence cytokine secretion: targeting sortilin in immune cells has been proposed to attenuate inflammation and reduce atherosclerosis disease progression (Mortensen et al., 2014). Additionally, US 2016/0331746 describes various scaffolds of small molecules capable of binding to the active site of sortilin. Sortilin is involved in the regulation of glucose uptake (Shi & Kandror. 2005) and the development of lipid disorder diseases (Gao et al., 2017).

Further, plasma sortilin levels have been reported to be a potential biomarker for identifying patients with either coronary heart disease or diabetes mellitus (Oh et al., 2017; Møller et al., 2021). Patients that showed increased sortilin levels within their plasma, and therefore identifiable as suffering from the above conditions, also displayed enhanced glucose levels suggesting sortilin as a therapeutic target for treating these conditions. Soluble sortilin is also proposed as a treatment for type II diabetes (WO2021116290 A1, 2021).

TAR DNA-binding protein 43 (TDP-43) has been implicated in various neurodegenerative diseases. For example, TDP-43 inclusion bodies have been found in cases of amyotrophic lateral sclerosis (ALS), frontotemporal lobar degeneration (FTLD), and Alzheimer's disease (AD) (Meneses et al., 2021).

TDP-43 regulates the splicing of several gene products, including Sortilin. In humans, the splicing involves inclusion of a cryptic exon 17b (between exon 17 and 18), which introduces a stopcodon in the stalk, potentially generating a non membrane bound fragment (Prudencio et al., 2012). Furthermore, it has been shown that PGRN can reduce levels of insoluble TDP-43 and slow down axonal degeneration (Beel et al., 2018). Sortilin inhibition increases PGRN levels and is therefore beneficial in the treatment of neurodegenerative diseases in which TDP 43 is implicated.

Sortilin has been linked to various conditions affecting the central nervous system (CNS). Some studies suggest a role of circulating sortilin in patients with psychiatric disorders such as depression, which may relate to altered activity of neurotrophic factors (Buttenshon et al., 2015); and it has also been reported that sortilin plays a role in brain aging, Alzheimer's Disease and frontotemporal dementia (Xu et al., 2019). However, delivering therapeutic agents that are capable of crossing the blood-brain barrier to the CNS presents a major challenge.

The blood-brain barrier is a highly selective semipermeable border of endothelial cells that prevents solutes in the circulating blood from non-selectively crossing into the extracellular fluid of the CNS where neurons reside. Therapy of neurological diseases is therefore limited due to the restricted penetration of therapeutic agents across the blood-brain barrier.

Therapeutic agents for treating CNS diseases therefore must be capable of crossing the blood-brain barrier. In addition to this, they must also have a sufficient unbound drug concentration in the brain, as the free drug hypothesis states that only unbound compound is able to interact with and elicit a pharmacological effect.

To determine the unbound fraction of a test compound (Fub), sample supernatants may be analysed by methods such as liquid chromatography with tandem mass spectrometry (LC-MS/MS). The unbound fraction may then be calculated from the peak area ratios obtained for each matrix according to the following formula:

$$\text{Fub} = \text{CPBS} / \text{Cplasma}$$

where CPBS and Cplasma are the analyte concentrations in PBS (receiver) and plasma (donor), respectively.

Recovery samples may be prepared in each condition but without dialysis, and may be used for evaluation of recovery from dialysis experiments using the following formula:

$$\% \text{ Recovery} = 100\times(\text{VPBS}\times\text{CPBS}+\text{Vplasma}\times\text{Cplasma})/\text{Vplasma}\times\text{Crecovery}$$

where VPBS is the volume on the receiver side (PBS) and Vplasma is the volume on donor side (plasma) of the dialysis device. Crecovery is the analyte concentration measured from the recovery sample. Compounds such as propranolol or fluoxetine, may be included in experiments as controls.

The unbound fraction in brain (Fub, brain) may be calculated from the measured value in brain homogenate (Fub, meas), taking into account the dilution factor used in preparing the brain homogenate:

$$F_{ub,\,brain} = \frac{1/D}{\left(\frac{1}{F_{ub,\,meas}}\right) - 1 + (\frac{1}{D})}$$

where D = dilution factor.

The brain/plasma unbound partition coefficient (Kpuu) may be determined as a ratio between the free compound concentrations in plasma and brain:

$$K_{puu} = \frac{C_{ub,brain}}{C_{ub,plasma}}$$

Where Cu,brain = unbound concentration in brain (C × Fub, brain); wherein

C = concentration at steady state; and
Cub,plasma = unbound concentration in plasma (C × Fub).

Alternatively a ratio of AUCs can be used to determine Kpuu. The compound of interest being dosed to a relevant species of interest at a known concentration by oral or intravenous route. Timecourses of concentration of compound in the plasma and cerebral spinal fluid (CSF) are measured. The plasma concentration is corrected to account for unbound fraction of compound. Areas under the CSF concentration curve and free fraction in plasma concentration curve are calculated by known methods and a ratio is determined to give:

$$\text{Kpuu} = \text{AUC0-infcsf} / (\text{AUC0-infplasma x } (\%\text{Fuplasma}/100))$$

For the treatment of CNS diseases, it is desirable for the Kpuu to have the highest possible value, above 0. A value around 1 indicating that the free fraction compound freely permeates the blood brain barrier; a value above 1 suggesting that an active influx transport mechanism at the blood brain barrier is involved; and less than 1, which indicates that the free fraction compound is either poorly permeable or is recognized by an active efflux mechanism, reducing the exposure in the CNS while passing back through the blood-brain barrier to plasma or the CSF. A Kpuu value of 0 or close to 0, indicates a poorly permeable compound or a highly active efflux mechanism that in any case will make highly improbable to reach a meaningful exposure in the CNS of the desired active species.

In addition to effectively targeting and binding to sortilin, compounds useful for modulating sortilin activity in a subject preferably have optimised pharmacokinetics. For example, this may equate to a prolonged half-life in vivo or improved delivery to target tissues. Improved persistence of such compounds in a subject is an important clinical parameter. This determines the required dosages and frequency of dosing of the compounds, as well as formulation requirements needed to achieve the desired therapeutic effect upon administration.

An increase in the in-vivo half-life ensures that the compounds remain therapeutically effective in the subject for as long as possible before being metabolised or excreted by the body, which permits administration of lower effective dosages and/or less frequent dosing of the therapeutic compounds. Accordingly, such compounds, with increased half-life are of significant pharmaceutical importance.

The current inventors have devised compounds which can modulate sortilin activity, as disclosed in European Application No. EP21194937.5 and PCT Application No. PCT/EP2022/074536, the entire contents of which are hereby incorporated by reference.

In view of the above, there is an unmet need for further compounds that may be used in the treatment and prevention of medical conditions in which modulation of sortilin is beneficial. In particular, there is an unmet need for further sortilin modulators that are capable of crossing the blood brain barrier and are therefore useful in the treatment

of diseases of the central nervous system. Furthermore, there is a need for sortilin modulators that have an improved half-life in vivo.

**BRIEF DESCRIPTION OF THE FIGURES**

Figures 1 to 10 depict x-ray derived images of Example 5 bound to h-Sortilin.

Figures 11A to 11C depict electron density maps from the x-ray diffraction data for Example 5.

**DISCLOSURE OF THE INVENTION**

Surprisingly, it has been found that compounds of Formula (I) modulate the activity of sortilin and are therefore useful in the treatment or prevention of conditions in which modulation of sortilin is beneficial. Furthermore, the compounds may cross the blood brain barrier and may therefore be particularly useful in the treatment of diseases of the central nervous system.

Without wishing to be bound by theory, it is believed that the backbone of the compounds of Formula (I) is not recognised by peptidases as a natural amino acid residue due to quaternary carbon adjacent to the carboxylic acid group. Accordingly, it is expected that the unnatural amino acid residue will resist degradation for longer and metabolise at a slower rate. It is therefore expected that the compounds of the invention will exhibit an improved half-life in vivo, for example compared to the compounds disclosed in EP21194937.5 and PCT/EP2022/074536.

In a first aspect of the invention, there is provided a compound of Formula (I):

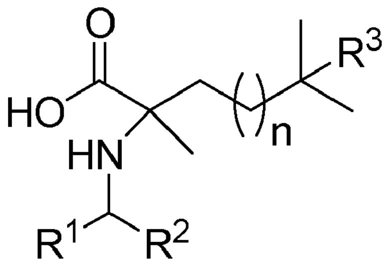

(I)

or a pharmaceutically acceptable salt, solvate, hydrate, tautomer, optical isomer, N-oxide, and/or prodrug thereof; wherein

$R^1$ is selected from the group consisting of:

(i) $C_1$-$C_4$ alkyl optionally substituted with $C_6$-$C_{10}$ aryl; and

(ii) $C_6$-$C_{10}$ aryl and 5 to 10-membered heteroaryl, optionally substituted with one or more substituents independently selected from the group consisting of - OH, halo, $C_1$-$C_4$ alkyl, $C_1$-$C_4$ alkoxy, $C_1$-$C_4$ hydroxyalkyl, $C_1$-$C_4$ haloalkyl, $C_1$-$C_4$ haloalkoxy, $C_1$-$C_4$ hydroxyalkoxy, acetyl, cyano, $C_6$-$C_{10}$ aryl, 5- to 10-membered heteroaryl, 5- to 10-membered heterocycloalkyl, -O-($C_6$-$C_{10}$ aryl), -O-$CH_2$-($C_6$-$C_{10}$ aryl), and -$NR^4R^5$; and

$R^2$ is selected from the group consisting of H, $C_1$-$C_4$ alkyl, $C_1$-$C_4$ haloalkyl, $C_1$-$C_4$ hydroxyalkyl, and phenyl;

$R^3$ is H or -$CH_3$;

$R^4$ and $R^5$ are each independently H or $C_1$-$C_4$ alkyl and

n = 0 or 1.

Surprisingly, the applicant has found that when $R^3$ is -$CH_3$ and n is 1, the compound of Formula (I) exhibits improved binding to sortilin. In a preferred aspect of the invention, the compound of Formula (I) therefore has the following formula:

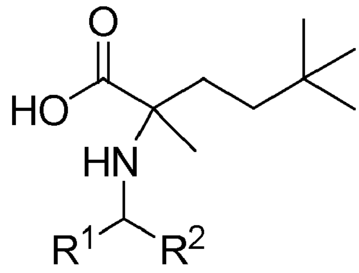

In the compounds of the invention, $R^2$ is preferably selected from the group consisting of H, $-CH_3$, $-CH_2F$, $-CHF_2$, $-CF_3$, $-(C_2H_4)-OH$, and phenyl, more preferably wherein $R^2$ is selected from the group consisting of H, $-CH_3$, $-CHF_2$, $-CF_3$, $-(C_2H_4)-OH$, and phenyl, more preferably H.

$R^4$ and $R^5$ are preferably each independently $C_1$-$C_2$ alkyl, more preferably $-CH_3$.

In a preferred aspect of the invention, $R^1$ is selected from the group consisting of:

(i) $C_1$-$C_2$ alkyl optionally substituted with phenyl; and

(ii) phenyl, naphthyl, 5- or 6-membered monocyclic heteroaryl, and 9- or 10-membered fused bicyclic heteroaryl, optionally substituted with one or more substituents independently selected from the group consisting of -OH, halo, $C_1$-$C_4$ alkyl, $C_1$-$C_4$ alkoxy, $C_1$-$C_4$ hydroxyalkyl, $C_1$-$C_4$ haloalkyl, $C_1$-$C_4$ haloalkoxy, $C_1$-$C_4$ hydroxyalkoxy, acetyl, cyano, $C_6$-$C_{10}$ aryl, 5- to 10-membered heteroaryl, 5- to 10-membered heterocycloalkyl, -O-($C_6$-$C_{10}$ aryl), -O-$CH_2$-($C_6$-$C_{10}$ aryl), and $-NR^4R^5$.

As used herein, the term "5- or 6-membered monocyclic heteroaryl" refers to an aromatic ring having 5 or 6 ring atoms, wherein at least one ring atom is a heteroatom and the remaining ring atoms are carbon.

As used herein, the term "9- or 10-membered fused bicyclic heteroaryl" refers to an aromatic ring system containing two rings fused together such that they share two adjacent ring atoms. Preferably, the 9- or 10-membered fused bicyclic heteroaryl group contains a 6-membered ring fused to a 5- or 6-membered ring.

The 9- or 10-membered fused bicyclic heteroaryl group has 9 or 10 ring atoms, wherein at least one ring atom is a heteroatom and the remaining ring atoms are carbon.

Preferably, each ring atom in the 5- or 6-membered monocyclic heteroaryl group and the 9- or 10-membered fused bicyclic heteroaryl group of $R^1$ is independently selected from the group consisting of C, N, S, and O. More preferably, one to three ring atoms are independently selected from the group consisting of N, S, and O and the remaining ring atoms are C.

More preferably, each ring atom in the 5- or 6-membered monocyclic heteroaryl group of $R^1$ is independently C or N. Most preferably, one or two ring atoms are N and the remaining ring atoms are C.

Examples of the 5- or 6-membered monocyclic heteroaryl group of $R^1$ include: pyrrolyl, pyrazolyl, imidazolyl, triazolyl, pyridyl, pyrimidinyl, pyrazinyl, pyridazinyl, and triazinyl. Preferably, the 5- or 6-membered heteroaryl group is selected from pyrazolyl, pyridyl, and pyrimidinyl. More preferably, the 5- or 6-membered heteroaryl group is one of the following groups:

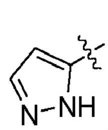 , 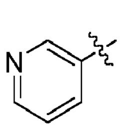 , 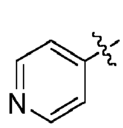 , 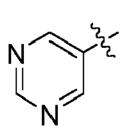 , and 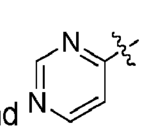 .

Examples of the 9- or 10-membered fused bicyclic heteroaryl group of $R^1$ include: quinolinyl, isoquinolinyl, quinoxalinyl, phthalazinyl, quinazolinly, cinnolinyl, naphthyridinyl, pyridopyrimidinyl, pyridopyrazinyl, indolyl, indazolyl, benzimidazolyl, azaindolyl, azaindazolyl, pyrazolopyrimidinyl, benzofuranyl, benzothiophenyl, benzoisoxazolyl, benzoisothiazolyl, benzoxazolyl, benzothiazolyl, benzothiadiazolyl, and benzotriazolyl. Preferably, the 9- or 10-membered fused bicyclic heteroaryl group is selected from quinolinyl, isoquinolinyl, quinoxalinyl, naphthyridinyl, indolyl, azaindolyl, indazolyl, benzimidazolyl, benzotriazolyl, benzothiazolyl, and benzofuranyl. More preferably, the 9- or 10-membered fused bicyclic heteroaryl group is one of the following groups:

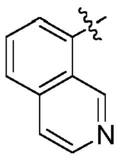, 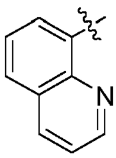, 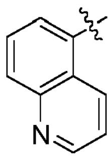, 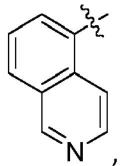, 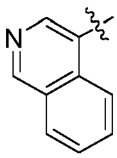, 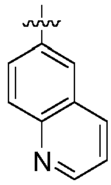, 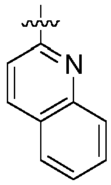, 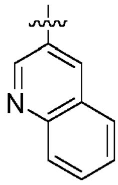, 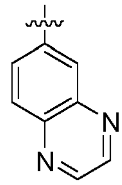

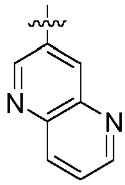, 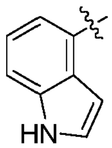, 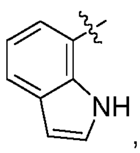, 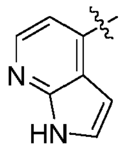, 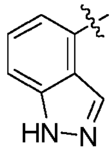, 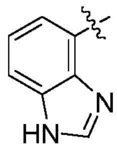, 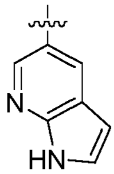, 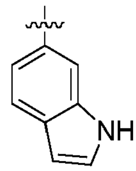,

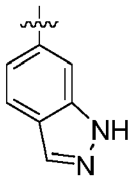, 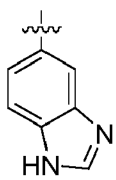, 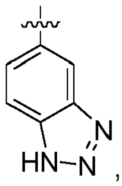, 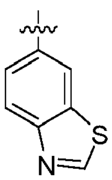, 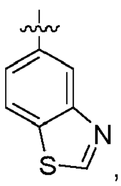, 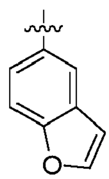, and 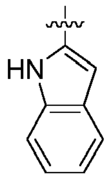.

In a preferred aspect of the invention, $R^1$ is selected from the group consisting of:

(i) $C_1$-$C_2$ alkyl optionally substituted with phenyl; and

(ii) phenyl, naphthyl, 5- or 6-membered monocyclic heteroaryl, and 9- or 10-membered fused bicyclic heteroaryl, optionally substituted one or more substituents independently selected from the group consisting of -OH, halo, $C_1$-$C_4$ alkyl, $C_1$-$C_4$ alkoxy, $C_1$-$C_4$ hydroxyalkyl, $C_1$-$C_4$ haloalkyl, $C_1$-$C_4$ haloalkoxy, acetyl, cyano, 5- or 6-membered heteroaryl, 5-membered heterocycloalkyl, -O-phenyl, and -$NR^4R^5$, preferably -OH, halo, $C_1$-$C_2$ alkyl, $C_1$-$C_2$ alkoxy, $C_1$-$C_2$ hydroxyalkyl, $C_1$-$C_2$ haloalkyl, $C_1$-$C_2$ haloalkoxy, acetyl, cyano, imidazolyl, triazolyl, pyridyl, pyrrolidinyl, -O-phenyl, and -$NR^4R^5$.

In a more preferred aspect of the invention, $R^1$ is selected from the group consisting of:

(i) $C_1$-$C_2$ alkyl optionally substituted with phenyl; and

(ii) phenyl and 5- or 6-membered monocyclic heteroaryl, optionally substituted with one or more substituents independently selected from the group consisting of -OH, halo, $C_1$-$C_2$ alkyl, $C_1$-$C_2$ alkoxy, $C_1$-$C_2$ hydroxyalkyl, $C_1$-$C_2$ haloalkyl, $C_1$-$C_2$ haloalkoxy, acetyl, cyano, imidazolyl, triazolyl, pyridyl, pyrrolidinyl, -O-phenyl, and -$NR^4R^5$; and

(iii) naphthyl and 9- or 10-membered fused bicyclic heteroaryl, optionally substituted with one or more substituents independently selected from the group consisting of halo, $C_1$-$C_2$ alkyl, and $C_1$-$C_2$ alkoxy.

In a more preferred aspect of the invention, $R^1$ is selected from the group consisting of:

(i) $C_1$-$C_2$ alkyl optionally substituted with phenyl;

(ii) phenyl optionally substituted with one or more substituents independently selected from the group consisting of -OH, halo, $C_1$-$C_2$ alkyl, $C_1$-$C_2$ alkoxy, $C_1$-$C_2$ hydroxyalkyl, $C_1$-$C_2$ haloalkyl, $C_1$-$C_2$ haloalkoxy, acetyl, cyano, imidazolyl, triazolyl, pyridyl, pyrrolidinyl, -O-phenyl, and -$NR^4R^5$;

(iii) 5- or 6-membered heteroaryl, optionally substituted with one or more substituents independently selected from the group consisting of halo, $C_1$-$C_2$ alkyl, $C_1$-$C_2$ alkoxy, and -O-phenyl;

(iv) naphthyl optionally substituted with one or more substituents independently selected from the group consisting of halo and $C_1$-$C_2$ alkoxy; and

(v) 9- or 10-membered fused bicyclic heteroaryl, optionally substituted with one or more substituents independently selected from the group consisting of halo and $C_1$-$C_2$ alkyl.

In a more preferred aspect of the invention, $R^1$ is selected from the group consisting of:

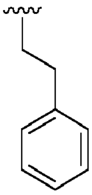, 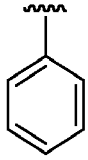, 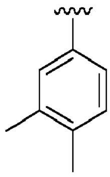, 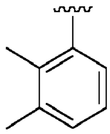, 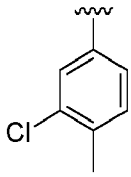, 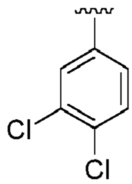, 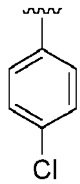, 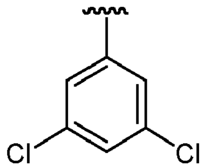,

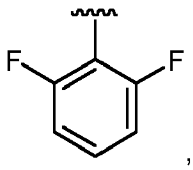, 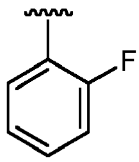, 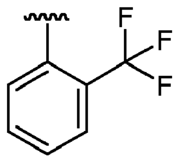, 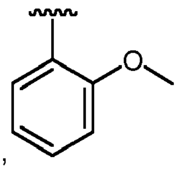, 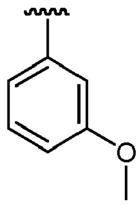, 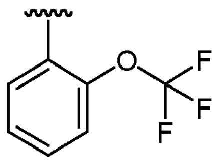,

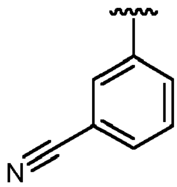, 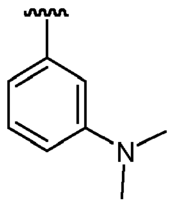, 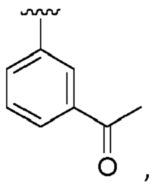, 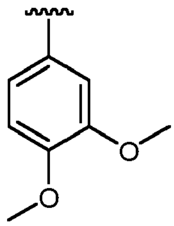, 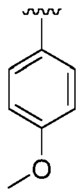, 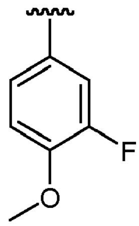, 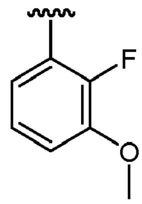,

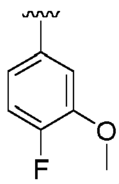, 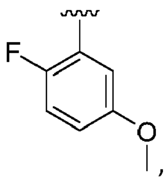, 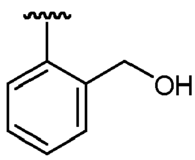, 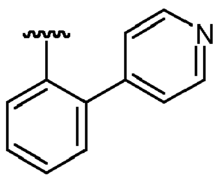, 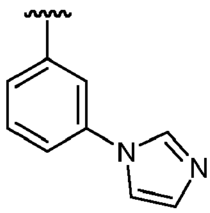,

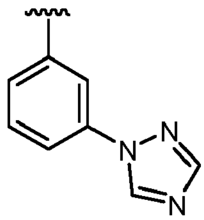, 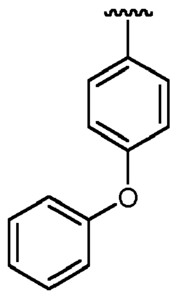, 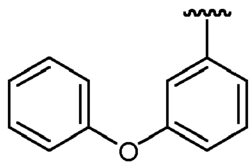, 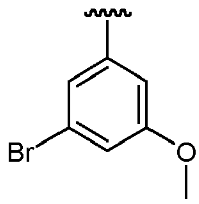, 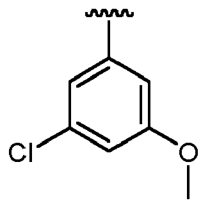,

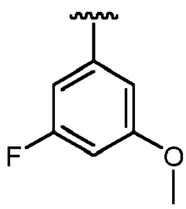, 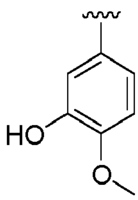, 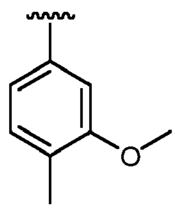, 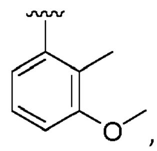, 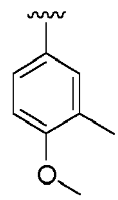, 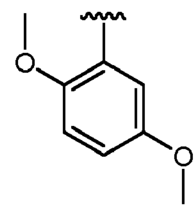,

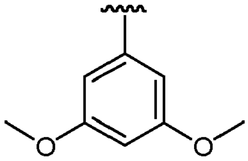, 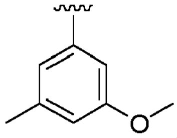, 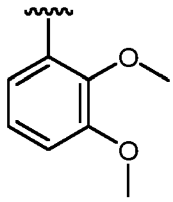, 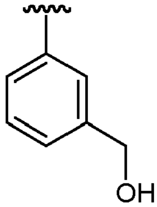, 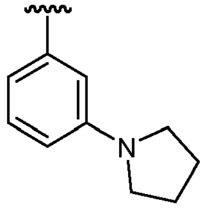,

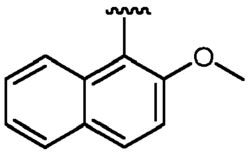, 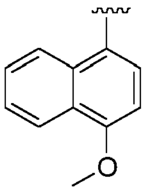, 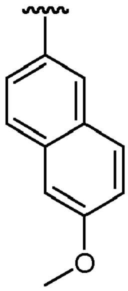, 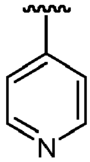, 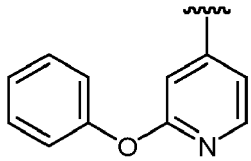, 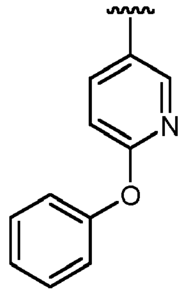,

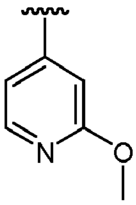, 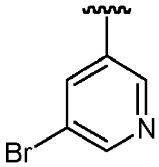, 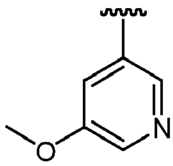, 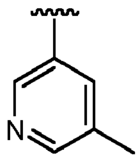, 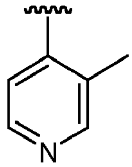, 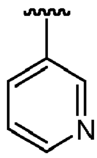, 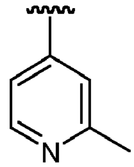,

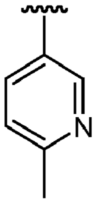, 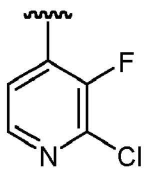, 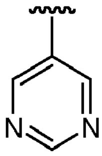, 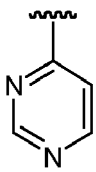, 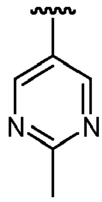, 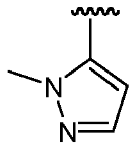, 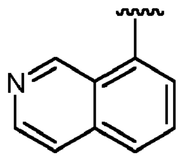,

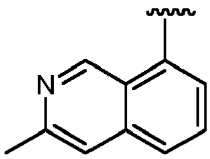, 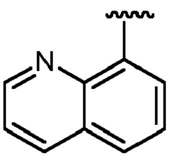, 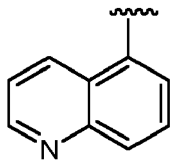, 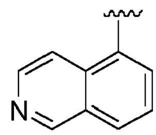, 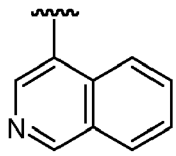, 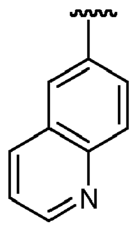,

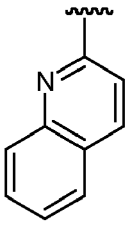, 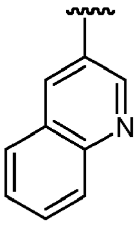, 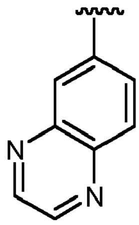, 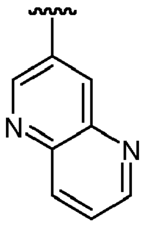, 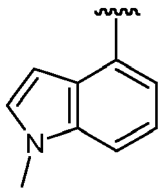, 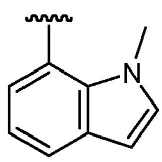, 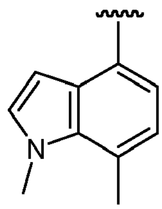

, 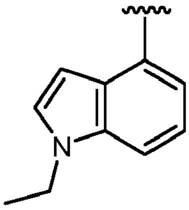 , 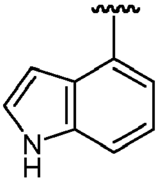 , 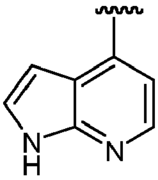 , 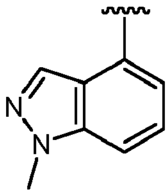 , 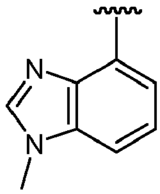 , 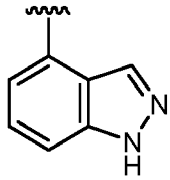 ,

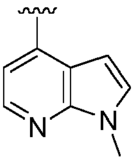 , 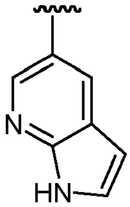 , 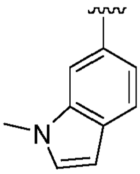 , 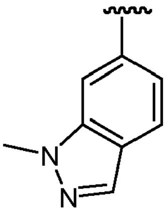 , 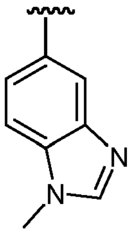 , 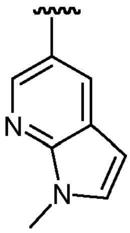 , 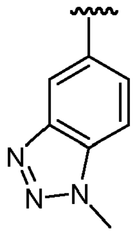 ,

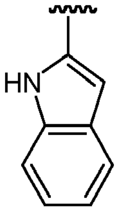 , 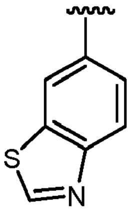 , 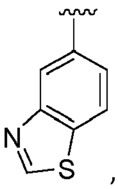 , 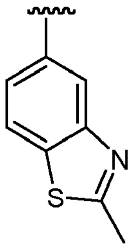 , and 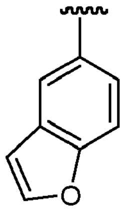 ,

more preferably $R^1$ is selected from the group consisting of:

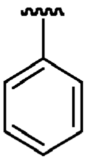 , 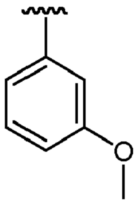 , 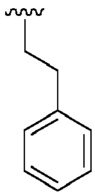 , and 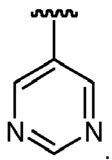 .

In the compounds of the invention, when $R^2$ is phenyl, it is preferred that $R^1$ is phenyl.

Particular compounds of the invention are those listed below.

(R)-2-benzylamino-2,5,5-trimethylhexanoic acid;

(S)-2-benzylamino-2,5,5-trimethylhexanoic acid;

(R)-2,5,5-trimethyl-2-(3-phenylpropylamino)hexanoic acid;

(S)-2,5,5-trimethyl-2-(3-phenylpropylamino)hexanoic acid-,

2,5,5-trimethyl-2-(3-phenylpropylamino)hexanoic acid;

(R)-2-{[(m-methoxyphenyl)methyl]amino}-2,5,5-trimethylhexanoic acid;

(S)-2-{[(m-methoxyphenyl)methyl]amino}-2,5,5-trimethylhexanoic acid;

(R)-2,5,5-trimethyl-2-{[(5-pyrimidinyl)methyl]amino}hexanoic acid;

(S)-2,5,5-trimethyl-2-{[(5-pyrimidinyl)methyl]amino}hexanoic acid;

2-{[(m-methoxyphenyl)methyl]amino}-2,5-dimethylhexanoic acid;

2,5,5-trimethyl-2-{[(5-pyrimidinyl)methyl]amino}hexanoic acid;

2-{[(m-methoxyphenyl)methyl]amino}-2,5,5-trimethylhexanoic acid;

2-benzylamino-2,5,5-trimethylhexanoic acid;

or a pharmaceutically acceptable salt, solvate, hydrate, tautomer, optical isomer, N-oxide, and/or prodrug thereof.

According to a second aspect of the invention, there is a pharmaceutical composition comprising a compound according to the invention and a pharmaceutically acceptable carrier, excipient, and/or diluent.

According to a third aspect of the invention, there is provided a compound or pharmaceutical composition according to the invention for use in therapy.

According to a fourth aspect of the invention, there is provided a compound or pharmaceutical composition according to the invention for use in the treatment or prevention of a neurodegenerative disorder, a psychiatric disorder, an inflammatory disorder, a lysosomal storage disorder, a cancer, pain, diabetes mellitus, retinopathies such as diabetic retinopathy, brain tumours, glaucoma, uveitis, cardiovascular diseases, kidney disease, psoriasis, hereditary eye conditions, chronic pain, hearing loss or diseases characterized by misfolded tau.

Preferably, the neurodegenerative disorder is selected from motor neuron diseases, Frontotemporal Lobar Degeneration (FTLD), frontotemporal dementia, Alzheimer's disease, Parkinson's disease, Huntington's disease, prion diseases such as Creutzfeldt-Jakob disease (CJD), acute brain injury, spinal cord injury and stroke, preferably wherein the motor neuron disease is selected from amyotrophic lateral sclerosis (ALS), Primary Lateral Sclerosis, and Progressive Muscular Atrophy.

The neurodegenerative disorder is preferably a neurodegenerative disorder characterised by misfolded TAR DNA binding protein 43 (tdp-43). In other words, the neurodegenerative disease is characterized by truncated tdp-43 and inclusion bodies. Examples of such diseases include amyotrophic lateral sclerosis, Alzheimer's disease, Frontotemporal Lobar Degeneration, and frontotemporal dementia.

Preferably, the psychiatric disorder is selected from bipolar disorder, major depression, post-traumatic stress disorder, and anxiety disorders;

Preferably, the inflammatory disorder may be selected from inflammatory diseases and neuroinflammation.

Preferably, the lysosomal storage disorder is selected from the group consisting of NCL/Batten Disease caused by mutations in CLN gene CLN1 (PPT1), CLN2 (TPP1), CLN3, CLN4 (DNAJC5), CLN5, CLN6, CLN7 (MFSD8), CLN8, CLN10 (CTSD), CLN11, CLN12 (ATP13A2), CLN13 (CTSF), CLN14 (KCTD7), CLCN6, and/or SGSH; Pompe disease, Fabry disease, Gaucher disease, Niemann-Pick disease Types A, B, and C; GM1 gangliosidosis, GM2 gangliosidosis (including Sandhoff and Tay-Sachs), mucopolysachariddoses (MPS) types I (Hurler disease)/II (Hunter disease)/IIIa (Sanfilippo A)/IIIB (Sanfilippo B)/IIIc (Sanfilippo C)/IIId (Sanfilippo D)/IVA (Morqui" A)/'VB/VI/VII (Sly)/IX, mucolipisosis III (I-cell) and IV, multiple sulfatase deficiency; sialidosis, galactosialidosis, $\alpha$-mannosidosis, $\beta$-mannosidosis, apartylglucosaminuria, fucosidosis, Schindler disease, metachromatic leukodystrophy caused by deficiencies in either arylsulfatase A or Saposin B, globoid cell leukodystrophy (Krabbe disease), Farber lipogranulomatosis, Wolman and cholesteryl ester storage disease, pycnodystostosis, cystinosis, Salla disease, Danon disease, Griscelli disease Types ½/3, Hermansky Pudliak Disease, and Chédiak-Higashi syndrome.

Preferably, the cancer is selected from breast cancer, lung cancer, ovarian cancer, prostate cancer, thyroid cancer, pancreatic cancer, glioblastoma and colorectal cancer.

Preferably, the cardiovascular disease is preferably selected from atherosclerosis, cardiomyopathy, heart attack, arrhythmias, heart failure, and ischemic heart disease.

Preferably, the hearing loss is selected from noise-induced hearing loss, ototoxicity induced hearing loss, age-induced hearing loss, idiopathic hearing loss, tinnitus and sudden hearing loss.

According to a sixth aspect of the invention, there is provided the use of the compound according to the invention for the manufacture of a medicament for the treatment or prevention of neurodegenerative disorder, a psychiatric disorder, an inflammatory disorder, a lysosomal storage disorder, a cancer, pain, diabetes mellitus, retinopathies such as diabetic retinopathy, brain tumours, glaucoma, uveitis, cardiovascular diseases, kidney disease, psoriasis, hereditary eye conditions, chronic pain, hearing loss or diseases characterized by misfolded tau.

According to a sixth aspect of the invention, there is provided a method for the treatment or prevention of a disease or condition responsive to sortilin modulation comprising administering a therapeutically effective amount of a compound or pharmaceutical composition according to the invention.

The compounds of the invention may include isotopically-labelled and/or isotopically-enriched forms of the compounds. The compounds of the invention herein may contain unnatural proportions of atomic isotopes at one or more of the atoms that constitute such compounds. Examples of isotopes that can be incorporated into the disclosed

compounds include isotopes of hydrogen, carbon, nitrogen, oxygen, phosphorus, sulfur, chlorine, such as 2H, 3H, 11C, 13C, 14C, 13N, 150, 170, 32P, 35S, 18F, 36Cl.

The compounds of the invention may be used as such or, where appropriate, as pharmacologically acceptable salts (acid or base addition salts) thereof. The pharmacologically acceptable addition salts mentioned below are meant to comprise the therapeutically active non-toxic acid and base addition salt forms that the compounds are able to form. Compounds that have basic properties can be converted to their pharmaceutically acceptable acid addition salts by treating the base form with an appropriate acid. Exemplary acids include inorganic acids, such as hydrogen chloride, hydrogen bromide, hydrogen iodide, sulphuric acid, phosphoric acid; and organic acids such as formic acid, acetic acid, propanoic acid, hydroxyacetic acid, lactic acid, pyruvic acid, glycolic acid, maleic acid, malonic acid, oxalic acid, benzenesulphonic acid, toluenesulphonic acid, methanesulphonic acid, trifluoroacetic acid, fumaric acid, succinic acid, malic acid, tartaric acid, citric acid, salicylic acid, p-aminosalicylic acid, pamoic acid, benzoic acid, ascorbic acid and the like. Compounds that have acidic properties can be converted to their pharmaceutically acceptable basic addition salts by treating the acid form with an appropriate base. Exemplary base addition salt forms are the sodium, potassium, calcium salts, and salts with pharmaceutically acceptable amines such as, for example, ammonia, alkylamines, benzathine, and amino acids, such as, e.g. arginine and lysine. The term addition salt as used herein also comprises solvates which the compounds and salts thereof are able to form, such as, for example, hydrates, alcoholates and the like.

Throughout the present disclosure, a given chemical formula or name shall also encompass all pharmaceutically acceptable salts, solvates, hydrates, N-oxides, and/or prodrug forms thereof. It is to be understood that the compounds of the invention include any and all hydrates and/or solvates of the compound formulas. It is appreciated that certain functional groups, such as the hydroxy, amino, and like groups form complexes and/or coordination compounds with water and/or various solvents, in the various physical forms of the compounds. Accordingly, the above formulas are to be understood to include and represent those various hydrates and/or solvates.

Compounds of the invention also include tautomeric forms. Tautomeric forms result from the swapping of a single bond with an adjacent double bond together with the concomitant migration of a proton. Tautomeric forms include prototropic tautomers which are isomeric protonation states having the same empirical formula and total charge. Example prototropic tautomers include ketone - enol pairs, amide - imidic acid pairs, lactam - lactim pairs, amide - imidic acid pairs, enamine - imine pairs, and annular forms where a proton can occupy two or more positions of a heterocyclic system, for example, 1H- and 3H-imidazole, 1H, 2Hand 4H- 1,2,4-triazole, 1H- and 2H- isoindole, and 1H- and 2H-pyrazole. Tautomeric forms can be in equilibrium or sterically locked into one form by appropriate substitution.

The compounds described herein can be asymmetric (e.g. having one or more stereogenic centres). All stereoisomers, such as enantiomers and diastereomers, are intended unless otherwise indicated. Compounds of the present invention that contain asymmetrically substituted carbon atoms can be isolated in optically active or racemic forms. Methods on how to prepare optically active forms from optically active starting materials are known in the art, such as by resolution of racemic mixtures or by stereoselective synthesis. Many geometric isomers of olefins, C=N double bonds, and the like can also be present in the compounds described herein, and all such stable isomers are contemplated in the present invention. Cis- and trans-geometric isomers of the compounds of the present invention are described and may be isolated as a mixture of isomers or as separated isomeric forms.

In the case of the compounds which contain an asymmetric carbon atom, the invention relates to the D form, the L form, and D,L mixtures and also, where more than one asymmetric carbon atom is present, to the diastereomeric forms. Those compounds of the invention which contain asymmetric carbon atoms, and which as a rule accrue as racemates, can be separated into the optically active isomers in a known manner, for example using an optically active acid. However, it is also possible to use an optically active starting substance from the outset, with a corresponding optically active or diastereomeric compound then being obtained as the end product.

Preferably, the compounds of Formula (I) are compounds of Formula (Ia)

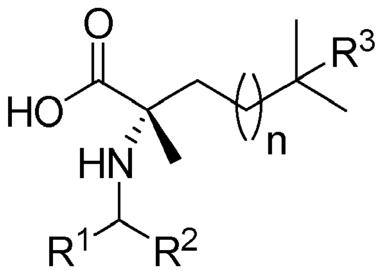

(Ia)

The term "prodrugs" refers to compounds that may be converted under physiological conditions or by solvolysis to a biologically active compound of the invention. A prodrug may be inactive when administered to a subject in need thereof, but is converted in vivo to an active compound of the invention. Prodrugs are typically rapidly transformed in vivo to yield the parent compound of the invention, e.g. by hydrolysis in the blood. The prodrug compound usually offers advantages of solubility, tissue compatibility or delayed release in a mammalian organism (see Silverman, R. B., The Organic Chemistry of Drug Design and Drug Action, 2nd Ed., Elsevier Academic Press (2004), page 498 to 549). Prodrugs of a compound of the invention may be prepared by modifying functional groups, such as a hydroxy, amino or mercapto groups, present in a compound of the invention in such a way that the modifications are cleaved, either in routine manipulation or in vivo, to the parent compound of the invention. Examples of prodrugs include, but are not limited to, acetate, formate and succinate derivatives of hydroxy functional groups or phenyl carbamate derivatives of amino functional groups.

The compounds of the invention may be sortilin inhibitors, binders, modulators or antagonists. As used herein, the term "sortilin antagonist", "sortilin inhibitor", "sortilin binder" or "sortilin modulator" (used interchangeably) refers to a substance that interferes with, blocks, or otherwise attenuates the effect of, a sortilin protein binding to progranulin, or neurotensin or another extracellular ligand, or a pro-neurotrophin (e.g., pro-NGF, proNT3, pro-BDNF) or preventing the formation of the trimeric complex between sortilin, p75NTR and the pro-neurotrophin. The term "sortilin antagonist" also includes a substance or agent that interferes with the formation of a high affinity trimeric complex. In the latter scenario, it is recognised that a trimeric complex may be formed in that sortilin can bind to p75NTR (but not pro-NGF) and p75NTR can simultaneously bind the NGF domain of pro-NGF. However, the resulting trimeric complex may be of lower affinity for its receptor and as a result have significantly reduced capacity to stimulate apoptosis via the mechanism described above. Skeldal et al. (2012) demonstrated that the apoptotic function of the trimeric complex is abolished when sortilin is devoid in its intracellular domain. The term "sortilin antagonist" also includes a substance or agent that interferes with, blocks, or otherwise attenuates the effect of, a sortilin protein interacting with p75NTR. This interaction may be completely prevented, in which case the trimeric complex is prevented from forming, or only partially prevented, in which case the trimeric complex may be formed but may have reduced biological potency. Skeldal et al showed that complex formation between sortilin and p75NTR relies on contact points in the extracellular domains of the receptors and that the interaction critically depends on an extracellular juxtamembrane 23-amino acid sequence of p75NTR. Thus, the sortilin antagonist may interfere with this 23-amino acid sequence or proximal sequences in the molecules. "Sortilin antagonists" may act as ligand cellular uptake inhibitors wherein the ligands may be progranulin, neurotensin, BDNF etc.

The compounds of the invention may cross the blood brain barrier and therefore may be particularly useful in the treatment or prevention of diseases of the central nervous system, including neurodegenerative disorders selected from motor neuron diseases, Frontotemporal Lobar Degeneration (FTLD), frontotemporal dementia, Alzheimer's disease, Parkinson's disease, Huntington's disease, prion diseases such as Creutzfeldt-Jakob disease (CJD), acute brain injury, spinal cord injury and stroke; a psychiatric disorder selected from bipolar disorder, major depression, post-traumatic stress disorder, and anxiety disorders; hearing loss selected from noise-induced hearing loss, ototoxicity induced hearing loss, age-induced hearing loss, idiopathic hearing loss, tinnitus and sudden hearing loss; brain tumours, retinopathies, glaucoma, neuroinflammation, chronic pain and diseases characterized by misfolded tau.

The compounds of the invention may have a Kpuu of more than 0.1, such as between 0.1 and 10, between 0.1 and 5, between 0.1 and 3, between 0.1 and 2, between 0.1 and 1, between 0.1 and 0.8, between 0.1 and 0.6, between 0.1 and 0.5, between 0.1 and 0.4, between 0.1 and 0.3, or between 0.1 and 0.2.

The term "treatment" as used herein may include prophylaxis of the named disorder or condition, or amelioration or elimination of the disorder once it has been established. The term "prevention" refers to prophylaxis of the named disorder or condition.

Methods delineated herein include those wherein the subject is identified as in need of a particular stated treatment. Identifying a subject in need of such treatment can be in the judgment of a subject or a health care professional and can be subjective (e.g. opinion) or objective (e.g. measurable by a test or diagnostic method).

In other aspects, the methods herein include those further comprising monitoring subject response to the treatment administrations. Such monitoring may include periodic imaging or sampling of subject tissue, fluids, specimens, cells, proteins, chemical markers, genetic materials, etc. as markers or indicators of the treatment regimen. In other methods, the subject is pre-screened or identified as in need of such treatment by assessment for a relevant marker or indicator of suitability for such treatment.

The invention provides a method of monitoring treatment progress. The method includes the step of determining a level of diagnostic marker (Marker) (e.g. any target or cell type delineated herein modulated by a compound herein) or diagnostic measurement (e.g., screen, assay) in a subject suffering from or susceptible to a disorder or symptoms thereof delineated herein, in which the subject has been administered a therapeutic amount of a compound herein sufficient to treat the disease or symptoms thereof. The level of Marker determined in the method can be compared to known levels of Marker in either healthy normal controls or in other afflicted patients to establish the subject's disease status. In preferred embodiments, a second level of Marker in the subject is determined at a time point later than the

determination of the first level, and the two levels are compared to monitor the course of disease or the efficacy of the therapy. In certain preferred embodiments, a pre-treatment level of Marker in the subject is determined prior to beginning treatment according to this invention; this pre-treatment level of Marker can then be compared to the level of Marker in the subject after the treatment commences, to determine the efficacy of the treatment.

A level of Marker or Marker activity in a subject may be determined at least once. Comparison of Marker levels, e.g., to another measurement of Marker level obtained previously or subsequently from the same patient, another patient, or a normal subject, may be useful in determining whether therapy according to the invention is having the desired effect, and thereby permitting adjustment of dosage levels as appropriate. Determination of Marker levels may be performed using any suitable sampling/expression assay method known in the art or described herein. Preferably, a tissue or fluid sample is first removed from a subject. Examples of suitable samples include blood, urine, tissue, mouth or cheek cells, and hair samples containing roots. Other suitable samples would be known to the person skilled in the art. Determination of protein levels and/or mRNA levels (e.g., Marker levels) in the sample can be performed using any suitable technique known in the art, including, but not limited to, enzyme immunoassay, ELISA, radiolabelling/assay techniques, blotting/chemiluminescence methods, real-time PCR, and the like.

For clinical use, the compounds disclosed herein are formulated into pharmaceutical compositions (or formulations) for various modes of administration. It will be appreciated that compounds of the invention may be administered together with a physiologically acceptable carrier, excipient, and/or diluent (i.e. one, two, or all three of these). The pharmaceutical compositions disclosed herein may be administered by any suitable route, preferably by oral, rectal, nasal, topical (including ophthalmic, buccal and sublingual), sublingual, transdermal, intrathecal, transmucosal or parenteral (including subcutaneous, intramuscular, intravenous and intradermal) administration. Other formulations may conveniently be presented in unit dosage form, e.g., tablets and sustained release capsules, and in liposomes, and may be prepared by any methods well known in the art of pharmacy. Pharmaceutical formulations are usually prepared by mixing the active substance, or a pharmaceutically acceptable salt thereof, with conventional pharmaceutically acceptable carriers, diluents or excipients. Examples of excipients are water, gelatin, gum arabicum, lactose, microcrystalline cellulose, starch, sodium starch glycolate, calcium hydrogen phosphate, magnesium stearate, talcum, colloidal silicon dioxide, and the like. Such formulations may also contain other pharmacologically active agents, and conventional additives, such as stabilizers, wetting agents, emulsifiers, flavouring agents, buffers, and the like. Usually, the amount of active compounds is between 0.1-95% by weight of the preparation, preferably between 0.2-20% by weight in preparations for parenteral use and more preferably between 1-50% by weight in preparations for oral administration. The formulations can be further prepared by known methods such as granulation, compression, microencapsulation, spray coating, Etc. The formulations may be prepared by conventional methods in the dosage form of tablets, capsules, granules, powders, syrups, suspensions, suppositories or injections. Liquid formulations may be prepared by dissolving or suspending the active substance in water or other suitable vehicles. Tablets and granules may be coated in a conventional manner. To maintain therapeutically effective plasma concentrations for extended periods of time, compounds disclosed herein may be incorporated into slow-release formulations.

The dose level and frequency of dosage of the specific compound will vary depending on a variety of factors including the potency of the specific compound employed, the metabolic stability and length of action of that compound, the patient's age, body weight, general health, sex, diet, mode and time of administration, rate of excretion, drug combination, the severity of the condition to be treated, and the patient undergoing therapy. The daily dosage may, for example, range from about 0.001 mg to about 100 mg per kilo of body weight, administered singly or multiply in doses, e.g. from about 0.01 mg to about 25 mg each. Normally, such a dosage is given orally but parenteral administration may also be chosen.

## DEFINITIONS

As used herein, the term "sortilin" may refer to full length sortilin (also referred to as immature sortilin), comprising a signal peptide, a propeptide, a Vps10p domain, a 10CC domain, a transmembrane domain and a large cytoplasmic tail, having an amino acid sequence according to SEQ ID NO: 1 or SEQ ID NO: 2, or it may refer to mature sortilin, comprising a Vps10p domain, a 10CC domain, a transmembrane domain and a large cytoplasmic tail, having an amino acid sequence according to SEQ ID NO: 3, or a naturally occurring fragment, homologue or variant thereof. The term "sortilin" or "sortilin molecule" are used interchangeably herein. It is understood that sortilin is capable of interacting with a pro-neurotrophin molecule to form a sortilin/pro-neurotrophin complex. This sortilin/pro-neurotrophin complex may or may not be capable of interacting with a p75NTR molecule to form a trimeric complex comprising sortilin, pro-neurotrophin and p75NTR. It is understood that this trimeric complex may be responsible for adverse biological responses, such as stimulating apoptosis in retinal and ganglion cells, and controlling growth cone retraction of projecting axons (Jansen et al., 2007; Nykjaer et al., 2004; Santos et al., 2012; Skeldal et al., 2012).

As used herein, the term "pro-neurotrophin" refers to the larger precursors of neurotrophins, which undergo proteolytic cleavage to yield the mature form of the neurotrophin. Neurotrophins are a family of proteins that induce the

survival, development and function of neurons, and are commonly referred to as growth factors. Pro-neurotrophins are biologically active and have distinct roles compared to their neurotrophin counterparts, such as induction of apoptosis. Examples of pro-neurotrophins include pro-NGF, pro-BDNF, proNT3 and proNT4. Pro-neurotrophins may also control synaptic plasticity. Whereas mature neurotrophins induce synaptic strength, in their proforms they may weaken synapses.

"Optional" or "optionally" means that the subsequently described event or circumstance may, but need not, occur, and that the description includes instances where the event or circumstance occurs and instances in which it does not.

The term "heteroatom" means O, N, or S.

The term "($C_1$-$C_n$)alkyl" denotes a straight, branched, cyclic, or partially cyclic alkyl group having from 1 to n carbon atoms, i.e. 1, 2, 3... or n carbon atoms. For the "($C_1$-$C_n$)alkyl" group to comprise a cyclic portion it should be formed of at least three carbon atoms. For parts of the range "($C_1$-$C_n$)alkyl" all subgroups thereof are contemplated. For example, in the range ($C_1$-$C_4$)alkyl, all subgroups such as ($C_1$-$C_4$)alkyl, ($C_1$-$C_3$)alkyl, ($C_1$-$C_2$)alkyl, ($C_1$)alkyl, ($C_2$-$C_4$)alkyl, ($C_2$-$C_3$)alkyl, ($C_2$)alkyl, ($C_3$-$C_4$)alkyl, ($C_3$)alkyl, and ($C_4$)alkyl. Examples of "$C_1$-$C_4$ alkyl" include methyl, ethyl, n-propyl, isopropyl, cyclopropyl, n-butyl, isobutyl, sec-butyl, t-butyl, cyclobutyl etc.

The term "($C_1$-$C_n$) haloalkyl" denotes a $C_1$-$C_n$ alkyl as described above substituted with at least one halogen atom, which is preferably, F, Cl, Br and I, more preferably F and Cl, and most preferably F.

The term "($C_1$-$C_n$) hydroxyalkyl" denotes a $C_1$-$C_n$ alkyl as described above substituted with at least one -OH group.

The term "($C_1$-$C_n$)alkoxy" denotes -O-(($C_1$-$C_n$)alkyl) in which a ($C_1$-$C_n$)alkyl group is as defined above and is attached to the remainder of the compound through an oxygen atom.

The term "($C_1$-$C_n$) haloalkoxy" denotes a $C_1$-$C_n$ alkoxy as described above substituted with at least one halogen atom, which is preferably, F, Cl, Br and I, more preferably F and Cl, and most preferably F.

The term "($C_1$-$C_n$) hydroxyalkoxy" denotes a $C_1$-$C_n$ alkoxy as described above substituted with at least one -OH group.

When a term denotes a range, for instance "1 to 4 carbon atoms" in the definition of ($C_1$-$C_4$)alkyl, each integer is considered to be disclosed, i.e. 1, 2, 3, and 4.

The term "halo" means a halogen atom, and is preferably, F, Cl, Br and I, more preferably F, C!, and Br.

The term "$C_6$-$C_{10}$" aryl denotes an aromatic monocyclic or fused bicyclic hydrocarbon ring system comprising 6 to 10 ring atoms.

The term "5- to 10-membered heterocycloalkyl" denotes a non-aromatic ring system having 5 to 10 ring atoms, in which at least one ring atom is a heteroatom and the remaining ring atoms are carbon. Preferably each heteroatom is independently selected from N, S, or O, more preferably N. Preferably, no more than 2 ring atoms are a heteroatom. More preferably, only one ring atom is a heteroatom.

"An effective amount" refers to an amount of a compound of the invention that confers a therapeutic effect on the treated subject. The therapeutic effect may be objective (i.e. measurable by some test or marker) or subjective (i.e. subject gives an indication of or feels an effect).

As used herein, the terms "administration" or "administering" mean a route of administration for a compound disclosed herein. Exemplary routes of administration include, but are not limited to, oral, intraocular, intravenous, intraperitoneal, intraarterial, and intramuscular. The preferred route of administration can vary depending on various factors, e.g. the components of the pharmaceutical composition comprising a compound disclosed herein, site of the potential or actual disease and severity of disease.

The terms "subject" and "patient" are used herein interchangeably. They refer to a human or another mammal (e.g., mouse, rat, rabbit, dog, cat, cattle, swine, sheep, horse or primate) that can be afflicted with or is susceptible to a disease or disorder but may or may not have the disease or disorder. It is preferred that the subject is human.

Compounds of the invention may be disclosed by the name or chemical structure. If a discrepancy exists between the name of a compound and its associated chemical structure, then the chemical structure prevails.

The invention will now be further illustrated by the following non-limiting examples. The specific examples below are to be construed as merely illustrative, and not limitative of the remainder of the disclosure in any way whatsoever. Without further elaboration, it is believed that one skilled in the art can, based on the description herein, utilise the present invention to its fullest extent. All references and publications cited herein are hereby incorporated by reference in their entirety.

## PREPARATION OF COMPOUNDS OF THE INVENTION

The compounds of the invention can be prepared according to the following General Synthetic Procedures scheme by methods well known and appreciated in the art. Suitable reaction conditions are well known in the art and appropriate substitutions of solvents and co-reagents are within the common general knowledge of the person skilled in the art. Likewise, it will be appreciated by those skilled in the art that synthetic intermediates may be isolated and/or

purified by various well-known techniques as needed or desired, and that frequently, it will be possible to use various intermediates directly in subsequent synthetic steps with little or no purification. Furthermore, the skilled person will appreciate that in some circumstances, the orders in which moieties are introduced is not critical. The particular order of steps required to produce the compounds of formula (I) is dependent upon the particular compound being synthesized, the starting compound, and the relative liability of the substituted moieties as is well appreciated by those of ordinary skill in the art. All substituents, unless otherwise indicated, are as previously defined, and all reagents are well known and appreciated in the art.

Suitable starting materials, either optically active as single enantiomers or as racemic mixtures and protected amino acids of general formula AA-1 are either commercially available or may be prepared by a variety of methods. For example, as illustrated in the General Synthetic Procedure, Scheme 1, the carboxylic acid functionality of appropriately substituted amino acids of general formula AA-1, can be used as the free acid, PG = H, or protected as a suitable derivative, for example as a methyl ester. Functionalisation of the primary amine present in Int 1 can be done by a variety of methods, and for the purpose of exemplification, by a condensation step involving a suitably substituted carbonyl compound aldehyde or ketone to yield an imine type Int 2. When treated by a reductive reagent, as for example but not limited to, sodium triacetoxy borohydride in a suitable solvent mixture like acetic acid and dichloromethane the imine function can be reduced to afford an intermediate of type Int 3 that is a precursor of compounds of Formula (I) after liberation of the carboxylic acid functionality. Alternatively, an amino acid of type AA-2 can be used as starting material. By using, as previously discussed, a condensation step to obtain an intermediate of type Int 4, as shown in Scheme 2, that when treated with a suitable base, will lose its most acidic proton, resulting in a transient carbanion that can, thus, be quenched by an alkylating agent such as, but not limited to, Methyl iodide, resulting in a compound of type Int 2. When treated by a reductive reagent, as for example but not limited to, sodium triacetoxy borohydride in a suitable solvent mixture like acetic acid and dichloromethane the imine function can be reduced to afford an intermediate of type Int 3 or hydrolysed by treatment with an aqueous solution acidic or basic to provide the generic intermediate Int 5. As in Scheme 1, Compounds of Formula (I) can then be obtained directly from Int 3 by liberation of the acidic moiety, with or without, further functionalisation of the amine group.

Another possible approach from a readily amenable starting amino acid of the type AA-3, as represented in the general Synthetic Procedures Scheme 3, uses an alkylation step between the suitable protected AA-3 or Int 6 and a reagent of type, for exemplification purposes, 1-bromo-3,3-dimethylbutane to yield generic intermediates of type Int 2, previously discussed. Advantageously, intermediates of type AA-1 to AA-3 can be obtained by procedures known, to those skilled in the art, or purchased from commercial sources.

GENERAL SYNTHETIC PROCEDURES

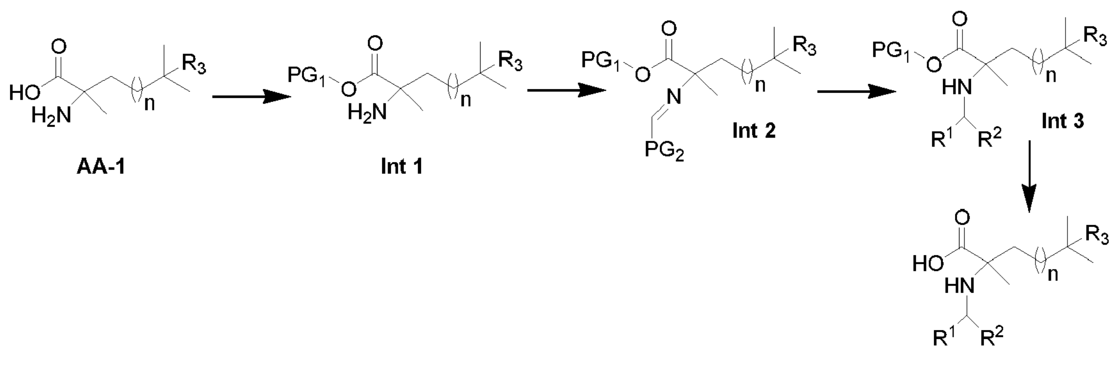

AA-1 Int 1 Int 2 Int 3 Formula (I)

## Scheme 1

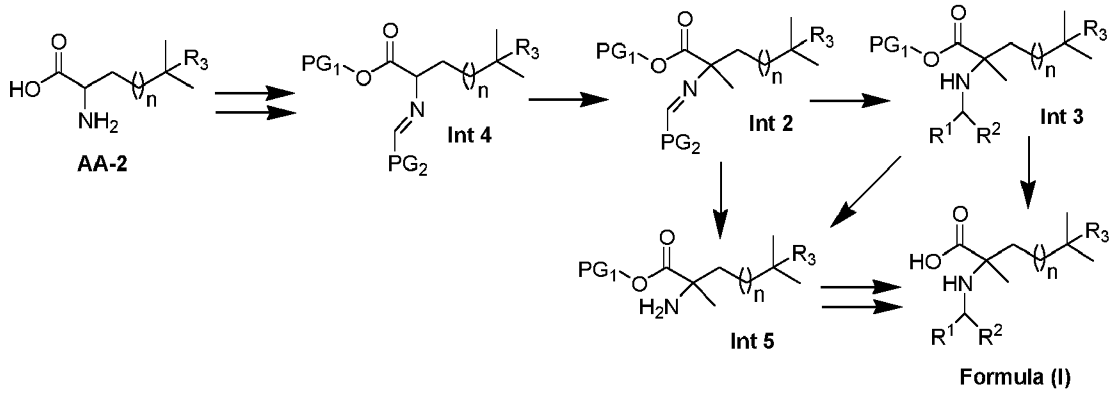

## Scheme 2

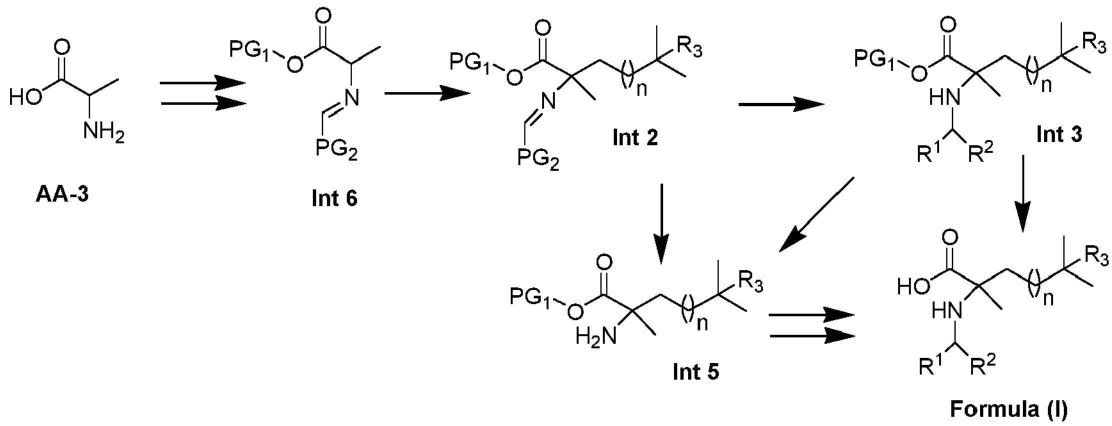

## Scheme 3

The compounds of general formula (I) may be prepared by a variety of procedures, some of which are described below. The products of each step can then be recovered by conventional methods including extraction, evaporation, precipitation, chromatography, filtration, trituration, crystallisation and the like.

Compounds of general formula (I) may contain one or more stereogenic centres. Those can be introduced from available single enantiomers, optically active, starting materials of the type AA-1, AA-2, or AA-3. The integrity of the existing stereogenic centre can be confirmed by analytical techniques well known to those skilled in the art like for example chiral support high pressure chromatography. Alternatively, when racemic starting materials are used, it will be appreciated that if desired, single isomer products can be obtained as single enantiomers or as single diastereoisomers, by known techniques like preparative chiral support high pressure chromatography.

The skilled artisan will also appreciate that not all of the substituents in the compounds of formula (I) will tolerate certain reaction conditions employed to synthesise the compounds. These moieties may be introduced at a convenient point in the synthesis, or may be protected and then deprotected as necessary or desired, as is well known in the art. The skilled artisan will appreciate that the protecting groups may be removed at any convenient point in the synthesis of the compounds of the present invention. Methods for introducing or removing protecting groups used in this invention are well known in the art; see, for example, Greene and Wuts, Protective Groups in Organic Synthesis, 4th Ed., John Wiley and Sons, New York (2006).

## EXAMPLES

### Abbreviations

approx: approximately; aq: aqueous; br: broad; ca.: circa; CDI: 1,1'-Carbonyldiimidazole; CPME: cyclopentyl methyl ether; d: doublet; DCM: dichloromethane; DIC: N,N'-Diisopropylcarbodiimide; dioxane: 1,4-dioxane; DIPEA: diisopropylethylamine; DMF: dimethylformamide; eq.: equivalent; Et3N: triethylamine; EtOAc: ethyl acetate; EtOH: ethanol; Fmoc: fluorenylmethoxycarbonyl; Boc: tert-butoxycarbonyl; h: hours; min: minutes: HATU: 2-(3H-[1,2,3]triazolo[4,5-b]pyridin-3-yl)-1,1,3,3-tetramethyl isouronium hexafluorophosphate(V); HPLC: high performance liquid chromatography; IPA, isopropanol; LC: liquid chromatography; m: multiplet; M: molar, molecular ion; MeCN: acetonitrile; MeOH: methanol; MS: mass spectrometry; NMR: nuclear magnetic resonance; PDA: photodiode array; q: quartet; rt: room temperature (ca. 20 °C); RT: retention time; s: singlet, solid; SPPS: solid phase peptide synthesis. t: triplet; TBAF: tetrabutylammonium fluoride; TBME: tert-butyl methyl ether; TFA: trifluoroacetic acid; THF: tetrahydrofuran; UPLC: ultra-performance liquid chromatography; UV: ultraviolet.

Other abbreviations are intended to convey their generally accepted meaning.

**General Experimental Conditions**

All starting materials and solvents were obtained either from commercial sources or prepared according to the literature citation. Reaction mixtures were magnetically stirred, and reactions performed at room temperature (ca. 20 °C) unless otherwise indicated.

Column chromatography was performed on an automated flash chromatography system, such as a CombiFlash Rf system, using pre-packed silica (40 μm) cartridges, unless otherwise indicated.

**Analytical Methods**

1H-NMR spectra were recorded at 400 MHz on a Bruker Avance AV-I-400 or on a Bruker Avance AV-II-400 instrument. Chemical shift values are expressed in ppm-values relative to tetramethylsilane unless noted otherwise. The following abbreviations or their combinations are used for multiplicity of NMR signals: br = broad, d = doublet, m = multiplet, q = quartet, quint = quintet, s = singlet and t = triplet.

Method 1: Apparatus: Waters IClass; Bin. Pump: UPIBSM, SM: UPISMFTN with SO; UPCMA, PDA: UPPDATC, 210-320 nm, SQD: ACQ-SQD2 ESI; ELSD: gaspressure 40 psi, drift tube temp: 50°C; column: Waters XSelect CSH C18, 50×2.1mm, 2.5μm, Temp: 25°C, Flow: 0.6 mL/min, Gradient: t0 = 5% B, t2.0min = 98% B, t2.7min = 98% B, Posttime: 0.3 min, Eluent A: 10mM ammonium bicarbonate in water (pH=9.5), Eluent B: acetonitrile.

Method 2: Apparatus: Agilent 1260 Infinity, 1260 G1312B Bin. Pump, 1260 G1367E WPS, 1260 TCC G1316A Column Comp. 1260 G1315C DAD (210-320 nm, 210 and 220nm), PDA (210-320 nm), G6130B MSD ESI pos/neg (mass range 100-1000), Column: Waters XSelect CSH C18 (30×2.1mm 3.5μ), Flow: 1 ml/min; Column Temp: 25°C, Eluent A: 10 mM ammonium bicarbonate in water (pH 9), Eluent B: Acetonitrile, Gradient: t=0 min 5% B, t=1.6 min 98% B, t=3 min 98% B, Postrun: 1.3 min.

Method 3: Apparatus: Agilent Technologies G6130B Quadrupole; HPLC instrument type: Agilent Technologies 1290 preparative LC; Column: Waters XSelect CSH (C18, 100x30mm, 10μ); Flow: 55 mL/min; Column temp: RT; Eluent A: 0.1% formid acid in water; Eluent B: 100% acetonitrile lin. gradient: t=0 min 20% B, t=2 min 20% B, t=8.5 min 60% B, t=10 min 100% B, t=13 min 100% B; Detection: DAD (220-320 nm); Detection: MSD (ESI pos/neg) mass range: 100 - 1000; fraction collection based on MS and DAD.

Method 4: Apparatus: ACQ-SQD2; HPLC instrument type: Waters Modular Preparative HPLC System; column: Waters XSelect (C18, 100x30mm, 10μm); flow: 55 ml/min prep pump; column temp: RT; eluent A: 10mM ammonium bicarbonate in water pH=9.5, eluent B: 100% acetonitrile; detection: DAD (220-320 nm); detection: MSD (ESI pos/neg) mass range: 100 - 800; fraction collection based on MS and DAD.

Method 5: Apparatus: Waters IClass; Bin. Pump: UPIBSM, SM: UPISMFTN with SO; UPCMA, PDA: UPPDATC, 210-320 nm, SQD: MS: QDA ESI, pos/neg 100-800; column: Waters XSelect CSH C18, 50×2.1mm, 2.5μm, Temp: 25°C, Flow: 0.6 mL/min, Gradient: t0 = 5% B, t1.3min = 98% B, t1.7min = 98% B, Posttime: 0.3 min, Eluent A: 10mM ammonium bicarbonate in water (pH=9.5), Eluent B: acetonitrile. MS parameters: Source: ESI; Capillary: 2500 V; Cone: 20 V; Extractor: 3.0 V; RF: 2.5 V; Source Temp.: 150°C; Desolvation Temp.: 600°C; Cone Gas Flow: 80 L/Hr; Desolvation Gas Flow: 1000 L/Hr; Full MS scan: MS range 100-800 (positive and negative mode); scan: 0.4 sec.

Method 6: Apparatus: Waters I-Class UPLC, Binary Solvent Manager (BSM), Sample Manager-FTN (SM-FTN) and Sample Organizer (SO), Column Manager (CM-A), PDA 210-320nm, QDa ESI 100-800 (pos) 100-800 (neg), Column: XSelect CSH C18 XP (50×2.1mm 2.5μm) Flow: 0.6 ml/min; Column temp: 25°C, Eluent A: 10mM ammoniumbicarbonate in water (pH 9.5), Eluent B: acetonitrile, Gradient: t=0 min 5% B, t=2 min 98% B, t=2.7 min 98% B, Postrun: 0.3 min.

Method 7: Apparatus: Waters Acquity UPC2: Waters ACQ-ccBSM Binary Pump; Waters ACQ-CCM Convergence Manager; Waters ACQ-SM Sample Manager - Fixed Loop; Waters ACQ-CM Column Manager - 30S; Waters ACQ-PDA Photodiode Array Detector; Waters ACQ-ISM Make Up Pump, Waters Acquity QDa MS Detector; Column: Lux i-Cellulose-5 (100x4.6mm 5μm); Column temp: 35 °C; Flow: 2.5 ml/min; ABPR: 170 bar; Eluent A: CO2, Eluent B: Methanol + 20mM ammonia; Gradient: t=0 min 5% B, t=5 min 50% B, t=6 min 50% B; Detection: 210-320nm.

Method 8: Apparatus: Waters Prep 100 SFC UV/MS directed system; Waters 2998 Photodiode Array (PDA) Detector; Waters Acquity QDa MS detector; Waters 2767. Sample Manager; Column: Phenomenex Lux i-Cellulos-5 (250x21.2 mm, 5 μm); Column temp: 35°C; Flow: 70 ml/min; ABPR: 120 bar; Eluent A: CO2, Eluent B: 20 mM Ammonia

in Methanol; Detection: PDA (210-400 nm); Gradient: t=0 min 25% B; t=5 min 45% B. Sample concentration 20 mg/ml. Injection volume: 500 μl. Timed fraction collection.

Method 9: Apparatus: Waters Prep 100 SFC UV/MS directed system; Waters 2998 Photodiode Array (PDA) Detector; Waters Acquity QDa MS detector; Waters 2767 Sample Manager; Column: Diacel Chiralpak IC for SFC (250x20 mm, 5 μm); Column temp: 35°C; Flow: 70 ml/min; ABPR: 120 bar; Eluent A: CO2, Eluent B: 20 mM Ammonia in Methanol; Linear gradient: t=0 min 20% B, t=4.5 min 50% B; Detection: PDA (210-400 nm); Fraction collection: PDA TIC.

Method 10: Apparatus: Waters Acquity UPC2: Waters ACQ-ccBSM Binary Pump; Waters ACQ-CCM Convergence Manager; Waters ACQ-SM Sample Manager - Fixed Loop; Waters ACQ-CM Column Manager - 30S; Waters ACQ-PDA Photodiode Array Detector; Waters ACQ-ISM Make Up Pump, Waters Acquity QDa MS Detector; Column: Chiralpak IC (100x4.6mm 5μm); Column temp: 35°C; Flow: 2.5 ml/min; ABPR: 170 bar; Eluent A: CO2, Eluent B: Methanol + 20mM ammonia; Linear gradient: t=0 min 5% B, t=5 min 50% B, t=6 min 50% B; Detection: 210-320nm.

Method 11: Apparatus: Waters Acquity UPC2: Waters ACQ-ccBSM Binary Pump; Waters ACQ-CCM Convergence Manager; Waters ACQ-SM Sample Manager - Fixed Loop; Waters ACQ-CM Column Manager - 30S; Waters ACQ-PDA Photodiode Array Detector; Waters ACQ-ISM Make Up Pump, Waters Acquity QDa MS Detector; Column: phenomenex iAmylose-3 (100x4.6mm 5μm); Column temp: (35°C); Flow: (2.5 ml/min); ABPR: (170 bar); Eluent A: CO2, Eluent B: Methanol + 20mM ammonia; Linear gradient: t=0 min 5% B, t=5 min 50% B, t=6 min 50% B; Detection: 210-320nm.

Method 12: Apparatus: Waters Prep 100 SFC UV/MS directed system; Waters 2998 Photodiode Array (PDA) Detector; Waters Acquity QDa MS detector; Waters 2767 Sample Manager; Column: phenomenex iAmylose-3 (250x20 mm, 5 μm); Column temp: 35°C; Flow: 70 ml/min; ABPR: 170 bar; Eluent A: CO2, Eluent B: 20 mM Ammonia in Methanol; Linear gradient: t=0 min 20% B, t=4.5 min 50% B; Detection: PDA (210-400 nm); Fraction collection: PDA TIC.

**Examples 1, 2 and 3**

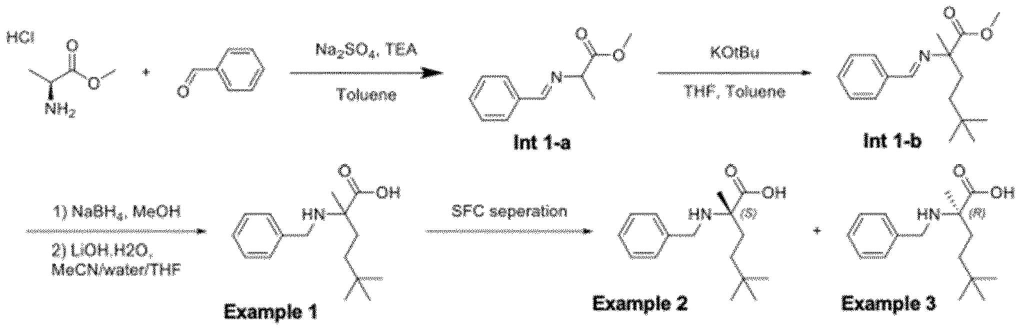


**Synthesis of methyl (E)-2-(benzylideneamino)propanoate (Int 1-a)**

Methyl *L*-alaninate hydrochloride (20 g, 143 mmol) in Toluene (dry) (100 ml) was stirred for 20 minutes. Benzaldehyde (14.71 ml, 145 mmol, 1.01 equiv), triethylamine (30.0 ml, 215 mmol, 1.5 equiv.) and sodium sulfate (12.21 g, 86 mmol, 0.6 equiv.) were added and the mixture was stirred at RT overnight. The reaction mixture was filtered. The residue was washed with toluene (20 mL) and the organic layer was concentrated in vacuo. Crude methyl (E)-2-(benzylideneamino)propanoate (23.5g, 123 mmol, 86%, 85% purity) was obtained as a white solid. LCMS (Method 2, 1.815 min; M+H = 192.1; calcd. 192.2) which was used without further purification.

**Synthesis of methyl methyl (E)-2-(benzylideneamino)-2,5,5-trimethylhexanoate (Int 1-b)**

Methyl (*E*)-2-(benzylideneamino)propanoate (10 g, 52.3 mmol) in toluene (dry) (50 mL) was heated to 40°C. 1-bromo-3,3-dimethylbutane (11.22 g, 68.0 mmol, 1.3 equiv.) was added followed by the addition of KOtBu (5.87 g, 52.3 mmol, 1.0 equiv.) in Tetrahydrofuran (dry) (50 mL). The mixture was stirred overnight. The mixture was cooled to room temperature and quenched with water (20 mL). The mixture was extracted with EtOAc, washed with brine and dried over sodium sulfate. Concentration *in vacuo* afforded methyl (*E*)-2-(benzylideneamino)-2,5,5-trimethylhexanoate (9.86 g, 35.8 mmol, 68% yield). LCMS (method 2, 2.345 min; M+H = 276.2; calcd. 276.2). $^1$H-NMR (400 MHz, CDCl3) $\delta$ 8.25 (s, 1H), 7.85 - 7.70 (m, 2H), 7.48 - 7.36 (m, 3H), 3.74 (s, 3H), 1.90 (tt, J = 8.6, 6.6 Hz, 2H), 1.49 (s, 3H), 1.25 - 1.14 (m, 2H), 0.89 (s, 9H).

**Synthesis of rac-2-benzylamino-2,5,5-trimethylhexanoic acid (Example 1), (*S*)-2-benzylamino-2,5,5-trimethylhexanoic acid (Example 2) and (*R*)-2-benzylamino-2,5,5-trimethylhexanoic acid (Example 3).**

Sodium borohydride (0.824 g, 21.79 mmol, 2.0 equiv.) was added to a solution of methyl (*E*)-2-(benzylideneamino)-2,5,5-trimethylhexanoate (3.0 g, 10.89 mmol) in methanol (40 ml) at 0 °C and mixture stirred at rt overnight. The mixture was partially concentrated and partitioned between DCM and water. The aqueous layer was extracted once with DCM and combined organic layers were washed with brine, dried over sodium sulfate concentrated *in vacuo,* affording 1.95 g of a yellow oil. The mixture was purified by flash column chromatography (reveleris 40 g silica cartrigde, gradient 0% to 100% EtOAc in heptane), affording methyl 2-(benzylamino)-2,5,5-trimethylhexanoate (781 mg, 2.82 mmol, 25% yield) as yellow oil. LCMS (Method 2, 2.304 min; M+H = 278.2; calcd. 278.2). The oil was dissolved in MeCN/water/THF (1/1/1, v/v/v, 9 mL), lithium hydroxide monohydrate (591 mg, 14.08 mmol, 5.0 equiv.) added and heated at 80°C overnight. Only 20% of saponified product was seen, however, light brown sticky solids had crashed out of solution, which were collected by filtration and dried in the vacuum stove overnight, affording impure target which was submitted for basic prep purification (Method 2). After purification, 20 mg was kept as the racemic and the remaining material (60 mg) was submitted for SFC purification (method 8). Purified fractions were combined, evaporated, redissolved in MeCN after weighing (0.05M) and a 0.1M MsOH in MeCN (1 equiv.) solution was added. Little water was added and freeze-dried, affording rac-2-benzylamino-2,5,5-trimethylhexanoic acid methanesulfonic acid (**Example 1,** 29 mg, 0.081 mmol, 2% yield, 100% purity). LCMS (Method 6, 1.020 min; M+H-MsOH = 264.2; calcd. 264.2). $^1$H-NMR (400 MHz, DMSO) δ 14.94 - 13.59 (m, 1H), 9.14 (s, 2H), 7.55 - 7.38 (m, 5H), 4.25 - 4.00 (m, 2H), 2.30 (s, 3H, MsOH), 1.92 (td, J = 13.3, 4.6 Hz, 1H), 1.81 (td, J = 13.3, 4.6 Hz, 1H), 1.56 (s, 3H), 1.28 (td, J = 13.0, 4.6 Hz, 1H), 1.17 (td, J = 12.9, 4.6 Hz, 1H), 0.88 (s, 9H).

(S)-2-benzylamino-2,5,5-trimethylhexanoic acid with methanesulfonic acid compound (**Example 2,** 21.2 mg, 0.059 mmol, 2% yield, 87.35% purity). LCMS (Method 6, 1.007 min; M+H-MsOH = 264.2; calcd. 264.2). LCMS (Method 7, 3.280; M+H-MsOH = 264.1; calcd. 264.2). $^1$H-NMR (400 MHz, DMSO) δ 9.59 - 8.45 (m, 2H), 7.52 - 7.39 (m, 5H), 4.13 (d, J = 12.7 Hz, 1H), 4.00 (d, J = 12.8 Hz, 1H), 2.29 (s, 3H, MsOH), 1.88 (td, J = 12.8, 4.4 Hz, 1H), 1.77 (td, J = 13.2, 4.4 Hz, 1H), 1.51 (s, 3H), 1.27 (td, J = 12.0, 3.6 Hz, 1H), 1.17 (td, J = 12.9, 4.8 Hz, 1H), 0.87 (s, 9H).

(R)-2-benzylamino-2,5,5-trimethylhexanoic acid compound with methanesulfonic acid (**Example 3,** 26.4 mg, 0.073 mmol, 2 % yield, 95.65% purity). LCMS (Method 6, 1.001 min; M+H-MsOH = 264.2; calcd. 264.2). LCMS (Method 7, 3.773; M+H-MsOH = 264.1; calcd. 264.2). $^1$H-NMR (400 MHz, DMSO) δ 9.14 (s, 2H), 7.54 - 7.41 (m, 5H), 4.25 - 4.10 (m, 1H), 4.10 - 3.99 (m, 1H), 2.30 (s, 3H), 1.92 (td, J = 13.4, 4.7 Hz, 1H), 1.81 (td, J = 13.3, 4.7 Hz, 1H), 1.56 (s, 3H), 1.28 (td, J = 13.0, 4.8 Hz, 1H), 1.17 (td, J = 12.9, 4.7 Hz, 1H), 0.88 (s, 9H).

**Examples 4, 5 and 6**

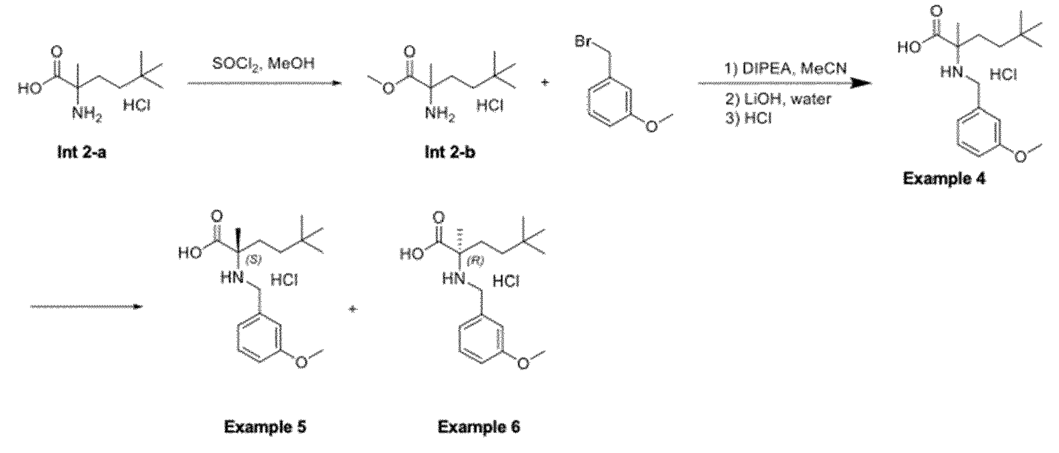


Int 2-a, Int 2-b, Example 4, Example 5, Example 6

**Synthesis of methyl 2-amino-2,5,5-trimethylhexanoate hydrochloride (Int 2-b)**

To an ice-cooled solution of commercially available 2-amino-2,5,5-trimethylhexanoic acid hydrochloride (**Int 2-a,** 250 mg, 1.192 mmol) in methanol (1 ml) under argon atmosphere was added dropwise thionyl chloride (0.261 ml, 3.58 mmol, 3.0 equiv.). The mixture was stirred at 0 °C for 15 min and then at rt for 3 hours. A white thick suspension had formed. The suspension was diluted with little MeOH and stirred overnight. Thionyl chloride (0.261 ml, 3.58 mmol)

was added additionally and after addition the mixture was heated under reflux for 4.5 hours. The mixture was allowed to cool to rt and solvent evaporated, MeCN added and solvent evaporated, affording methyl 2-amino-2,5,5-trimethylhexanoate hydrochloride (233 mg, 1.041 mmol, 87% yield) as an off-white turbid oil. LCMS (Method 2, 1.782min; M+H-HCl = 188.1; calcd. 188.2). $^1$H-NMR (400 MHz, DMSO) δ 8.53 (s, 2H), 3.77 (s, 3H), 1.83 - 1.70 (m, 2H), 1.47 (s, 3H), 1.33 - 1.20 (m, 1H), 1.06 - 0.93 (m, 1H), 0.85 (s, 9H).

The following intermediate, **Int 3-b** was prepared in an analogous manner to intermediate **Int 2-b,** starting from the corresponding commercially available amino acid **Int 3-a.**

| Intermediate | Structure | Yield | LCMS | NMR |
|---|---|---|---|---|
| **Int 3-b** | 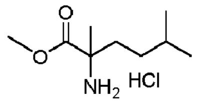 | 281 mg (1.340 mmol, quant. yield) | Method 2 , 1.835 min; M+H-MsOH$^-$= 174.1; calcd. 174.1 | $^1$H-NMR (400 MHz, $CDCl_3$) δ 8.90 (s, 2H), 3.82 (s, 3H), 2.08 - 1.89 (m, 2H), 1.74 (s, 3H), 1.56 - 1.51 (m, 2H), 1.17 - 1.05 (m, 1H), 0.89 (dd, *J* = 6.3, 4.6 Hz, 6H). |

**Synthesis of 2-((3-methoxybenzyl)amino)-2,5,5-trimethylhexanoic acid hydrochloride. Example 4**

DIPEA (0.136 ml, 0.781 mmol) and 1-(bromomethyl)-3-methoxybenzene (0.036 ml, 0.260 mmol) were added to a suspension of methyl 2-amino-2,5,5-trimethylhexanoate hydrochloride (58.25 mg, 0.260 mmol) in Acetonitrile (1 ml). Mixture heated at 80°C for 2h. The mixture was allowed to cool to rt, diluted with water (1 mL) and lithium hydroxide monohydrate (54.6 mg, 1.302 mmol, 5 equiv.) were added. The mixture was heated at 80°C overnight. The crude was filtered and purified by basic preparative prep (Method 4). Purified fractions were concentrated in the Genevac, and reformatted using 1M HCl, affording 2-((3-methoxybenzyl)amino)-2,5,5-trimethylhexanoic acid hydrochloride (**Example 4,** 25.8 mg, 0.078 mmol, 30 % yield, 100% purity) as a white solid. LCMS (Method 1, 1.163 min; M+H-HCl = 294.3; calcd. 294.2). $^1$H-NMR (400 MHz, DMSO) δ 9.94 - 8.64 (br, 1H)7.34 (t, J = 7.9 Hz, 1H), 7.17 (s, 1H), 7.07 (d, J = 7.8 Hz, 1H), 6.97 (dd, J = 8.2, 2.6 Hz, 1H), 4.12 (d, J = 12.7 Hz, 1H), 3.94 (d, J = 12.7 Hz, 1H), 1.98 - 1.75 (m, 2H), 1.52 (s, 3H), 1.31 - 1.12 (m, 2H), 0.86 (s, 9H).

The following **Examples 7** to **9** were prepared in an analogous manner to Example 4, starting from the corresponding ester.

| Example | Structure | Yield | LCMS | NMR |
|---|---|---|---|---|
| **7** | 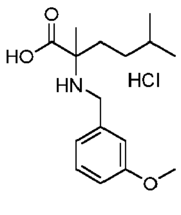 | 14.6 mg (12% yield, 100% purity) | Method 6, 0.947 min; M+H-HCl= 280.2; calcd. 280.2 | $^1$H-NMR (400 MHz, DMSO) δ 14.59 -13.80 (br, 1H), 9.57 (s, 1H), 9.19 (s, 1H), 7.36 (t, *J* = 7.9 Hz, 1H), 7.18 (s, 1H), 7.08 (d, *J* = 7.2 Hz, 1H), 6.99 (dd, *J* = 8.3, 2.6 Hz, 1H), 4.23 - 4.12 (m, 1H), 4.05 - 3.95 (m, 1H), 2.02 - 1.78 (m, 2H), 1.56 (s, 3H), 1.48 (dt, *J* = 13.4, 6.7 Hz, 1H), 1.35 - 1.13 (m, 2H), 0.87 (dd, *J* = 6.6, 2.5 Hz, 6H). |
| **8** | 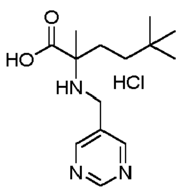 | 45.1 mg (44% yield, 98.63% purity | Method 6, 0.692 min; M+H-HCl= 266.2; calcd. 266.2 | $^1$H-NMR (400 MHz, DMSO) δ 14.67 - 13.92 (br, 1H), 10.14 (s, 1H), 9.46 (s, 1H), 9.22 (s, 1H), 8.97 (s, 2H), 4.15 (d, *J* = 7.5 Hz, 2H), 1.92 (pd, *J* = 13.8, 4.8 Hz, 2H), 1.61 (s, 3H), 1.28 (tt, *J* = 12.0, 6.1 Hz, 1H), 1.16 (td, *J* = 12.7, 4.8 Hz, 1H), 0.87 (s, 9H). |
| **9** | 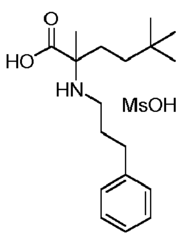 | 14.7 mg (16% yield, 98.09% purity) | Method 1, 1.141 min; M+H-MsOH= 292.5; calcd. 292.2 | $^1$H NMR (400 MHz, DMSO) δ 14.57 - 13.84 (m, 1H), 8.80 (s, 2H), 7.35 - 7.27 (m, 2H), 7.27 - 7.12 (m, 3H), 2.99 - 2.80 (m, 2H), 2.67 (t, *J* = 7.6 Hz, 2H), 2.31 (s, 3H, MsOH), 1.94 (p, *J* = 7.1 Hz, 2H), 1.84 - 1.68 (m, 2H), 1.44 (s, 3H), 1.29 - 1.16 (m, 1H), 1.18 - 1.01 (m, 1H), 0.84 (s, 9H). |

**Synthesis of (*S*)-2-((3-methoxybenzyl)amino)-2,5,5-trimethylhexanoic acid hydrochloride (Example 5) and (*R*)-2-((3-methoxybenzyl)amino)-2,5,5-trimethylhexanoic acid hydrochloride (Example 6). Stereochemistry assigned from X-Ray crystallography studies.**

2-((3-methoxybenzyl)amino)-2,5,5-trimethylhexanoic acid (**Example 4,** 158 mg, 0.539 mmol) was purified by SFC (method 9). Purified fractions were combined and concentrated. The fractions were reformatted using 1M aq. HCl, affording (*S*)-2-((3-methoxybenzyl)amino)-2,5,5-trimethylhexanoic acid hydrochloride (64 mg, 0.194 mmol, 27% yield, 99.69% purity, >99% ee). LCMS (Method 6, 0.993 min; M+H-HCl = 294.3; calcd. 294.2). LCMS (Method 10, 3.138 min; M+H-HCl = 294.1; calcd. 294.2). $^1$H-NMR (400 MHz, DMSO) $\delta$ 14.67 - 13.61 (br, 1H), 9.98 - 8.91 (m, 2H), 7.35 (t, J = 7.9 Hz, 1H), 7.22 - 7.16 (m, 1H), 7.09 (d, J = 7.6 Hz, 1H), 6.99 (dd, J = 8.2, 2.6 Hz, 1H), 4.17 (d, J = 12.7 Hz, 1H), 3.97 (d, J = 12.7 Hz, 1H), 3.79 (s, 3H), 1.96 (td, J = 13.2, 4.9 Hz, 1H), 1.86 (td, J = 13.1, 5.0 Hz, 1H), 1.56 (s, 3H), 1.32 - 1.12 (m, 2H), 0.87 (s, 9H).

and (*R*)-2-((3-methoxybenzyl)amino)-2,5,5-trimethylhexanoic acid hydrochloride (57.8 mg, 0.175 mmol, 24% yield, 99.76% purity, >99% ee). LCMS (Method 6, 0.992 min; M+H-HCl = 294.3; calcd. 294.2). LCMS (Method 10, 3.658 min; M+H-HCl = 294.1; calcd. 294.2). $^1$H-NMR (400 MHz, DMSO) $\delta$ 14.86 - 13.43 (br, 1H), 9.38 (s, 2H), 7.35 (t, J = 7.9 Hz, 1H), 7.19 (s, 1H), 7.09 (d, J = 8.1 Hz, 1H), 6.99 (dd, J = 8.1, 2.6 Hz, 1H), 4.16 (d, J = 12.7 Hz, 1H), 3.97 (d, J = 12.8 Hz, 1H), 3.79 (s, 3H), 1.95 (td, J = 13.9, 5.6 Hz, 1H), 1.85 (td, J = 13.2, 4.9 Hz, 1H), 1.56 (s, 3H), 1.32 - 1.13 (m, 2H), 0.87 (s, 9H).

The following **Examples 10** and **11,** were prepared in an analogous manner to **Examples 5** and **6,** starting from the corresponding racemic mixture.

| Example | Structure | Yield | LCMS | NMR |
|---|---|---|---|---|
| **10**[a] | 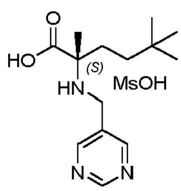 | 82.2 mg, 20% yield, 100% purity, >95% ee | Method 1, 0.712 min; M+H-MsOH= 266.4; calcd. 266.2 Method 11, 2.802 min; M+H-MsOH= 266.1; calcd. 266.2 | $^1$H-NMR (400 MHz, DMSO) $\delta$ 14.84 - 13.95 (br, 1H), 9.47 - 9.10 (br, 2H), 9.24 (s, 1H), 9.13 (s, 0H), 8.90 (s, 2H), 4.33 - 4.15 (m, 2H), 2.30 (s, 3H, MsOH), 1.98 - 1.77 (m, 2H), 1.58 (s, 3H), 1.30 (td, *J* = 12.8, 5.6 Hz, 1H), 1.14 (td, *J* = 12.5, 5.2 Hz, 1H), 0.89 (s, 9H). |
| **11**[a] | 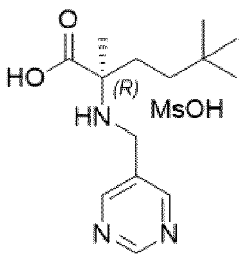 | 86.9 mg, 21% yield, 100% purity, >95% ee | Method 1, 0.713 min; M+H-MsOH= 266.5; calcd. 266.2 Method 11, 2.268 min; M+H-MsOH= 266.1; calcd. 266.2 | $^1$H NMR (400 MHz, DMSO) $\delta$ 9.23 (s, 1H), 9.81 - 9.17 (m, 2H), 8.90 (s, 2H), 4.32 - 4.17 (m, 2H), 2.31 (s, 3H, MsOH), 1.94 - 1.78 (m, 2H), 1.58 (s, 3H), 1.30 (td, *J* = 12.6, 5.6 Hz, 1H), 1.14 (td, *J* = 12.4, 5.2 Hz, 1H), 0.89 (s, 9H). |
| [a]; purified by Method 12; | | | | |

**The following Examples 12 and 13** can be prepared in an analogous manner to the other examples, starting from the corresponding racemic mixture.

| Example | Structure |
| --- | --- |
| 12 | 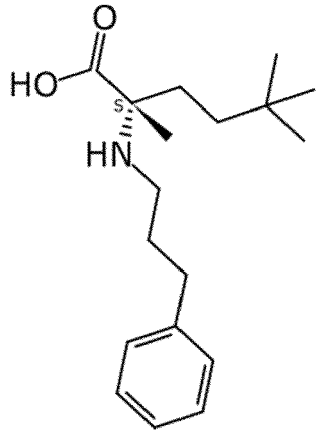 |
| 13 | 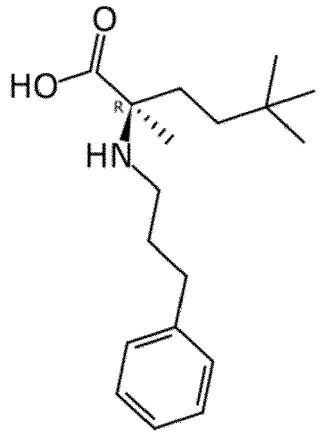 |

If the stereochemistry is not specified in the structures of the examples above, the compound was prepared as a racemic mixture.

## BIOLOGICAL DATA

### Neurotensin scintillation proximity assay

The exemplified compounds of the invention were tested in a Neurotensin (NTS) scintillation proximity assay (SPA). The $IC_{50}$ data is shown in the table below. NTS, which is a 13 amino acid neuropeptide, is a sortilin ligand. The $IC_{50}$ is a measure of the amount of the compound required to inhibit the binding of NTS to sortilin by 50%. The skilled person will recognise that the lower the $IC_{50}$ value, the less of the compound needed to achieve the desired effect, and as a result, the chances of undesirable off-target effects are reduced.

Compound affinity was determined by measuring the displacement of [$^3$H]-neurotensin binding to *h*-Sortilin in SPA format. Total volume of 40 µl in 50 mM HEPES pH 7.4 assay buffer containing 100 mM NaCl, 2.0 mM CaCl2, 0.1 % BSA and 0.1 % Tween-20. Compound pre-incubation for 30 minutes at room temperature with 150 nM of 6his-Sortilin before 5 nM [3H]-Neurotensin and Ni chelate imaging beads (Perkin Elmer) were added, after 6 hours the plate was read on a ViewLux with 360 s exposure time. Dose-response evaluation of compounds was performed with 8 concentrations of drugs (covering 3 decades). $IC_{50}$ values were calculated by nonlinear regression using the sigmoid concentration-response (variable slope) using CDD Vault software. All values reported are average of at least 2 determinations.

### Human Sortilin GCI binding assay

The exemplified compounds of the invention were tested in a Grating-Coupled Interferometry (GCI) Sortilin binding assay. The GCI data is shown in Table 1 below. The Equilibrium dissociation constant ($K_D$) is a measure of the propensity of a complex (i.e: a ligand and receptor) to dissociate and is used to describe the affinity of an interaction

between the constituents of the complex (i.e: a ligand and receptor). The skilled person will recognise that the lower the $K_D$ value, the less of the compound needed to achieve the desired effect, and as a result, the chances of undesirable off-target effects are reduced.

Compound affinity was determined by measuring the association and dissociation of compounds to Human (6-His)Sortilin (RnD Systems) using a Creoptix Wave instrument (Creoptix - Malvern Panalytical). Both Human (6-His)Sortilin immobilisation and compound evaluation were performed in HBS-N running buffer: 10 mM HEPES pH 7.4, 150 mM NaCl, 1 % DMSO - Cytiva which was filtered and degassed for 15 minutes before use.

A 4PCH WAVEchip (Creoptix - Malvern Panalytical) was conditioned prior to immobilisiation using 0.2x HBS-N running buffer with 0.1 M Borate pH 9.0, 1 M NaCl (Xantec Bioanalytics) injected across all flow cells. A buffer exchange was performed to 1x HBS-N running buffer and Human (6-His)Sortilin was immobililised in pH 5.0 Acetate solution (Cytiva) via amine coupling following EDC/NHS (Cytiva) surface activation and then passivated via injection of 50 mM Tris pH 7.4.

Compounds were tested in 'WAVE-RAPID' assays at a single concentration (usually: 1 μM, 10 μM or 100 μM) in time-response format (short pulses of increasing duration) using either 'Weak-Binders' (Settings: 400 μl/min flow rate, 40 Hz acquisiton rate, 5 seconds association time and 20 seconds dissociation time) or 'Intermediate Binders' (Settings: 100 μl/min flow rate, 10 Hz acquisiton rate, 25 seconds association time and 300 seconds dissociation time) settings.

Which was deemed most appropriate was based upon the affinity and kinetics of the compound tested.

DMSO calibration was performed as per manufacturers requirements by injection of running buffer containing DMSO 0.5% above that of the running buffer DMSO (1.5 % in that instance) every 20 cycles. All data was double referenced (blank and reference-channel subtracted) with $K_D$ values calculated by fitting to a '1:1 kinetic' binding model using 'WAVEcontrol' software (Creoptix - Malvern Panalytical).

**Progranulin cell surface engagement assay**

The exemplified compounds of the invention were tested in a progranulin (PGRN) cell surface engagement assay. The $IC_{50}$ data is shown in Table 1 below. PGRN is a sortilin ligand. The $IC_{50}$ is a measure of the amount of the compound required to inhibit the binding of PGRN to sortilin by 50%. The skilled person will recognise that the lower the $IC_{50}$ value, the less of the compound needed to achieve the desired effect, and as a result, the chances of undesirable off-target effects are reduced.

Compound affinity was determined by measuring the ability of compounds to displace the binding of Progranulin-Alexa Fluor 647 (PGRN-AF647) binding to the Sortilin receptor at the cell surface. HEK293 engineered to express sortilin were pre-incubated with compound for 30 minutes on ice in 20 μl assay buffer containing 1% BSA in PBS. Dose-response evaluation of compounds was performed with 10 concentrations of drugs (10 point half log curve). PGRN-AF647 was added at 100 nM concentration in 20 μl assay buffer for 2 hours on ice. Data was acquired using iQue flow cytometer (IntelliCyt). $IC_{50}$ values were calculated by nonlinear regression using the sigmoid concentration-response (variable slope) using CDD Vault software. All values reported are average of at least 2 determinations.

The data in the table below shows that the compounds of the invention are h-sortilin binders and inhibitors.

**Table 1**

| Example | h-Sortilin GCI $K_D$ (μM) | $IC_{50}$ [3H]Neurotensin SPA (μM) | $IC_{50}$ PGRN HEK293 (μM) |
|---|---|---|---|
| 1 | 0.621 | 2.50 | - |
| 2 | 0.393 | 2.97 | - |
| 3 | 21.4 | - | - |
| 4 | - | 4.02 | - |
| 5 | - | 1.33 | - |
| 7 | 1.27 | - | - |
| 8 | 4.22 | 0.840 | - |
| 9 | - | 9.74 | - |
| 10 | 0.804 | 0.383 | 0.68 |
| 11 | 73.5 | - | - |

**X ray Crystallography**

sSortilin, the luminal domain of sortilin, was obtained as previously described (Andersen et al., Acta Cryst. D, 2017). For crystallization 2 μl 5 mg/ml sSortilin in 50 mM Tris-HCl pH 7.3, 150 mM NaCl was mixed with 0.2 μl of the Example 5 dissolved in DMSO at concentrations of 8.49 mM. This drop was mixed with 2 μl of reservoir solution composed of 100 mM Hepes pH 7.3, 400 mM malonate pH 7.3, 7% v/v glycerol, 24% w/v PEG3350. The sitting drops were left to equilibrate by vapor diffusion with 500 μl reservoir solution. Crystals were mounted in litho-loops without further cryoprotection and were flash cooled in liquid nitrogen. Diffraction data were collected at beamline ID23-2, ESRF, Grenoble and processed using the XDS package (Kabsch, W., Acta Cryst. D, 2010) (see table 2 below).

Phases for the structure factors were obtained by molecular replacement using the known structure of sortilin (PDB entry: 3F6K) as search model and the program Phaser implemented in the Phenix software package (Afonine et al., Acta Cryst. D, 2012). A refined model was obtained by several cycles of model building in Coot (Emsley P. et al., Acta Cryst. D, 2010) and maximum likelihood refinement using Phenix.

Details of the diffraction data are provided in table 2 below (statistics for the highest-resolution shell are shown in parentheses).

**Table 2**

| Diffraction Data | Example 5 |
|---|---|
| Wavelength | 0.87313 Å |
| Resolution range | 44.01 - 2.6 (2.693 - 2.6) |
| Space group | C 1 2 1 |
| Unit cell | 161.37 78.28 111.6 90 126.89 90 |
| Total reflections | 672311 (70231) |
| Unique reflections | 34381 (3403) |
| Multiplicity | 19.6 (20.6) |
| Completeness (%) | 99.86 (100.00) |
| Mean I/sigma(I) | 19.19 (2.43) |
| Wilson B-factor | 76.35 |
| R-merge | 0.1 (1.55) |
| R-meas | 0.1028 (1.589) |
| R-pim | 0.02342 (0.3478) |
| CC1/2 | 0.999 (0.848) |
| CC* | 1 (0.958) |
| **Refinement and model quality** | |
| Reflections (work) | 34372 (3403) |
| Reflections (Free) | 1719 (170) |
| R-work | 0.1920 (0.3137) |
| R-free | 0.2309 (0.3755) |
| CC(work) | 0.956 (0.873) |
| CC(free) | 0.951 (0.760) |
| No. of non-hydrogen atoms | 5375 |
| macromolecules | 5170 |
| ligands | 174 |
| solvent | 31 |
| Protein residues | 655 |

(continued)

| Refinement and model quality | |
|---|---|
| RMS(bonds (Å) / angles (°)) | 0.003/0.48 |
| Ramachandran favored (%) | 96.47 |
| Ramachandran outliers (%) | 0 |
| Rotamer outliers (%) | 0.87 |
| Clashscore | 3.93 |
| Average B-factor | 101.51 |
| macromolecules | 100.83 |
| ligands | 126.92 |
| solvent | 72.18 |
| Number of TLS groups | 5 |

The X-ray derived images of Example 5 bound to h-Sortilin are shown in Figures 1 to 10. Figures 9 and 10 also depict an estimation of the atomic contribution to binding affinity.

Figures 11A to 11C depict electron density maps showing the electron density observed during x-ray crystallography fitted to the structure of the molecules to provide a representation of the crystal structure.

Details of the electron density maps are as follows:

Figure 11A - 2fo-fc map. Contouring level = 1.5σ.

Figure 11B - omit map. Contouring level = 4.0o.

Figure 11C - polder map. Contouring level = 6.0o.

**REFERENCES**

Andersen, J et al., Identification of the first small-molecule ligand of the neuronal receptor sortilin and structure determination of the receptor-ligand complex. Acta Crystallogr D Biol Crystallogr (2014), 70(Pt 2), pp.451-460;

Baker, M.et al., Mutations in progranulin cause tau-negative frontotemporal dementia linked to chromosome 17. Nature (2006), 442(7105), pp. 916-919;

Brouwers, N.et al., Genetic variability in progranulin contributes to risk for clinically diagnosed Alzheimer disease. Neurology, (2008), 71(9), pp. 656-664;

Buttenshon, H.N. et al., Increased serum levels of sortilin are associated with depression and correlated with BDNF and VEGF, Nature Translational Psychiatry (2015), 5(e677), pp. 1-7;

Carecchio, M.,et al., Cerebrospinal fluid biomarkers in Progranulin mutations carriers. J Alzheimers Dis (2011), 27(4), pp. 781-790;

Carrasquillo, M. et al.,. Genome-wide screen identifies rs646776 near sortilin as a regulator of progranulin levels in human plasma. Am J Hum Genet (2010), 87(6), pp. 890-897;

Chen, Z. Y.et al., Sortilin controls intracellular sorting of brain-derived neurotrophic factor to the regulated secretory pathway. J Neurosci (2005), 25(26), pp. 6156-6166;

Cruts, M. et al., Loss of progranulin function in frontotemporal lobar degeneration. Trends Genet (2008), 24(4), pp. 186-194;

De Muynck, L. et al., The neurotrophic properties of progranulin depend on the granulin E domain but do not require sortilin binding. Neurobiol Aging (2013), 34(11), pp. 2541-2547;

Egashira, Y. et al.,The growth factor progranulin attenuates neuronal injury induced by cerebral ischemia-reperfusion through the suppression of neutrophil recruitment. J Neuroinflammation (2013), 10, pp. 105;

Galimberti, D. et al.,. GRN variability contributes to sporadic frontotemporal lobar degeneration. J Alzheimers Dis (2010), 19(1), pp. 171-177;

Galimberti, D.et al.,. Progranulin as a therapeutic target for dementia. Expert Opin Ther Targets (2018), 22(7), pp.

579-585. doi:10.1080/14728222.2018.1487951
Gao, A. et al., Implications of Sortilin in Lipid Metabolism and Lipid Disorder Diseases. DNA and Cell Biology (2017), 36(12), pp.1050-1061;
Gass, J. et al., Progranulin regulates neuronal outgrowth independent of sortilin. Mol Neurodegener (2012), 7, pp. 33;
Gass, J. et al., Progranulin: an emerging target for FTLD therapies. Brain Res (2012), 1462, pp. 118-128;
Gijselinck, I.,et al., Granulin mutations associated with frontotemporal lobar degeneration and related disorders: an update. Hum Mutat (2008), 29(12), pp. 1373-1386;
Goettsch, C., et al., Sortilin and Its Multiple Roles in Cardiovascular and Metabolic Diseases. Atherosclerosis, Thrombosis and Vascular Biology (2017), 38(1), pp. 19-25
Jansen, P., et al., Roles for the pro-neurotrophin receptor sortilin in neuronal development, aging and brain injury. Nature Neuroscience (2007), 10(11), pp.1449-1457;
Hu, F. et al., Sortilin-mediated endocytosis determines levels of the frontotemporal dementia protein, progranulin. Neuron (2010), 68(4), pp. 654-667;
Huang, G. et al., Insulin responsiveness of glucose transporter 4 in 3T3-L1 cells depends on the presence of sortilin. Mol Biol Cell (2013), 24(19), pp.3115-3122;
Kaddai, V. et al. Involvement of TNF-$\alpha$ in abnormal adipocyte and muscle sortilin expression in obese mice and humans. Diabetologia (2009) 52, pp. 932-940;
Kjolby, M.et al., Sort1, encoded by the cardiovascular risk locus 1p13.3, is a regulator of hepatic lipoprotein export. Cell Metab (2010), 12(3), pp. 213-223;
Laird, A. S. et al., Progranulin is neurotrophic in vivo and protects against a mutant TDP-43 induced axonopathy. PLoS One (2010), 5(10), e13368;
Lee, W. et al., Targeted manipulation of the sortilin-progranulin axis rescues progranulin haploinsufficiency. Hum Mol Genet (2014), 23(6), pp. 1467-1478;
Martens, L.et al., Progranulin deficiency promotes neuroinflammation and neuron loss following toxin-induced injury. J Clin Invest (2012), 122(11), pp. 3955-3959;
Mazella, J. et al., The 100-kDa neurotensin receptor is gp95/sortilin, a non-G-protein-coupled receptor. J Biol Chem (1998), 273(41), pp. 26273-26276;
Miyakawa, S. et al, Anti-sortilin1 Antibody Up-Regulates Progranulin via Sortilin1 Down-Regulation. Front Neurosci (2020), 14, pp. 586107;
Møller et al. Sortilin as a Biomarker for Cardiovascular Disease Revisited. Frontiers in Cardiovascular Medicine (2021), 8, 652584;
Mortensen, M.B. et al., Targeting sortilin in immune cells reduces proinflammatory cytokines and atherosclerosis. J Clin Invest (2014), 124(12), pp. 5317-5322;
Nykjaer, A et al., Sortilin is essential for proNGF-induced neuronal cell death. Nature (2014), 427(6977), pp. 843-848;
Nykjaer, A., & Willnow, T. E, Sortilin: a receptor to regulate neuronal viability and function. Trends Neurosci (2012), 35(4), pp. 261-270.
Oh, T.J. et al., Circulating sortilin level as a potential biomarker for coronary atherosclerosis and diabetes mellitus. Cardiovascular Diabetology (2017), 16(92);
Pan, X. et al., Sortilin and retromer mediate retrograde transport of Glut4 in 3T3-L1 adipocytes. Mol Biol Cell (2017), 28(12), pp.1667-1675;
Petersen, C. et al., Molecular identification of a novel candidate sorting receptor purified from human brain by receptor-associated protein affinity chromatography. J Biol Chem (1997), 272(6), pp. 3599-3605;
Pickford, F.et al., Progranulin is a chemoattractant for microglia and stimulates their endocytic activity. Am J Pathol (2011), 178(1), pp. 284-295;
Pottier, C., et al., Potential genetic modifiers of disease risk and age at onset in patients with frontotemporal lobar degeneration and GRN mutations: a genome-wide association study. Lancet Neurol (2018), 17(6), pp. 548-558;
Quistgaard, E. et al., Ligands bind to Sortilin in the tunnel of a ten-bladed beta-propeller domain. Nat Struct Mol Biol (2009), 16(1), pp. 96-98;
Santos, A. M. et al., Sortilin Participates in Light-dependent Photoreceptor Degeneration in Vivo. PLoS ONE (2012), 7(4), pp. e36243-e36243.16. Kuruvilla, R. et al., A neurotrophin signaling cascade coordinates sympathetic neuron development through differential control of TrkA trafficking and retrograde signalling. Cell (2004), 118(2), pp. 243-255;
Shi, J. & Kandror, K. V., Sortilin Is Essential and Sufficient for the Formation of Glut4 Storage Vesicles in 3T3-L1 Adipocytes. Developmental Cell (2005), 9, pp. 99-108;
Schroder, T. et al., The identification of AF38469: an orally bioavailable inhibitor of the VPS10P family sorting receptor Sortilin. Bioorg Med Chem Lett (2014), 24(1), pp. 177-180;
Sheng, J. et al.,Progranulin polymorphism rs5848 is associated with increased risk of Alzheimer's disease. Gene (2014), 542(2), pp. 141-145;Skeldal, S. et al., Mapping of the Interaction Site between Sortilin and the p75 Neurotrophin Receptor Reveals a Regulatory Role for the Sortilin Intracellular Domain in p75 Neurotrophin Receptor

Shedding and Apoptosis. J Biol Chem (2012), 21(287), pp. 43798-43809;
Tauris, J., et al., Proneurotrophin-3 May Induce Sortilin-Dependent Death In Inner Ear Neurons. Eur J Neuroscience (2020), 33(4), pp.622-31;
Tang, W. et al., The growth factor progranulin binds to TNF receptors and is therapeutic against inflammatory arthritis in mice. Science (2011), 332(6028), pp. 478-484;
Tao, J.et al., Neuroprotective effects of progranulin in ischemic mice. Brain Res (2012), 1436, pp. 130-136;
Tenk, H.K., et al., ProBDNF induces neuronal apoptosis via activation of a receptor complex of p75NTR and sortilin. J Neuroscience (2005), 10(11), pp. 1449-1457
Van Kampen, J. M.,et al., Progranulin gene delivery protects dopaminergic neurons in a mouse model of Parkinson's disease. PLoS One (2014), 9(5), e97032;
Willnow, T. E.et al., VPS10P-domain receptors - regulators of neuronal viability and function. Nat Rev Neurosci (2008), 9(12), pp. 899-909;
Willnow, T.E., et al., Sortilins: new players in lipoprotein metabolism. Current Opinion in Lipidology (2011), 22(2), pp. 79-85.
Wuts, P.G.M. and Greene, T.W, Greene's Protective Groups in Organic Synthesis, 4th Edition, John Wiley and Sons, New York (2006);
Xu, S.H. et al., Regional and Cellular Mapping of Sortilin Immunoreactivity in Adult Human Brain, Frotiers in Neuroanatomy (2019), 13(31), pp. 1-27;
Yano, H., et al., Proneurotrophin-3 is a neuronal apoptotic ligand: evidence for retrograde-directed cell killing. J Neurosci (2009), 29(47), pp. 14790-14802;
Yin, F., et al., Exaggerated inflammation, impaired host defense, and neuropathology in progranulin-deficient mice. J Exp Med (2010), 207(1), pp. 117-128;
Zheng, Y., et al., C-terminus of progranulin interacts with the beta-propeller region of sortilin to regulate progranulin trafficking. PLoS One (2011), 6(6), e21023;
Zhou, X. et al., Prosaposin facilitates sortilin-independent lysosomal trafficking of progranulin. J Cell Biol (2015), 210(6), pp. 991-1002;
Meneses et al., TDP-43 Pathology in Alzheimer's Disease, Mol Neurodegeneration (2021), 16, 84;
Prudencio et al., Misregulation of human sortilin splicing leads to the generation of a nonfunctional progranulin receptor, Proc Natl Acad Sci USA (2012), 109(52): 21510-21515;
Beel et al., Progranulin reduces insoluble TDP-43 levels, slows down axonal degeneration and prolongs survival in mutant TDP-43 mice, Mol Neurodegener. (2018), 13: 55.

**Sequences referenced throughout the specification and forming part of the description**

SEQ ID NO: 1 (full length sortilin- isoform 1)

1 MERPWGAADG LSRWPHGLGL LLLLQLLPPS TLSQDRLDAP PPPAAPLPRW
51 SGPIGVSWGL RAAAAGGAFP RGGRWRRSAP GEDEECGRVR DFVAKLANNT
101 HQHVFDDLRG SVSLSWVGDS TGVILVLTTF HVPLVIMTFG QSKLYRSEDY
151 GKNFKDITDL INNTFIRTEF GMAIGPENSG KVVLTAEVSG GSRGGRIFRS
201 SDFAKNFVQT DLPFHPLTQM MYSPQNSDYL LALSTENGLW VSKNFGGKWE
251 EIHKAVCLAK WGSDNTIFFT TYANGSCKAD LGALELWRTS DLGKSFKTIG
301 VKIYSFGLGG RFLFASVMAD KDTTRRIHVS TDQGDTWSMA QLPSVGQEQF
351 YSILAANDDM VFMHVDEPGD TGFGTIFTSD DRGIVYSKSL DRHLYTTTGG
401 ETDFTNVTSL RGVYITSVLS EDNSIQTMIT FDQGGRWTHL RKPENSECDA
451 TAKNKNECSL HIHASYSISQ KLNVPMAPLS EPNAVGIVIA HGSVGDAISV
501 MVPDVYISDD GGYSWTKMLE GPHYYTILDS GGIIVAIEHS SRPINVIKFS
551 TDEGQCWQTY TFTRDPIYFT GLASEPGARS MNISIWGFTE SFLTSQWVSY
601 TIDFKDILER NCEEKDYTIW LAHSTDPEDY EDGCILGYKE QFLRLRKSSM
651 CQNGRDYVVT KQPSICLCSL EDFLCDFGYY RPENDSKCVE QPELKGHDLE
701 FCLYGREEHL TTNGYRKIPG DKCQGGVNPV REVKDLKKKC TSNFLSPEKQ
751 NSKSNSVPII LAIVGLMLVT VVAGVLIVKK YVCGGRFLVH RYSVLQQHAE
801 ANGVDGVDAL DTASHTNKSG YHDDSDEDLL E

SEQ ID NO: 2 (full length sortilin- isoform 2)

1 MERPWGAADG LSRWPHGLGL LLLLQLLPPS TLSQDRLDAP PPPAAPLPRW
51 SGPIGVSWGL RAAAAGGAFP RGGRWRRSAP GEDEECGRVR DFVAKLANNT
101 HQHVFDDLRG SVSLSWVGDS TGVILVLTTF HVPLVIMTFG QSKLYRSEDY
151 GKNFKDITDL INNTFIRTEF GMAIGPENSG KVVLTAEVSG GSRGGRIFRS
201 SDFAKNFVQT DLPFHPLTQM MYSPQNSDYL LALSTENGLW VSKNFGGKWE
251 EIHKAVCLAK WGSDNTIFFT TYANGSCTDL GALELWRTSD LGKSFKTIGV
301 KIYSFGLGGR FLFASVMADK DTTRRIHVST DQGDTWSMAQ LPSVGQEQFY
351 SILAANDDMV FMHVDEPGDT GFGTIFTSDD RGIVYSKSLD RHLYTTTGGE
401 TDFTNVTSLR GVYITSVLSE DNSIQTMITF DQGGRWTHLR KPENSECDAT
451 AKNKNECSLH IHASYSISQK LNVPMAPLSE PNAVGIVIAH GSVGDAISVM
501 VPDVYISDDG GYSWTKMLEG PHYYTILDSG GIIVAIEHSS RPINVIKFST
551 DEGQCWQTYT FTRDPIYFTG LASEPGARSM NISIWGFTES FLTSQWVSYT
601 IDFKDILERN CEEKDYTIWL AHSTDPEDYE DGCILGYKEQ FLRLRKSSVC
651 QNGRDYVVTK QPSICLCSLE DFLCDFGYYR PENDSKCVEQ PELKGHDLEF
701 CLYGREEHLT TNGYRKIPGD KCQGGVNPVR EVKDLKKKCT SNFLSPEKQN

751 SKSNSVPIIL AIVGLMLVTV VAGVLIVKKY VCGGRFLVHR YSVLQQHAEA
801 NGVDGVDALD TASHTNKSGY HDDSDEDLLE

SEQ ID NO: 3 (mature sortilin)

1 MTFGQSKLYR SEDYGKNFKD ITDLINNTFI RTEFGMAIGP ENSGKVVLTA
51 EVSGGSRGGR IFRSSDFAKN FVQTDLPFHP LTQMMYSPQN SDYLLALSTE
101 NGLWVSKNFG GKWEEIHKAV CLAKWGSDNT IFFTTYANGS CTDLGALELW
151 RTSDLGKSFK TIGVKIYSFG LGGRFLFASV MADKDTTRRI HVSTDQGDTW
201 SMAQLPSVGQ EQFYSILAAN DDMVFMHVDE PGDTGFGTIF TSDDRGIVYS
251 KSLDRHLYTT TGGETDFTNV TSLRGVYITS VLSEDNSIQT MITFDQGGRW
301 THLRKPENSE CDATAKNKNE CSLHIHASYS ISQKLNVPMA PLSEPNAVGI
361 VIAHGSVGDA ISVMVPDVYI SDDGGYSWTK MLEGPHYYTI LDSGGIIVAI
401 EHSSRPINVI KFSTDEGQCW QTYTFTRDPI YFTGLASEPG ARSMNISIWG
451 FTESFLTSQW VSYTIDFKDI LERNCEEKDY TIWLAHSTDP EDYEDGCILG
501 YKEQFLRLRK SSVCQNGRDY VVTKQPSICL CSLEDFLCDF GYYRPENDSK
551 CVEQPELKGH DLEFCLYGRE EHLTTNGYRK IPGDKCQGGV NPVREVKDLK
601 KKCTSNFLSP EKQNSKSNSV PIILAIVGLM LVTVVAGVLI VKKYVCGGRF
651 LVHRYSVLQQ HAEANGVDGV DALDTASHTN KSGYHDDSDE DLLE

SEQ ID NO: 4 (Murine Sortilin)

>sp|Q6PHU5|SORT_MOUSE Sortilin OS=Mus musculus OX=10090 GN=Sort1 PE=1 SV=1
MERPRGAADGLLRWPLGLLLLLQLLPPAAVGQDRLDAPPPPAPPLLRWAGPVGVSWGLRA
AAPGGPVPRAGRWRRGAPAEDQDCGRLPDFIAKLTNNTHQHVFDDLSGSVSLSWVGDSTG
VILVLTTFQVPLVIVSFGQSKLYRSEDYGKNFKDITNLINNTFIRTEFGMAIGPENSGKV
ILTAEVSGGSRGGRVFRSSDFAKNFVQTDLPFHPLTQMMYSPQNSDYLLALSTENGLWVS
KNFGEKWEEIHKAVCLAKWGPNNIIFFTTHVNGSCKADLGALELWRTSDLGKTFKTIGVK
IYSFGLGGRFLFASVMADKDTTRRIHVSTDQGDTWSMAQLPSVGQEQFYSILAANEDMVF
MHVDEPGDTGFGTIFTSDDRGIVYSKSLDRHLYTTTGGETDFTNVTSLRGVYITSTLSED
NSIQSMITFDQGGRWEHLRKPENSKCDATAKNKNECSLHIHASYSISQKLNVPMAPLSEP
NAVGIVIAHGSVGDAISVMVPDVYISDDGGYSWAKMLEGPHYYTILDSGGIIVAIEHSNR
PINVIKFSTDEGQCWQSYVFTQEPIYFTGLASEPGARSMNISIWGFTESFITRQWVSYTV
DFKDILERNCEEDDYTTWLAHSTDPGDYKDGCILGYKEQFLRLRKSSVCQNGRDYVVAKQ
PSVCPCSLEDFLCDFGYFRPENASECVEQPELKGHELEFCLYGKEEHLTTNGYRKIPGDK
CQGGMNPAREVKDLKKKCTSNFLNPTKQNSKSNSVPIILAIVGLMLVTVVAGVLIVKKYV
CGGRFLVHRYSVLQQHAEADGVEALDSTSHAKSGYHDDSDEDLLE

**Claims**

**1.** A compound of formula (I)

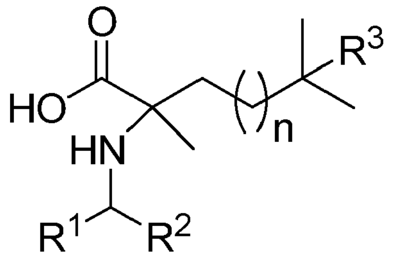

(I)

or a pharmaceutically acceptable salt, solvate, hydrate, tautomer, optical isomer, N-oxide, and/or prodrug thereof; wherein
$R^1$ is selected from the group consisting of:

(i) $C_1$-$C_4$ alkyl optionally substituted with $C_6$-$C_{10}$ aryl; and
(ii) $C_6$-$C_{10}$ aryl and 5 to 10-membered heteroaryl, optionally substituted with one or more substituents independently selected from the group consisting of - OH, halo, $C_1$-$C_4$ alkyl, $C_1$-$C_4$ alkoxy, $C_1$-$C_4$ hydroxyalkyl, $C_1$-$C_4$ haloalkyl, $C_1$-$C_4$ haloalkoxy, $C_1$-$C_4$ hydroxyalkoxy, acetyl, cyano, $C_6$-$C_{10}$ aryl, 5- to 10-membered heteroaryl, 5- to 10-membered heterocycloalkyl, -O-($C_6$-$C_{10}$ aryl), -O-$CH_2$-($C_6$-$C_{10}$ aryl), and -$NR^4R^5$; and

$R^2$ is selected from the group consisting of H, $C_1$-$C_4$ alkyl, $C_1$-$C_4$ haloalkyl, $C_1$-$C_4$ hydroxyalkyl, and phenyl;
$R^3$ is H or -$CH_3$;
$R^4$ and $R^5$ are each independently H or $C_1$-$C_4$ alkyl; and
n = 0 or 1.

**2.** The compound according to claim 1, wherein $R^2$ is selected from the group consisting of H, -$CH_3$, -$CH_2F$, -$CHF_2$, -$CF_3$, -($C_2H_4$)-OH, and phenyl, preferably wherein $R^2$ is selected from the group consisting of H, -$CH_3$, -$CHF_2$, -$CF_3$, -($C_2H_4$)-OH, and phenyl, more preferably H; and/or

wherein $R^3$ is -$CH_3$; and/or
wherein $R^4$ and $R^5$ are each independently $C_1$-$C_2$ alkyl, preferably -$CH_3$; and/or
wherein n = 1.

**3.** The compound according to claim 1 or 2, wherein $R^1$ is selected from the group consisting of:

(i) $C_1$-$C_2$ alkyl optionally substituted with phenyl; and
(ii) phenyl, naphthyl, 5- or 6-membered monocyclic heteroaryl, and 9- or 10-membered fused bicyclic heteroaryl, optionally substituted with one or more substituents independently selected from the group consisting of -OH, halo, $C_1$-$C_4$ alkyl, $C_1$-$C_4$ alkoxy, $C_1$-$C_4$ hydroxyalkyl, $C_1$-$C_4$ haloalkyl, $C_1$-$C_4$ haloalkoxy, $C_1$-$C_4$ hydroxyalkoxy, acetyl, cyano, $C_6$-$C_{10}$ aryl, 5- to 10-membered heteroaryl, 5- to 10-membered heterocycloalkyl, -O-($C_6$-$C_{10}$ aryl), -O-$CH_2$-($C_6$-$C_{10}$ aryl), and -$NR^4R^5$.

**4.** The compound according to claim 3, wherein each ring atom in the 5- or 6-membered monocyclic heteroaryl group and the 9- or 10-membered fused bicyclic heteroaryl group of $R^1$ is independently selected from the group consisting of C, N, S, and O, preferably one to three ring atoms are independently selected from the group consisting of N, S, and O and the remaining ring atoms are C.

**5.** The compound according to claim 4, wherein each ring atom in the 5- or 6-membered monocyclic heteroaryl group of $R^1$ is independently C or N, preferably one or two ring atoms are N and the remaining ring atoms are C.

**6.** The compound according to any of claims 3 to 5, wherein the 5- or 6-membered monocyclic heteroaryl group of $R^1$ is selected from the group consisting of pyrrolyl, pyrazolyl, imidazolyl, triazolyl, pyridyl, pyrimidinyl, pyrazinyl, pyridazinyl, and triazinyl, preferably pyrazolyl, pyridyl, and pyrimidinyl, more preferably one of the following groups:

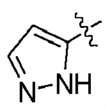, 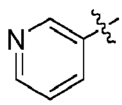, 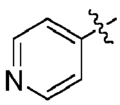, 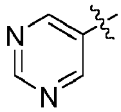, and 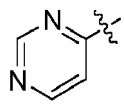,

wherein each group is optionally substituted.

**7.** The compound according to any of claims 3 to 6, wherein the 9- or 10-membered fused bicyclic heteroaryl group of $R^1$ is independently selected from the group consisting of quinolinyl, isoquinolinyl, quinoxalinyl, phthalazinyl, quinazolinly, cinnolinyl, naphthyridinyl, pyridopyrimidinyl, pyridopyrazinyl, indolyl, indazolyl, benzimidazolyl, azaindolyl, azaindazolyl, pyrazolopyrimidinyl, benzofuranyl, benzothiophenyl, benzoisoxazolyl, benzoisothiazolyl, benzoxazolyl, benzothiazolyl, benzothiadiazolyl, and benzotriazolyl, preferably, quinolinyl, isoquinolinyl, quinoxalinyl, naphthyridinyl, indolyl, azaindolyl, indazolyl, benzimidazolyl, benzotriazolyl, benzothiazolyl, and benzofuranyl, more preferably one of the following groups:

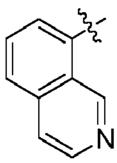, 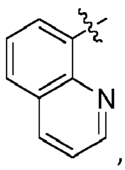, 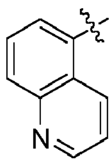, 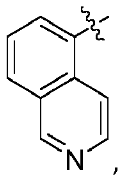, 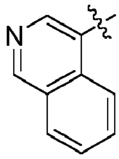, 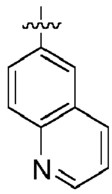, 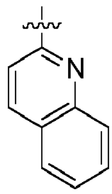, 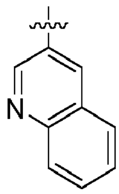, 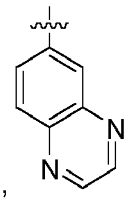

, 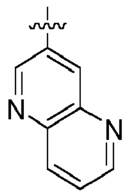, 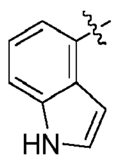, 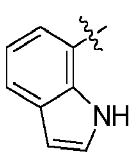, 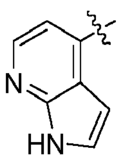, 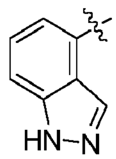, 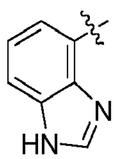, 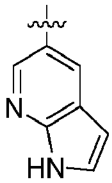, 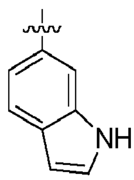,

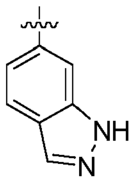, 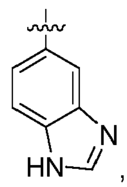, 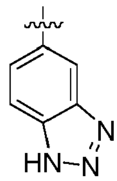, 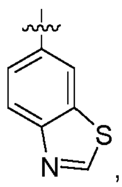, 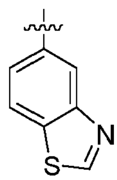, 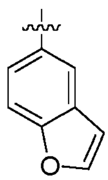, and 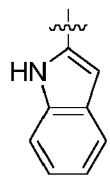,

wherein each group is optionally substituted.

**8.** The compound according to any preceding claim, wherein $R^1$ is selected from the group consisting of:

(i) $C_1$-$C_2$ alkyl optionally substituted with phenyl; and
(ii) phenyl, naphthyl, 5- or 6-membered monocyclic heteroaryl, and 9- or 10-membered fused bicyclic heteroaryl, optionally substituted one or more substituents independently selected from the group consisting of -OH, halo, $C_1$-$C_4$ alkyl, $C_1$-$C_4$ alkoxy, $C_1$-$C_4$ hydroxyalkyl, $C_1$-$C_4$ haloalkyl, $C_1$-$C_4$ haloalkoxy, acetyl, cyano, 5- or 6-membered heteroaryl, 5-membered heterocycloalkyl, -O-phenyl, and -$NR^4R^5$, preferably -OH, halo, $C_1$-$C_2$ alkyl, $C_1$-$C_2$ alkoxy, $C_1$-$C_2$ hydroxyalkyl, $C_1$-$C_2$ haloalkyl, $C_1$-$C_2$ haloalkoxy, acetyl, cyano, imidazolyl, triazolyl, pyridyl, pyrrolidinyl, -O-phenyl, and -$NR^4R^5$.

**9.** The compound according to any preceding claim, wherein $R^1$ is selected from the group consisting of:

(i) $C_1$-$C_2$ alkyl optionally substituted with phenyl; and
(ii) phenyl and 5- or 6-membered monocyclic heteroaryl, optionally substituted with one or more substituents independently selected from the group consisting of -OH, halo, $C_1$-$C_2$ alkyl, $C_1$-$C_2$ alkoxy, $C_1$-$C_2$ hydroxyalkyl, $C_1$-$C_2$ haloalkyl, $C_1$-$C_2$ haloalkoxy, acetyl, cyano, imidazolyl, triazolyl, pyridyl, pyrrolidinyl, -O-phenyl, and -$NR^4R^5$; and
(iii) naphthyl and 9- or 10-membered fused bicyclic heteroaryl, optionally substituted with one or more substituents independently selected from the group consisting of halo, $C_1$-$C_2$ alkyl, and $C_1$-$C_2$ alkoxy.

**10.** The compound according to claim 9, wherein $R^1$ is selected from the group consisting of:

(i) $C_1$-$C_2$ alkyl optionally substituted with phenyl;
(ii) phenyl optionally substituted with one or more substituents independently selected from the group consisting of -OH, halo, $C_1$-$C_2$ alkyl, $C_1$-$C_2$ alkoxy, $C_1$-$C_2$ hydroxyalkyl, $C_1$-$C_2$ haloalkyl, $C_1$-$C_2$ haloalkoxy, acetyl, cyano, imidazolyl, triazolyl, pyridyl, pyrrolidinyl, -O-phenyl, and $-NR^4R^5$;
(iii) 5- or 6-membered heteroaryl, optionally substituted with one or more substituents independently selected from the group consisting of halo, $C_1$-$C_2$ alkyl, $C_1$-$C_2$ alkoxy, and -O-phenyl;
(iv) naphthyl optionally substituted with one or more substituents independently selected from the group consisting of halo and $C_1$-$C_2$ alkoxy; and
(v) 9- or 10-membered fused bicyclic heteroaryl, optionally substituted with one or more substituents independently selected from the group consisting of halo and $C_1$-$C_2$ alkyl.

**11.** The compound according to any preceding claim, wherein $R^1$ is selected from the group consisting of:

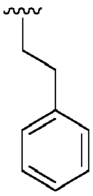, 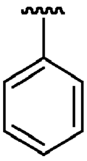, 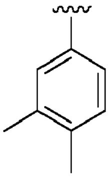, 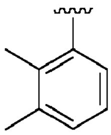, 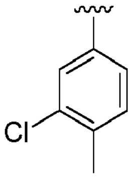, 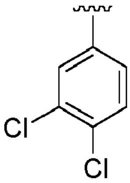, 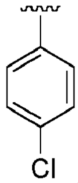, 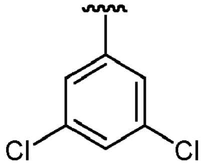,

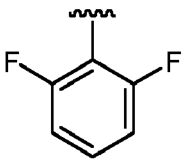, 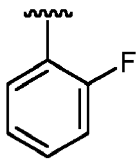, 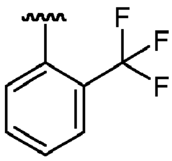, 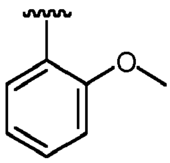, 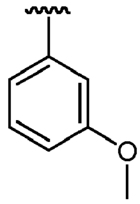, 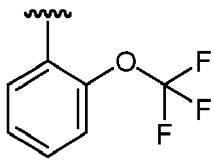,

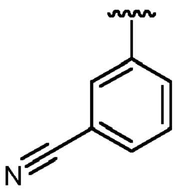, 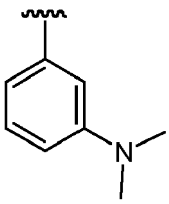, 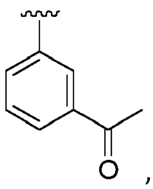, 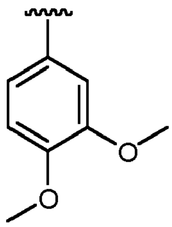, 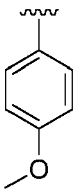, 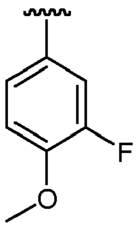, 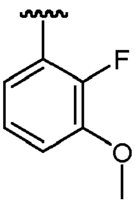,

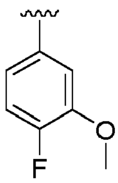, 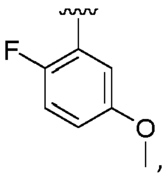, 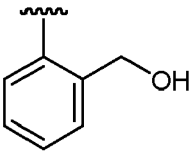, 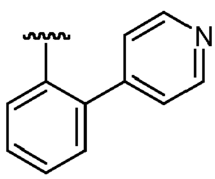, 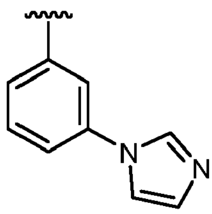,

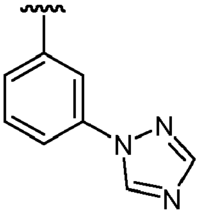, 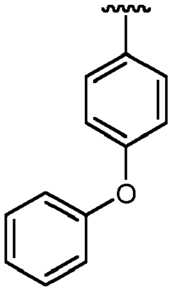, 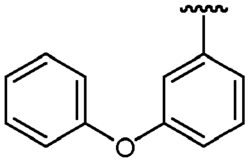, 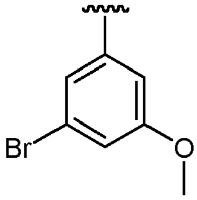, 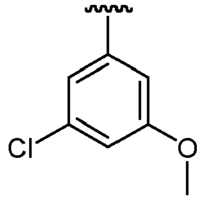,

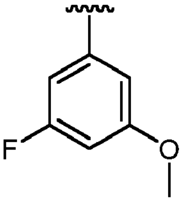, 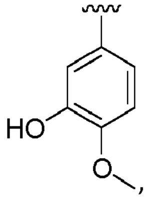, 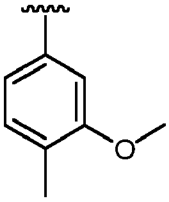, 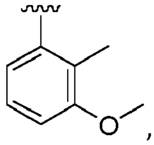, 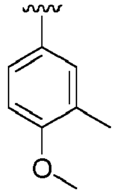, 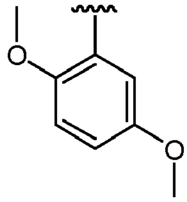,

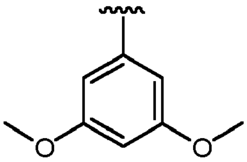, 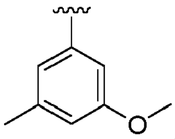, 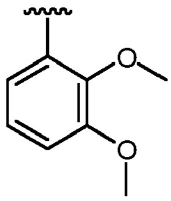, 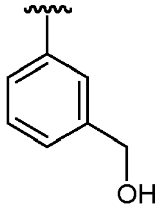, 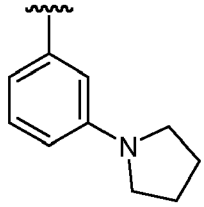,

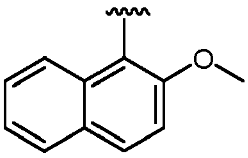, 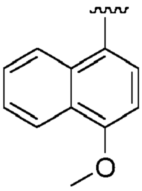, 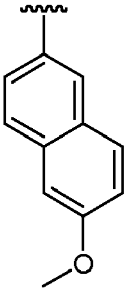, 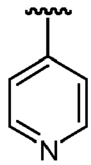, 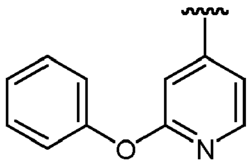, 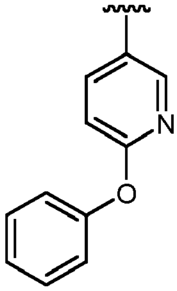,

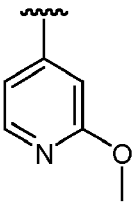, 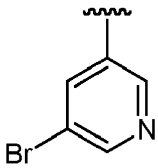, 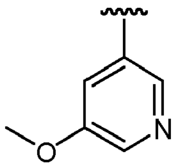, 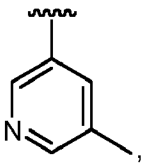, 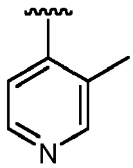, 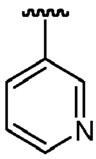, 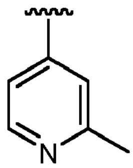,

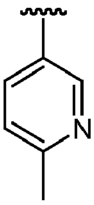, 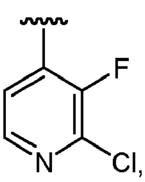, 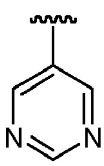, 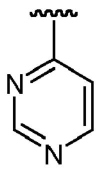, 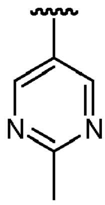, 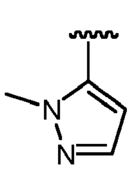, 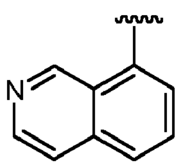,

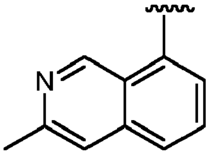, 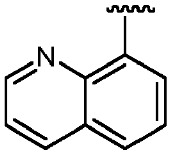, 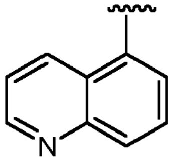, 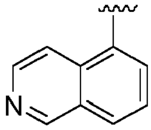, 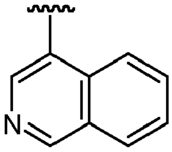, 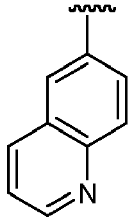,

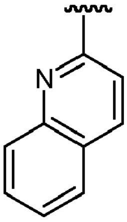, 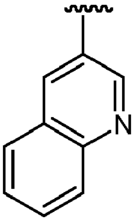, 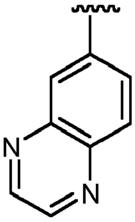, 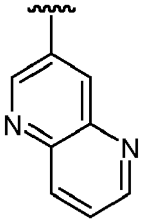, 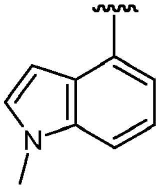, 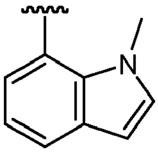, 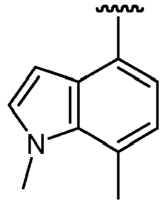,

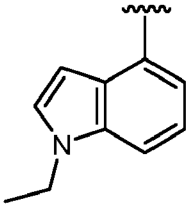, 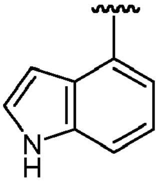, 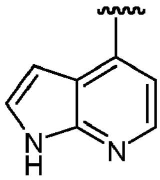, 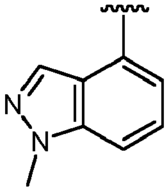, 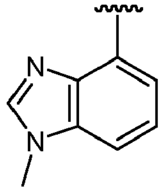, 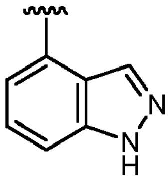,

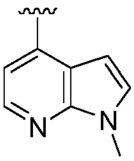, 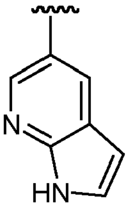, 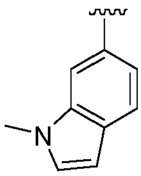, 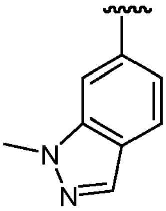, 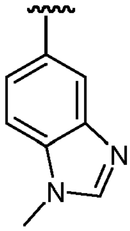, 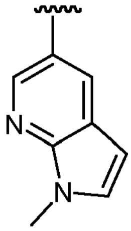, 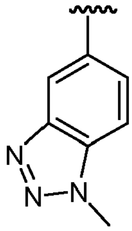,

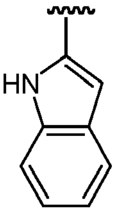, 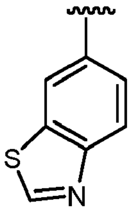, 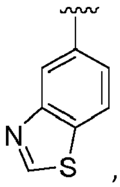, 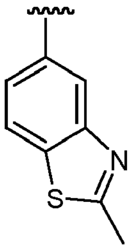, 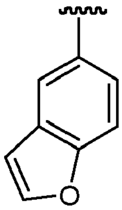,

preferably $R^1$ is selected from the group consisting of:

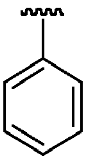, 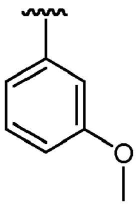, 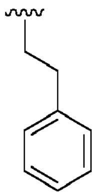, and 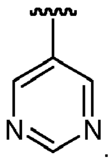.

**12.** The compound according to any preceding claim, wherein the compound is:

(R)-2-benzylamino-2,5,5-trimethylhexanoic acid;

(S)-2-benzylamino-2,5,5-trimethylhexanoic acid;
(R)-2,5,5-trimethyl-2-(3-phenylpropylamino)hexanoic acid;
(S)-2,5,5-trimethyl-2-(3-phenylpropylamino)hexanoic acid;
2,5,5-trimethyl-2-(3-phenylpropylamino)hexanoic acid;
(R)-2-{[(m-methoxyphenyl)methyl]amino}-2,5,5-trimethylhexanoic acid;
(S)-2-{[(m-methoxyphenyl)methyl]amino}-2,5,5-trimethylhexanoic acid;
(R)-2,5,5-trimethyl-2-{[(5-pyrimidinyl)methyl]amino}hexanoic acid;
(S)-2,5,5-trimethyl-2-{[(5-pyrimidinyl)methyl]amino}hexanoic acid;
2-{[(m-methoxyphenyl)methyl]amino}-2,5-dimethylhexanoic acid;
2,5,5-trimethyl-2-{[(5-pyrimidinyl)methyl]amino}hexanoic acid;
2-{[(m-methoxyphenyl)methyl]amino}-2,5,5-trimethylhexanoic acid;
2-benzylamino-2,5,5-trimethylhexanoic acid;
or a pharmaceutically acceptable salt, solvate, hydrate, tautomer, optical isomer, N-oxide, and/or prodrug thereof.

**13.** A pharmaceutical composition comprising a compound according to any preceding claim and a pharmaceutically acceptable carrier, excipient, and/or diluent.

**14.** The compound according to any one of claims 1 to 12, or the pharmaceutical composition of claim 13, for use in therapy.

**15.** The compound according to any one of claims 1 to 12, or the pharmaceutical composition of claim 13, for use in the treatment or prevention of a neurodegenerative disorder, a psychiatric disorder, an inflammatory disorder, a lysosomal storage disorder, a cancer, pain, diabetes mellitus, retinopathies such as diabetic retinopathy, brain tumours, glaucoma, uveitis, cardiovascular disease, kidney disease, psoriasis, hereditary eye conditions, chronic pain, hearing loss or diseases **characterized by** misfolded tau;

preferably wherein the neurodegenerative disorder is selected from motor neuron diseases, Frontotemporal Lobar Degeneration (FTLD), frontotemporal dementia, Alzheimer's disease, Parkinson's disease, Huntington's disease, prion diseases such as Creutzfeldt-Jakob disease (CJD), acute brain injury, spinal cord injury and stroke, preferably wherein the motor neuron disease is selected from amyotrophic lateral sclerosis (ALS), Primary Lateral Sclerosis, and Progressive Muscular Atrophy;
preferably wherein the neurodegenerative disorder is **characterised by** misfolded TAR DNA binding protein 43, such as amyotrophic lateral sclerosis, Alzheimer's disease, Frontotemporal Lobar Degeneration, or frontotemporal dementia;
preferably wherein the psychiatric disorder is selected from bipolar disorder, major depression, post-traumatic stress disorder, and anxiety disorders.
preferably wherein the inflammatory disorder is selected from inflammatory diseases and neuroinflammation;
preferably wherein the lysosomal storage disorder is selected from the group consisting of NCL/Batten Disease caused by mutations in CLN gene CLN1 (PPT1), CLN2 (TPP1), CLN3, CLN4 (DNAJC5), CLN5, CLN6, CLN7 (MFSD8), CLN8, CLN10 (CTSD), CLN11, CLN12 (ATP13A2), CLN13 (CTSF), CLN14 (KCTD7), CLCN6, and/or SGSH; Pompe disease, Fabry disease, Gaucher disease, Niemann-Pick disease Types A, B, and C; GM1 gangliosidosis, GM2 gangliosidosis (including Sandhoff and Tay-Sachs), mucopolysachariddoses (MPS) types I (Hurler disease)/II (Hunter disease)/IIIa (Sanfilippo A)/IIIB (Sanfilippo B)/IIIc (Sanfilippo C)/IIId (Sanfilippo D)/IVA (Morqui" A)/'VB/VI/VII (Sly)/IX, mucolipisosis III (I-cell) and IV, multiple sulfatase deficiency; sialidosis, galactosialidosis, α-mannosidosis, β-mannosidosis, apartylglucosaminuria, fucosidosis, Schindler disease, metachromatic leukodystrophy caused by deficiencies in either arylsulfatase A or Saposin B, globoid cell leukodystrophy (Krabbe disease), Farber lipogranulomatosis, Wolman and cholesteryl ester storage disease, pycnodystostosis, cystinosis, Salla disease, Danon disease, Griscelli disease Types ½/3, Hermansky Pudliak Disease, and Chediak-Higashi syndrome;
preferably wherein the cancer is selected from breast cancer, lung cancer, ovarian cancer, prostate cancer, thyroid cancer, pancreatic cancer, glioblastoma and colorectal cancer;
preferably wherein the cardiovascular disease is preferably selected from atherosclerosis, cardiomyopathy, heart attack, arrhythmias, heart failure, and ischemic heart disease; and
preferably wherein the hearing loss is selected from noise-induced hearing loss, ototoxicity induced hearing loss, age-induced hearing loss, idiopathic hearing loss, tinnitus and sudden hearing loss.

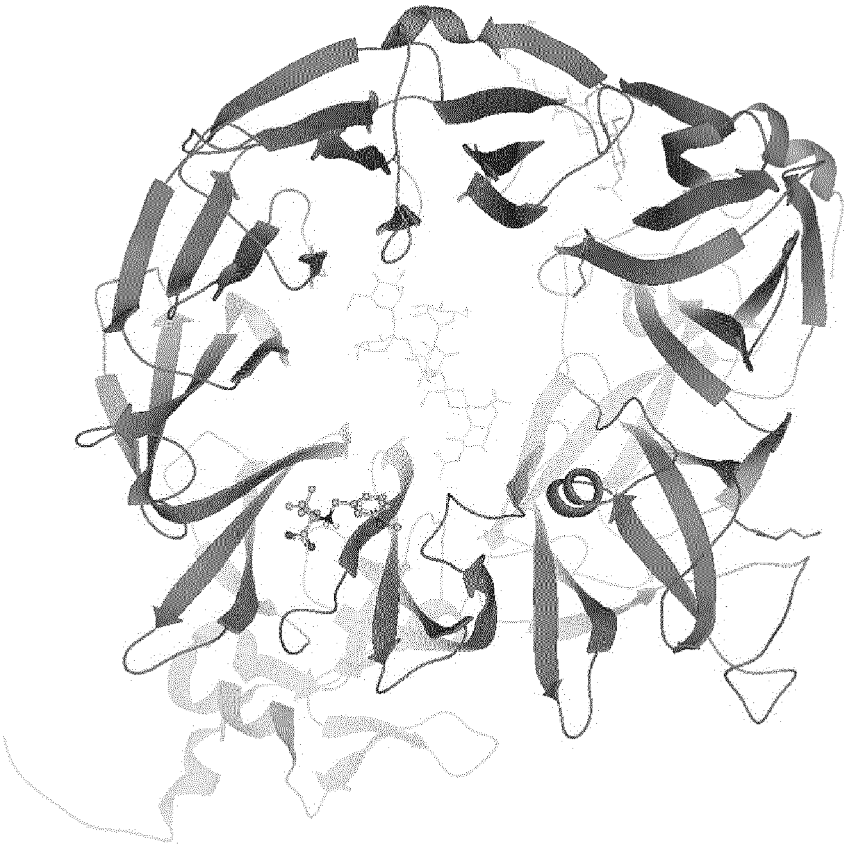

Figure 1

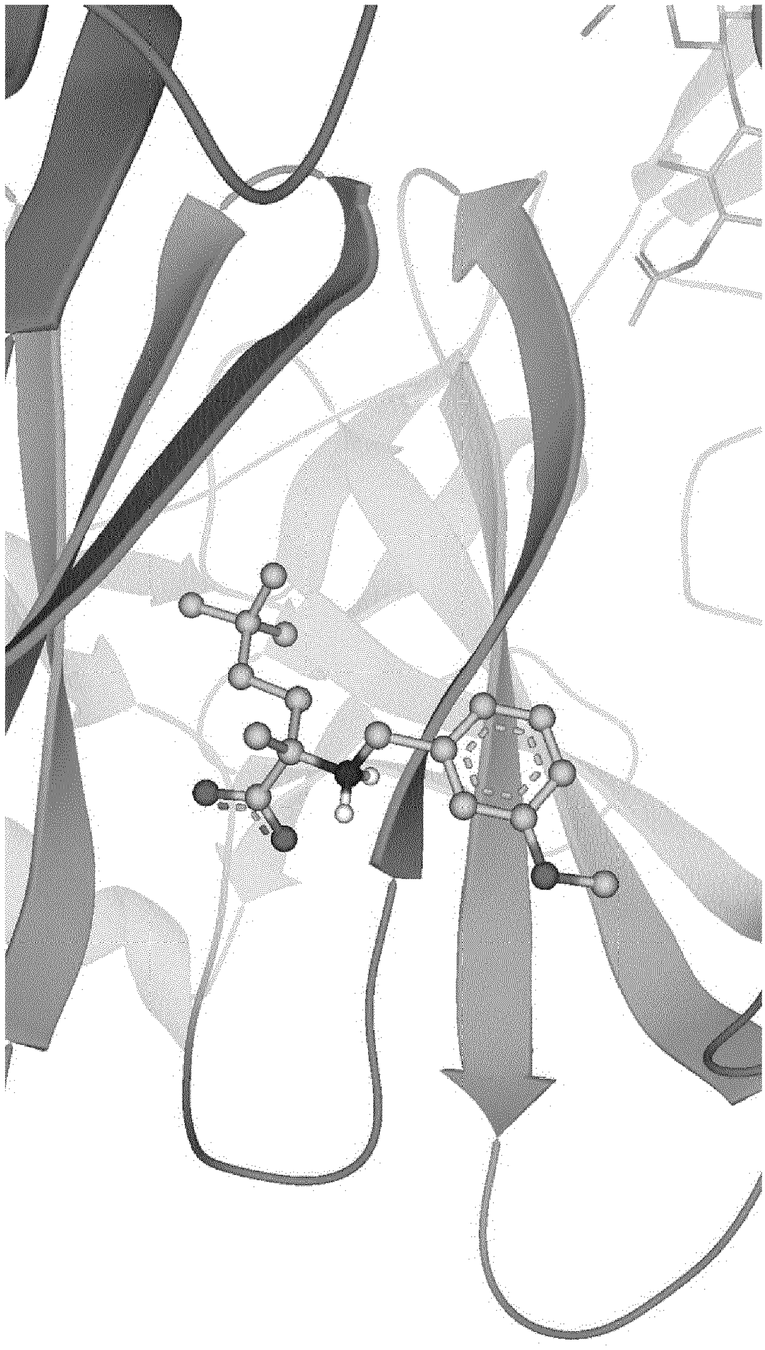

Figure 2

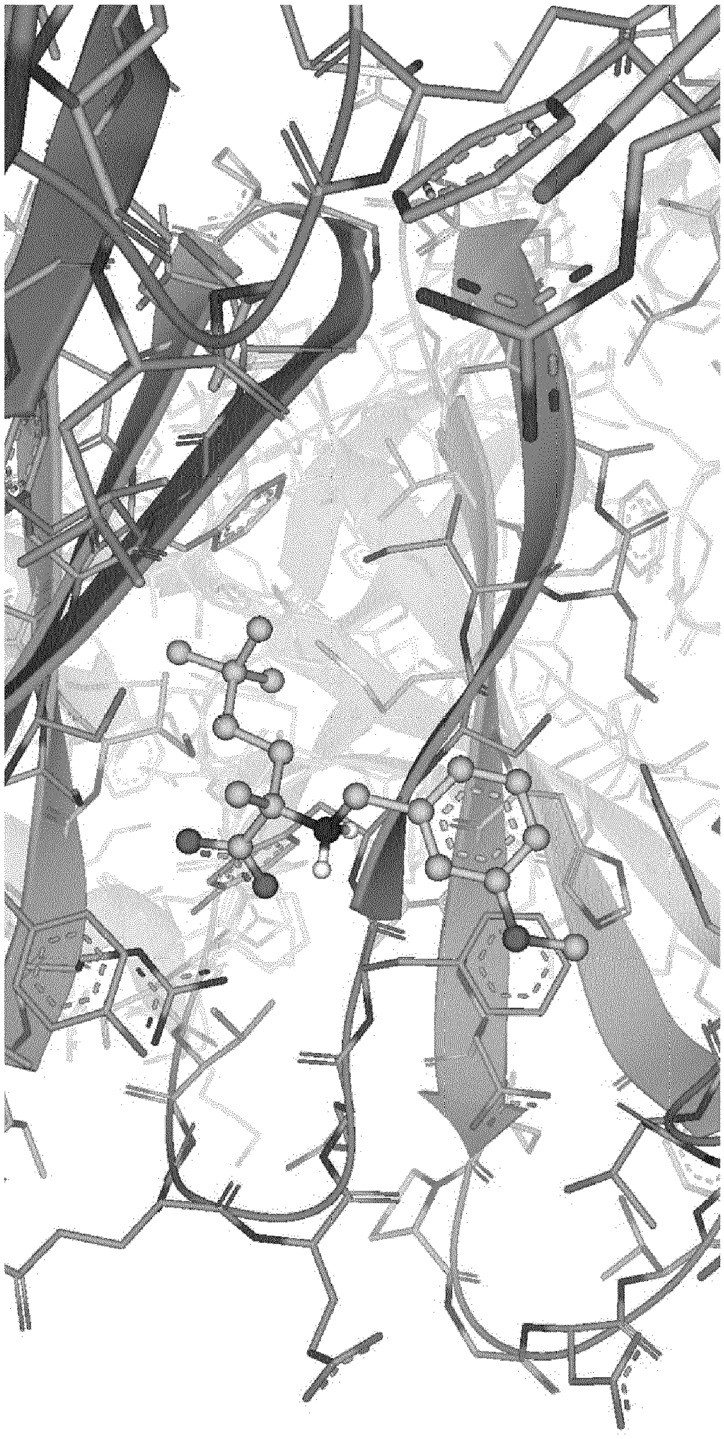

Figure 3

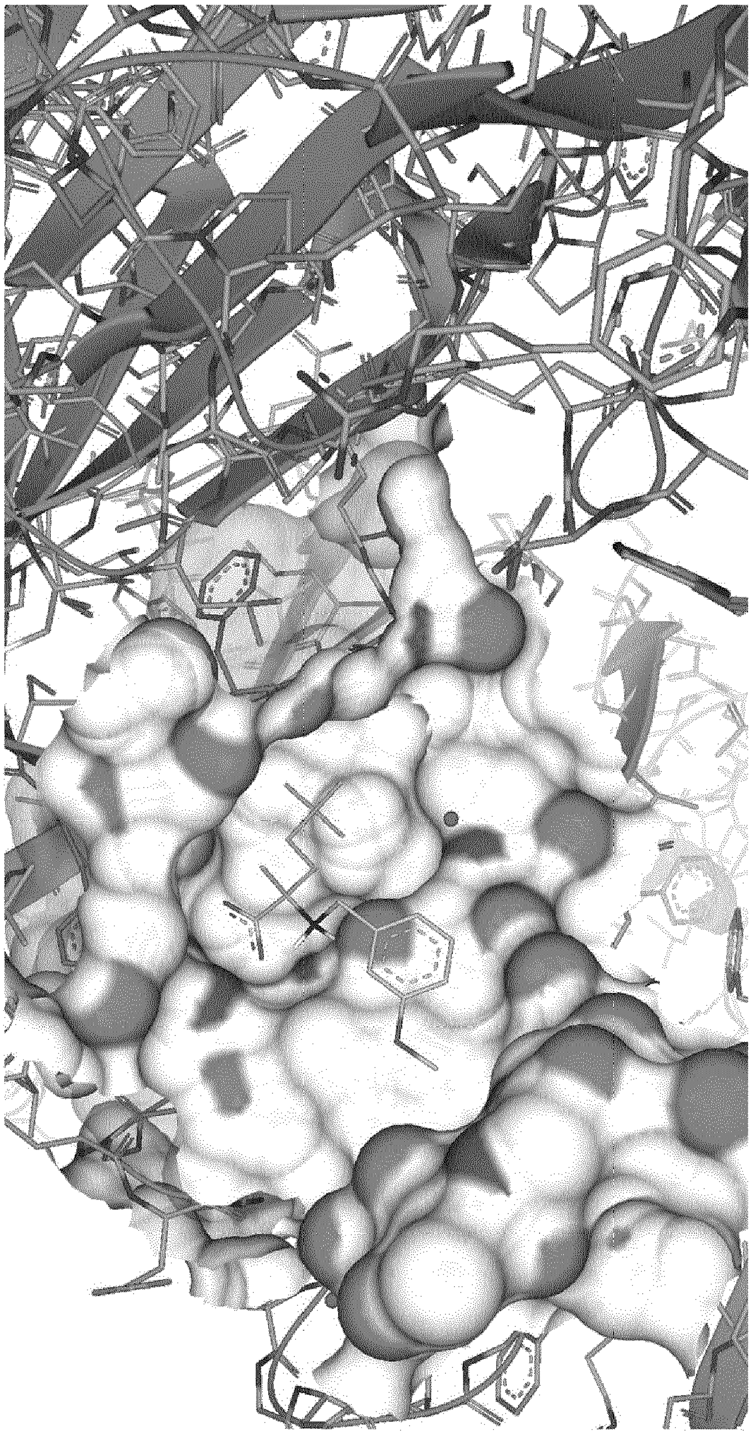

Figure 4

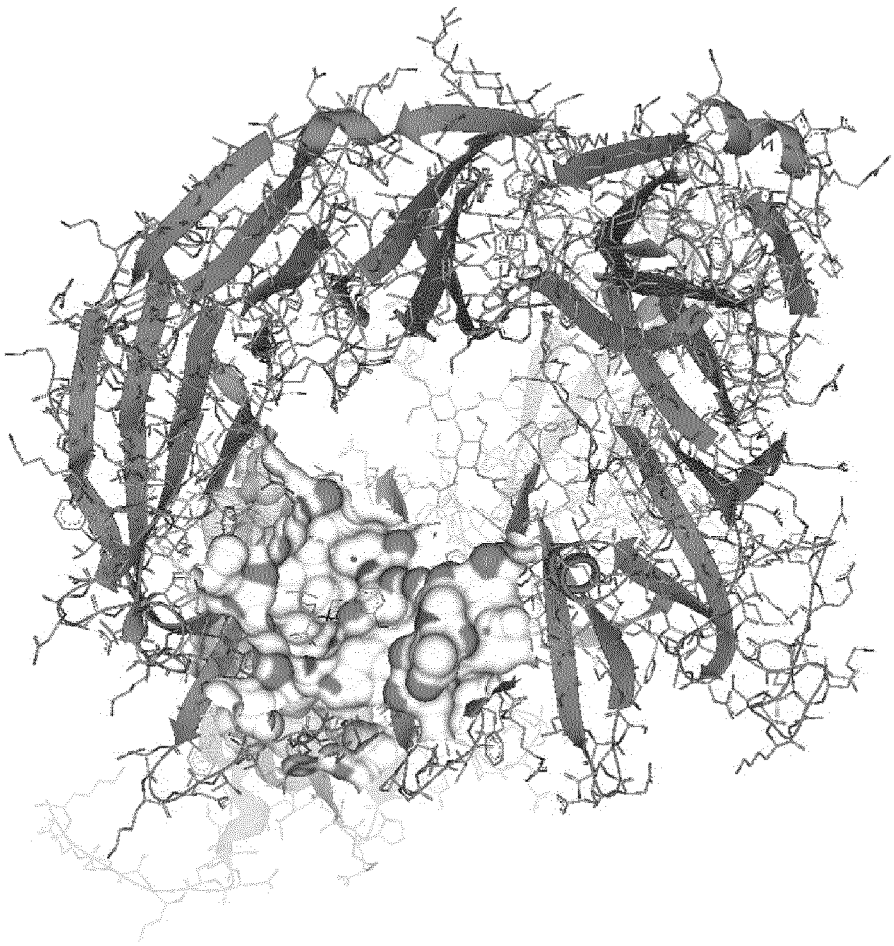

Figure 5

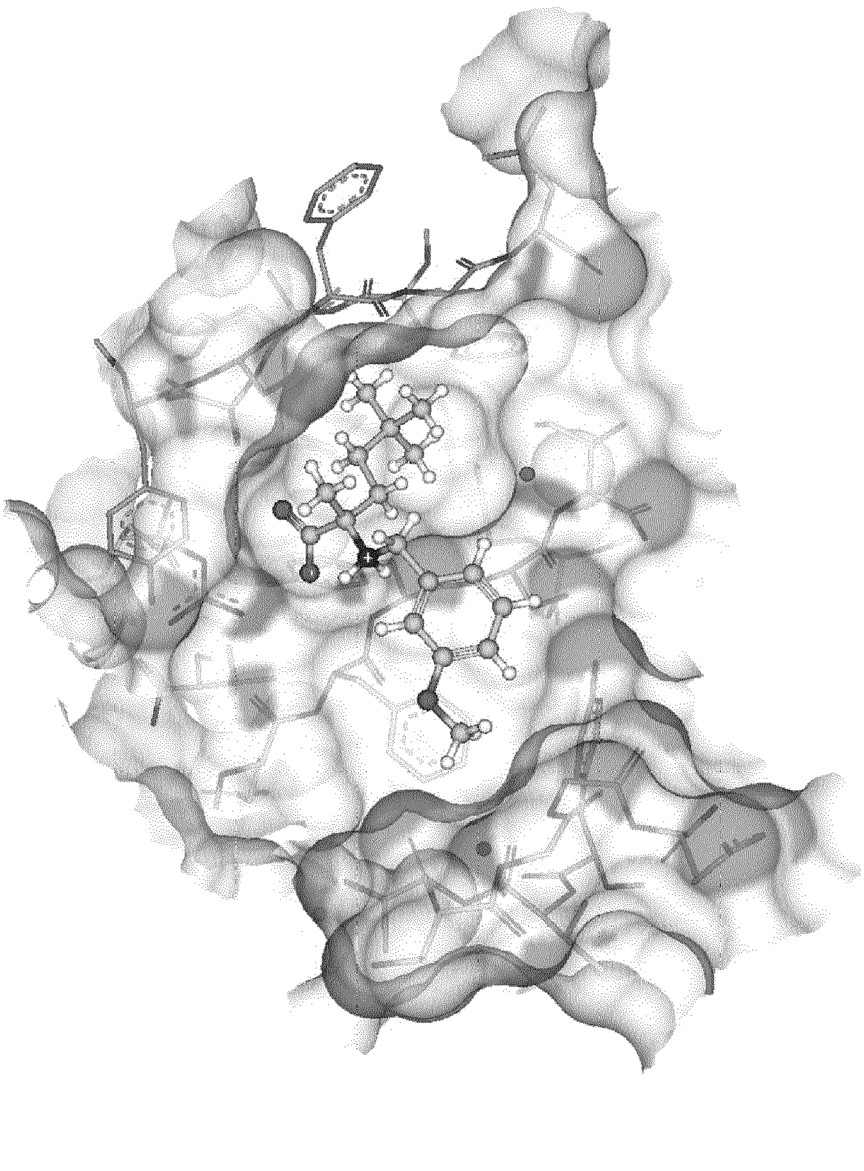

Figure 6

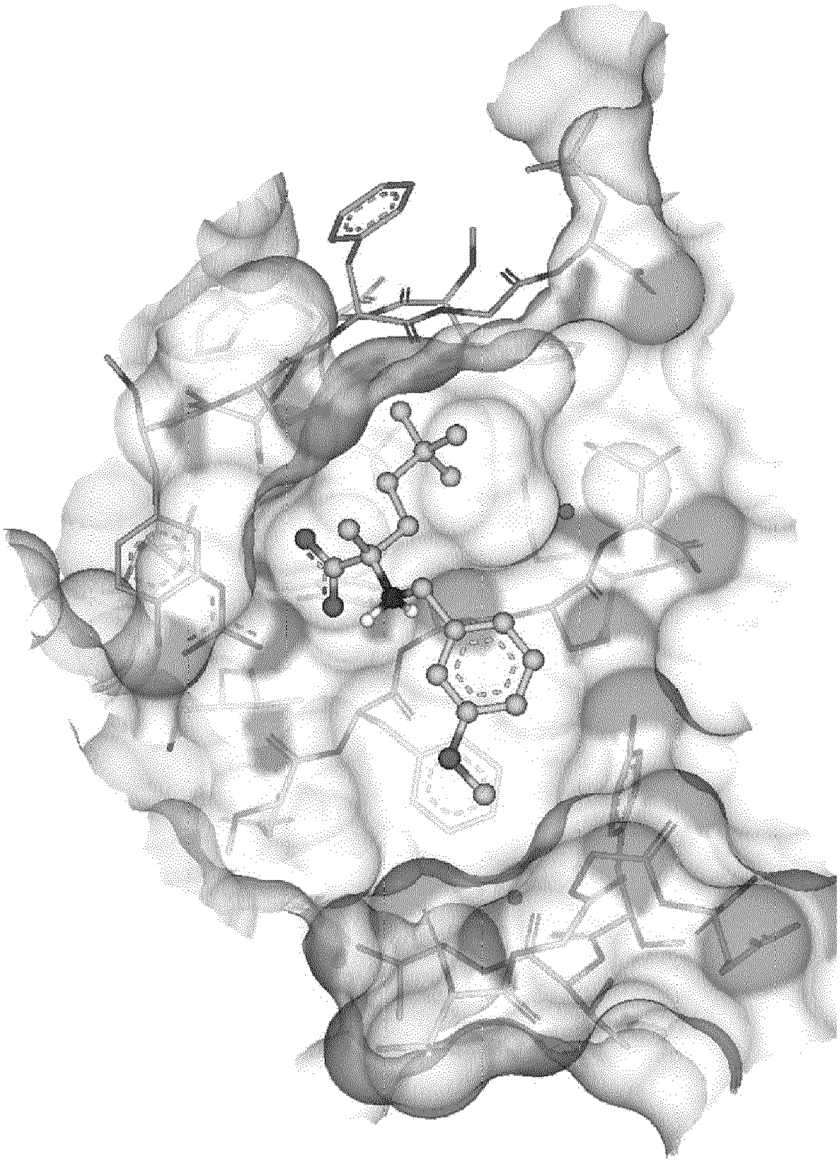

Figure 7

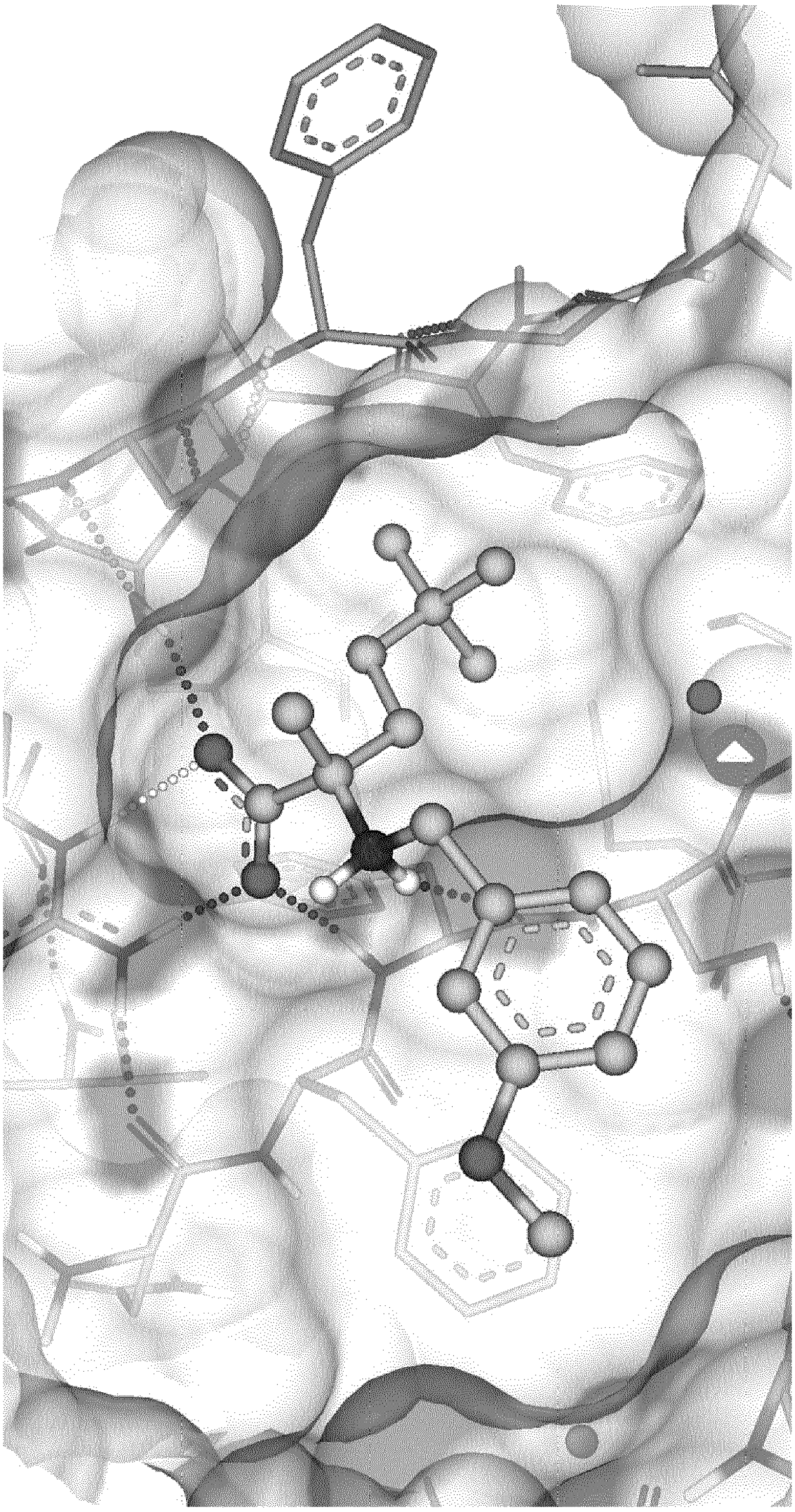

Figure 8

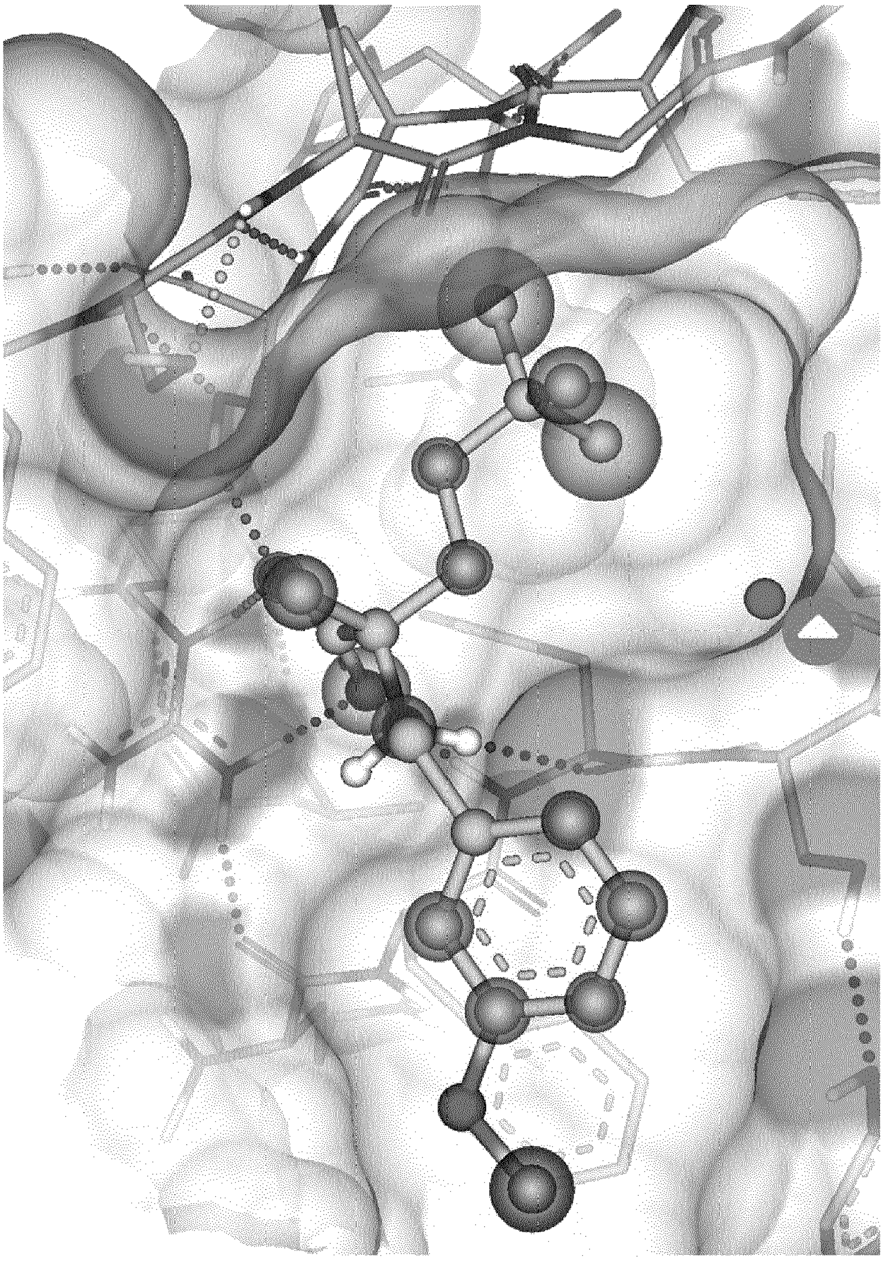

Figure 9

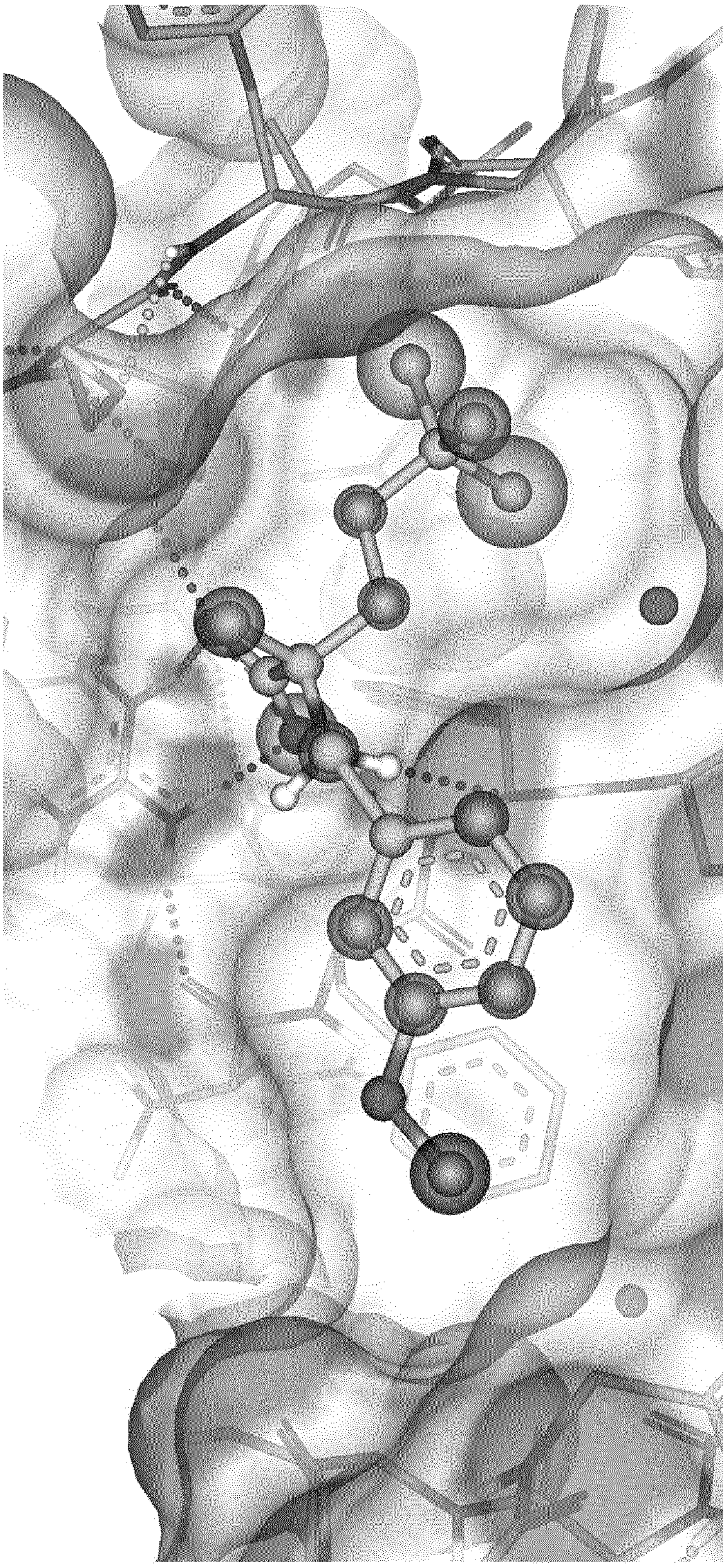

Figure 10

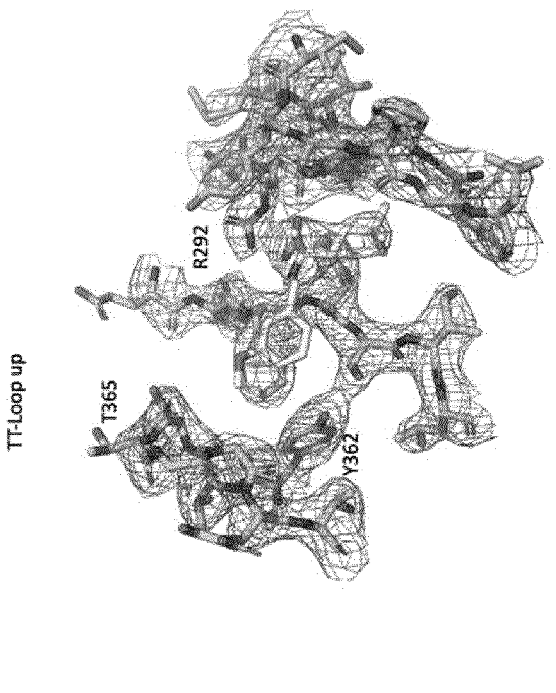


Figure 11A

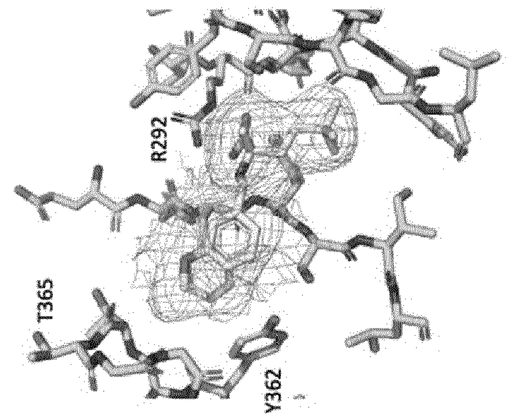


Figure 11B

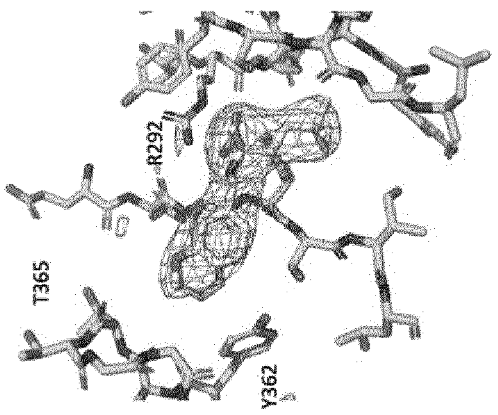


Figure 11C

Europäisches Patentamt
European Patent Office
Office européen des brevets

# EUROPEAN SEARCH REPORT

Application Number
EP 23 16 0616

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE REGISTRY [Online] CHEMICAL ABSTRACTS SERVICE, COLUMBUS, OHIO, US; 2 September 2009 (2009-09-02), XP002809925, retrieved from STN Database accession no. 1179516-24-5 * abstract * | 1,2 | INV. C07D239/26 C07C15/04 A61P25/18 A61P25/28 A61P35/00 |
| X | DATABASE REGISTRY [Online] CHEMICAL ABSTRACTS SERVICE, COLUMBUS, OHIO, US; 31 October 2010 (2010-10-31), XP002809926, retrieved from STN Database accession no. 1249720-92-0 * abstract * | 1,2 | |
| X | DATABASE REGISTRY [Online] CHEMICAL ABSTRACTS SERVICE, COLUMBUS, OHIO, US; 7 February 2016 (2016-02-07), XP002809927, Database accession no. 1861273-99-5 * abstract * | 1,2 | TECHNICAL FIELDS SEARCHED (IPC) C07D C07C A61P |
| X | DATABASE REGISTRY [Online] CHEMICAL ABSTRACTS SERVICE, COLUMBUS, OHIO, US; 14 June 2009 (2009-06-14), XP002809928, retrieved from STN Database accession no. 1157176-36-7 * abstract * | 1,2 | |

-/--

The present search report has been drawn up for all claims

1

| Place of search | Date of completion of the search | Examiner |
|---|---|---|
| Munich | 11 August 2023 | Bakboord, Joan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 3

Europäisches Patentamt
European Patent Office
Office européen des brevets

# EUROPEAN SEARCH REPORT

Application Number
EP 23 16 0616

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE REGISTRY [Online] CHEMICAL ABSTRACTS SERVICE, COLUMBUS, OHIO, US; 14 September 2009 (2009-09-14), XP002809929, retrieved from STN Database accession no. 1184235-35-5 * abstract * | 1,2 | |
| X | DATABASE REGISTRY [Online] CHEMICAL ABSTRACTS SERVICE, COLUMBUS, OHIO, US; XP002809930, retrieved from STN Database accession no. 1871518-02-3 * abstract * | 1,2 | |
| X | DATABASE REGISTRY [Online] CHEMICAL ABSTRACTS SERVICE, COLUMBUS, OHIO, US; 14 June 2009 (2009-06-14), XP002809931, retrieved from STN Database accession no. 11571174-94-1 * abstract * | 1,2 | TECHNICAL FIELDS SEARCHED (IPC) |
| X | DATABASE REGISTRY [Online] CHEMICAL ABSTRACTS SERVICE, COLUMBUS, OHIO, US; 15 June 2009 (2009-06-15), XP002809932, retrieved from STN Database accession no. 1157986-78-1 * abstract * | 1,2 | |
| | -/-- | | |

The present search report has been drawn up for all claims

1

| Place of search | Date of completion of the search | Examiner |
|---|---|---|
| Munich | 11 August 2023 | Bakboord, Joan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 23 16 0616

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE REGISTRY [Online] CHEMICAL ABSTRACTS SERVICE, COLUMBUS, OHIO, US; 2 September 2009 (2009-09-02), XP002809933, retrieved from STN Database accession no. 1179516-24-5 * abstract * ----- | 1,2 | |
| A | WO 2022/238565 A2 (INSUSENSE APS [DK]) 17 November 2022 (2022-11-17) * page 4, line 12 - line 24; claim 1 * ----- | 1-15 | |
| A | WO 2022/223805 A1 (INSUSENSE APS [DK]) 27 October 2022 (2022-10-27) * page 4, line 4 - line 16 * ----- | 1-15 | |

TECHNICAL FIELDS SEARCHED (IPC)

1

The present search report has been drawn up for all claims

| Place of search | Date of completion of the search | Examiner |
|---|---|---|
| Munich | 11 August 2023 | Bakboord, Joan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 3 of 3

## ANNEX TO THE EUROPEAN SEARCH REPORT ON EUROPEAN PATENT APPLICATION NO. EP 23 16 0616

This annex lists the patent family members relating to the patent documents cited in the above-mentioned European search report. The members are as contained in the European Patent Office EDP file on
The European Patent Office is in no way liable for these particulars which are merely given for the purpose of information.

11-08-2023

| Patent document cited in search report | Publication date | Patent family member(s) | Publication date |
|---|---|---|---|
| WO 2022238565 A2 | 17-11-2022 | EP 4089102 A1 | 16-11-2022 |
| | | WO 2022238565 A2 | 17-11-2022 |
| WO 2022223805 A1 | 27-10-2022 | EP 4079748 A1 | 26-10-2022 |
| | | WO 2022223805 A1 | 27-10-2022 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20160331746 A
- WO 2021116290 A1
- EP 21194937
- EP 2022074536 W


### Non-patent literature cited in the description


- **SILVERMAN, R. B.** The Organic Chemistry of Drug Design and Drug Action. Elsevier Academic Press, 2004, 498-549
- **GREENE ; WUTS.** Protective Groups in Organic Synthesis. John Wiley and Sons, 2006
- **ANDERSEN et al.** *Acta Cryst. D,* 2017
- **KABSCH, W.** *Acta Cryst. D,* 2010
- **AFONINE et al.** *Acta Cryst. D,* 2012
- **EMSLEY P. et al.** *Acta Cryst. D,* 2010
- **ANDERSEN, J et al.** Identification of the first small-molecule ligand of the neuronal receptor sortilin and structure determination of the receptor-ligand complex. *Acta Crystallogr D Biol Crystallogr,* 2014, vol. 70 (2), 451-460
- **BAKER, M. et al.** Mutations in progranulin cause tau-negative frontotemporal dementia linked to chromosome 17. *Nature,* 2006, vol. 442 (7105), 916-919
- **BROUWERS, N. et al.** Genetic variability in progranulin contributes to risk for clinically diagnosed Alzheimer disease. *Neurology,* 2008, vol. 71 (9), 656-664
- **BUTTENSHON, H.N. et al.** Increased serum levels of sortilin are associated with depression and correlated with BDNF and VEGF. *Nature Translational Psychiatry,* 2015, vol. 5, 1-7
- **CARECCHIO, M.** Cerebrospinal fluid biomarkers in Progranulin mutations carriers. *J Alzheimers Dis,* 2011, vol. 27 (4), 781-790
- **CARRASQUILLO, M. et al.** Genome-wide screen identifies rs646776 near sortilin as a regulator of progranulin levels in human plasma. *Am J Hum Genet,* 2010, vol. 87 (6), 890-897
- **CHEN, Z. Y. et al.** Sortilin controls intracellular sorting of brain-derived neurotrophic factor to the regulated secretory pathway. *J Neurosci,* 2005, vol. 25 (26), 6156-6166
- **CRUTS, M. et al.** Loss of progranulin function in frontotemporal lobar degeneration. *Trends Genet,* 2008, vol. 24 (4), 186-194
- **DE MUYNCK, L. et al.** The neurotrophic properties of progranulin depend on the granulin E domain but do not require sortilin binding. *Neurobiol Aging,* 2013, vol. 34 (11), 2541-2547
- **EGASHIRA, Y. et al.** The growth factor progranulin attenuates neuronal injury induced by cerebral ischemia-reperfusion through the suppression of neutrophil recruitment. *J Neuroinflammation,* 2013, vol. 10, 105
- **GALIMBERTI, D. et al.** GRN variability contributes to sporadic frontotemporal lobar degeneration. *J Alzheimers Dis,* 2010, vol. 19 (1), 171-177
- **GALIMBERTI, D. et al.** Progranulin as a therapeutic target for dementia. *Expert Opin Ther Targets,* 2018, vol. 22 (7), 579-585
- **GAO, A. et al.** Implications of Sortilin in Lipid Metabolism and Lipid Disorder Diseases. *DNA and Cell Biology,* 2017, vol. 36 (12), 1050-1061
- **GASS, J. et al.** Progranulin regulates neuronal outgrowth independent of sortilin. *Mol Neurodegener,* 2012, vol. 7, 33
- **GASS, J. et al.** Progranulin: an emerging target for FTLD therapies. *Brain Res,* 2012, vol. 1462, 118-128
- **GIJSELINCK, I.** Granulin mutations associated with frontotemporal lobar degeneration and related disorders: an update. *Hum Mutat,* 2008, vol. 29 (12), 1373-1386
- **GOETTSCH, C. et al.** Sortilin and Its Multiple Roles in Cardiovascular and Metabolic Diseases. *Atherosclerosis, Thrombosis and Vascular Biology,* 2017, vol. 38 (1), 19-25
- **JANSEN, P. et al.** Roles for the pro-neurotrophin receptor sortilin in neuronal development, aging and brain injury. *Nature Neuroscience,* 2007, vol. 10 (11), 1449-1457
- **HU, F. et al.** Sortilin-mediated endocytosis determines levels of the frontotemporal dementia protein, progranulin. *Neuron,* 2010, vol. 68 (4), 654-667

- **HUANG, G. et al.** Insulin responsiveness of glucose transporter 4 in 3T3-L1 cells depends on the presence of sortilin. *Mol Biol Cell,* 2013, vol. 24 (19), 3115-3122
- **KADDAI, V. et al.** Involvement of TNF-α in abnormal adipocyte and muscle sortilin expression in obese mice and humans. *Diabetologia,* 2009, vol. 52, 932-940
- **KJOLBY, M. et al.** Sort1, encoded by the cardiovascular risk locus 1p13.3, is a regulator of hepatic lipoprotein export. *Cell Metab,* 2010, vol. 12 (3), 213-223
- **LAIRD, A. S. et al.** Progranulin is neurotrophic in vivo and protects against a mutant TDP-43 induced axonopathy. *PLoS One,* 2010, vol. 5 (10), e13368
- **LEE, W. et al.** Targeted manipulation of the sortilin-progranulin axis rescues progranulin haploinsufficiency. *Hum Mol Genet,* 2014, vol. 23 (6), 1467-1478
- **MARTENS, L. et al.** Progranulin deficiency promotes neuroinflammation and neuron loss following toxin-induced injury. *J Clin Invest,* 2012, vol. 122 (11), 3955-3959
- **MAZELLA, J. et al.** The 100-kDa neurotensin receptor is gp95/sortilin, a non-G-protein-coupled receptor. *J Biol Chem,* 1998, vol. 273 (41), 26273-26276
- **MIYAKAWA, S. et al.** Anti-sortilin1 Antibody Up-Regulates Progranulin via Sortilin1 Down-Regulation. *Front Neurosci,* 2020, vol. 14, 586107
- **MØLLER et al.** Sortilin as a Biomarker for Cardiovascular Disease Revisited. *Frontiers in Cardiovascular Medicine,* 2021, vol. 8, 652584
- **MORTENSEN, M.B. et al.** Targeting sortilin in immune cells reduces proinflammatory cytokines and atherosclerosis. *J Clin Invest,* 2014, vol. 124 (12), 5317-5322
- **NYKJAER, A et al.** Sortilin is essential for proNGF-induced neuronal cell death. *Nature,* 2014, vol. 427, 843-848
- **NYKJAER, A. ; WILLNOW, T. E.** Sortilin: a receptor to regulate neuronal viability and function. *Trends Neurosci,* 2012, vol. 35 (4), 261-270
- **OH, T.J. et al.** Circulating sortilin level as a potential biomarker for coronary atherosclerosis and diabetes mellitus. *Cardiovascular Diabetology,* 2017, vol. 16 (92
- **PAN, X. et al.** Sortilin and retromer mediate retrograde transport of Glut4 in 3T3-L1 adipocytes. *Mol Biol Cell,* 2017, vol. 28 (12), 1667-1675
- **PETERSEN, C. et al.** Molecular identification of a novel candidate sorting receptor purified from human brain by receptor-associated protein affinity chromatography. *J Biol Chem,* 1997, vol. 272 (6), 3599-3605
- **PICKFORD, F. et al.** Progranulin is a chemoattractant for microglia and stimulates their endocytic activity. *Am J Pathol,* 2011, vol. 178 (1), 284-295
- **POTTIER, C. et al.** Potential genetic modifiers of disease risk and age at onset in patients with frontotemporal lobar degeneration and GRN mutations: a genome-wide association study. *Lancet Neurol,* 2018, vol. 17 (6), 548-558
- **QUISTGAARD, E. et al.** Ligands bind to Sortilin in the tunnel of a ten-bladed beta-propeller domain. *Nat Struct Mol Biol,* 2009, vol. 16 (1), 96-98
- **SANTOS, A. M. et al.** Sortilin Participates in Light-dependent Photoreceptor Degeneration in Vivo. *PLoS ONE,* 2012, vol. 7 (4), e36243-e36243.16
- **KURUVILLA, R. et al.** A neurotrophin signaling cascade coordinates sympathetic neuron development through differential control of TrkA trafficking and retrograde signalling. *Cell,* 2004, vol. 118 (2), 243-255
- **SHI, J. ; KANDROR, K. V.** Sortilin Is Essential and Sufficient for the Formation of Glut4 Storage Vesicles in 3T3-L1 Adipocytes. *Developmental Cell,* 2005, vol. 9, 99-108
- **SCHRODER, T. et al.** The identification of AF38469: an orally bioavailable inhibitor of the VPS10P family sorting receptor Sortilin. *Bioorg Med Chem Lett,* 2014, vol. 24 (1), 177-180
- **SHENG, J. et al.** Progranulin polymorphism rs5848 is associated with increased risk of Alzheimer's disease. *Gene,* 2014, vol. 542 (2), 141-145
- **SKELDAL, S. et al.** Mapping of the Interaction Site between Sortilin and the p75 Neurotrophin Receptor Reveals a Regulatory Role for the Sortilin Intracellular Domain in p75 Neurotrophin Receptor Shedding and Apoptosis. *J Biol Chem,* 2012, vol. 21 (287), 43798-43809
- **TAURIS, J. et al.** Proneurotrophin-3 May Induce Sortilin-Dependent Death In Inner Ear Neurons. *Eur J Neuroscience,* 2020, vol. 33 (4), 622-31
- **TANG, W. et al.** The growth factor progranulin binds to TNF receptors and is therapeutic against inflammatory arthritis in mice. *Science,* 2011, vol. 332 (6028), 478-484
- **TAO, J. et al.** Neuroprotective effects of progranulin in ischemic mice. *Brain Res,* 2012, vol. 1436, 130-136
- **TENK, H.K. et al.** ProBDNF induces neuronal apoptosis via activation of a receptor complex of p75NTR and sortilin. *J Neuroscience,* 2005, vol. 10 (11), 1449-1457
- **VAN KAMPEN, J. M.** Progranulin gene delivery protects dopaminergic neurons in a mouse model of Parkinson's disease. *PLoS One,* 2014, vol. 9 (5), e97032
- **WILLNOW, T. E. et al.** VPS10P-domain receptors - regulators of neuronal viability and function. *Nat Rev Neurosci,* 2008, vol. 9 (12), 899-909
- **WILLNOW, T.E. et al.** Sortilins: new players in lipoprotein metabolism. *Current Opinion in Lipidology,* 2011, vol. 22 (2), 79-85

- **WUTS, P.G.M. ; GREENE, T.W.** Greene's Protective Groups in Organic Synthesis. John Wiley and Sons, 2006
- **XU, S.H. et al.** Regional and Cellular Mapping of Sortilin Immunoreactivity in Adult Human Brain. *Frotiers in Neuroanatomy,* 2019, vol. 13 (31), 1-27
- **YANO, H. et al.** Proneurotrophin-3 is a neuronal apoptotic ligand: evidence for retrograde-directed cell killing. *J Neurosci,* 2009, vol. 29 (47), 14790-14802
- **YIN, F. et al.** Exaggerated inflammation, impaired host defense, and neuropathology in progranulin-deficient mice. *J Exp Med,* 2010, vol. 207 (1), 117-128
- **ZHENG, Y. et al.** C-terminus of progranulin interacts with the beta-propeller region of sortilin to regulate progranulin trafficking. *PLoS One,* 2011, vol. 6 (6), e21023
- **ZHOU, X. et al.** Prosaposin facilitates sortilin-independent lysosomal trafficking of progranulin. *J Cell Biol,* 2015, vol. 210 (6), 991-1002
- **MENESES et al.** TDP-43 Pathology in Alzheimer's Disease. *Mol Neurodegeneration,* 2021, vol. 16, 84
- **PRUDENCIO et al.** Misregulation of human sortilin splicing leads to the generation of a nonfunctional progranulin receptor. *Proc Natl Acad Sci USA,* 2012, vol. 109 (52), 21510-21515
- **BEEL et al.** Progranulin reduces insoluble TDP-43 levels, slows down axonal degeneration and prolongs survival in mutant TDP-43 mice. *Mol Neurodegener,* 2018, vol. 13, 55